(19) Europäisches Patentamt
European Patent Office
Office européen des brevets

(11) Veröffentlichungsnummer: **0 466 640 B1**

# (12) EUROPÄISCHE PATENTSCHRIFT

(45) Veröffentlichungstag der Patentschrift: **24.05.95**

(51) Int. Cl.[6]: **C09K 15/20**, C08K 5/16, C07C 255/41, C07C 255/40, C07C 323/12, C07C 323/22, C07C 323/48, C07F 9/40

(21) Anmeldenummer: **91810475.3**

(22) Anmeldetag: **19.06.91**

## (54) Alpha-Carbonylphenylacetonitrilderivate als Stabilisatoren für organische Materialien.

(30) Priorität: **28.06.90 CH 2160/90**

(43) Veröffentlichungstag der Anmeldung:
**15.01.92 Patentblatt 92/03**

(45) Bekanntmachung des Hinweises auf die Patenterteilung:
**24.05.95 Patentblatt 95/21**

(84) Benannte Vertragsstaaten:
**BE DE FR GB IT NL**

(56) Entgegenhaltungen:
**EP-A- 0 333 643** **CH-A- 573 386**
**US-A- 3 089 860** **US-A- 3 310 555**
**US-A- 3 344 023** **US-A- 4 093 587**

**CHEMICAL ABSTRACTS, FIFTH DECENNIAL INDEX, SUBJECTS A-Az, Bänder 41-50, 1947-1956, Seite 59 s, Columbus, Ohio, US; "Acetamide - 2-cyano-N-2-hydroxyethyl-2-phenyl-, in penicillin production, 43: 2273e"**

**Kirk-Othmer: Encyclopedia of Chemical Technology, vol 3, 1978. John Wiley & Sons. Pages 67,68.**

**Journal of Organic Chemistry, vol 37, No 12, 1972. Pages 1960-1966**

(73) Patentinhaber: **CIBA-GEIGY AG**
**Klybeckstrasse 141**
**CH-4002 Basel (CH)**

(72) Erfinder: **Nesvadba, Peter, Dr.**
**Route du Nord 5**
**CH-1723 Marly (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft Zusammensetzungen enthaltend ein oxidativem, thermischem und/oder aktinischem Abbau unterliegendes organisches Material und mindestens eine α-Carbonylphenylacetonitrilverbindung. Weitere Gegenstände der Erfindung sind die Verwendung dieser Verbindungen als Additive zum Stabilisieren von organischem Material sowie neue α-Carbonylphenylacetonitrilderivate.

Aus der JP-A 58-201851 ist bekannt, dass chlorhaltige Polymere durch Gemische aus organometallischen Ba-, Ca-, Mg-, Sr-, Li-, K-, Na-, Zn- und/oder Sn-Verbindungen, z.B. derartige Metallseifen von gesättigten oder ungesättigten Mono- oder Dicarboxylaten mit 6-22 C-Atomen, und Benzoylacetonitril oder Derivaten davon stabilisiert werden können.

Weitere α-Carbonylphenylacetonitrilderivate sind in zahlreichen Publikationen beschrieben, so z.B. in J.Org.Chem., 36, 3160-3168(1971), DE-A 2 033 910, US-A 3 793 364, Chem.Ber., 91, 1798(1958), CA 87-(23): 184219d, 91(5): 39426x, 94(19): 151882f, 97(11): 87060u, 98(17): 138991d, 100(7): 51289s, 103(3): 18295n, 103(25): 215070d, 104(25): 224795s, 106(9): 63041s, 109(11): 92273m, 110(11): 90027f und 110-(17): 154213y.

DE-OS-2,033,910 beschreibt Verbindungen der Formel

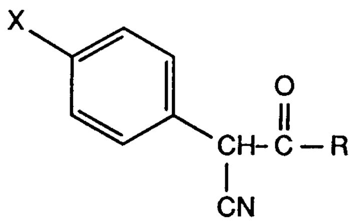 ,

worin R (O)Me, (O)Et, (O)Phenyl, $OC_{12}H_{15}$, $NH_2$, $NHCH_3$ und N<Pentamethylen (Piperidyl) ist und X für H, Me, MeO, Cl oder $NO_2$ steht. Die Verbindungen dienen als Ausgangssubstanzen zur Herstellung von Initiatoren für radikalische Reaktionen.

In der US-A-3,310,555 werden ähnliche Phenylacetonitrilderivate zur medizinischen Verwendung beschrieben.

Die CH-A-573 386 beschreibt eine Verbindung der Formel

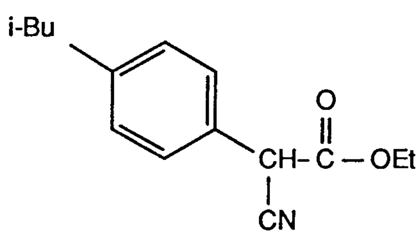

als Zwischenprodukt für die Herstellung von 2-(para-Isobutylphenyl)-buttersäure.

US-A-3,344,023 beschreibt im Zusammenhang mit blutdrucksenkenden Mitteln die Verbindungen 1-Cyano-1-(3,4-dimethoxyphenyl)2-butanon, -pentanon und -hexanon.

Gegenstand vorliegender Erfindung sind Zusammensetzungen enthaltend ein thermischem, oxidativem und/oder aktinischem Abbau unterliegendes organisches Material und mindestens eine Verbindung der Formel I

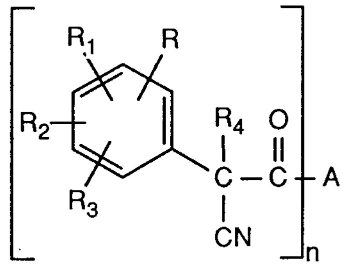

(I),

worin R Wasserstoff, -OH, Halogen, $C_1$-$C_{20}$-Alkyl, $C_2$-$C_{20}$-Alkenyl, $C_5$-$C_{12}$-Cycloalkyl, $C_1$-$C_4$-Alkyl-$C_5$-$C_{12}$-Cycloalkyl, Phenyl, Naphthyl, $C_1$-$C_{20}$-Alkoxy, Phenyloxy, Naphthyloxy, -S-$C_1$-$C_{20}$-Alkyl, -S-Phenyl, -S-Naphthyl, -O-CO-$C_1$-$C_{20}$-Alkyl, -O-CO-Phenyl, -O-CO-Naphthyl, -COOH, -COO-$C_1$-$C_{20}$-Alkyl, -COO-$C_2$-$C_{20}$-Alkenyl, -COO-Phenyl, -COO-Naphthyl, -CONR′R″, -CO-$C_1$-$C_{20}$-Alkyl, -CO-Phenyl, -CO-Naphthyl, -NHCO-$C_1$-$C_{20}$-Alkyl, -NHCO-$C_2$-$C_{20}$-Alkenyl, -NHCO-Phenyl oder -NHCO-Naphthyl bedeutet,
$R_1$ dieselbe Bedeutung wie R haben kann,
$R_2$ Wasserstoff, Halogen oder $C_1$-$C_4$-Alkyl ist und
$R_3$ Wasserstoff oder Halogen bedeutet,
oder zwei in ortho-Stellung zueinander gebundene Reste R, $R_1$, $R_2$ oder $R_3$ zusammen Tetramethylen oder -CH=CH-CH=CH- bilden, wobei die restlichen zwei von R, $R_1$, $R_2$ und $R_3$ Wasserstoff sind,
oder $R_1$ bis $R_3$ Wasserstoff sind und R bei n = 1 eine Gruppierung

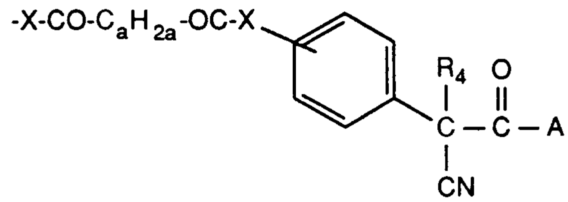

oder

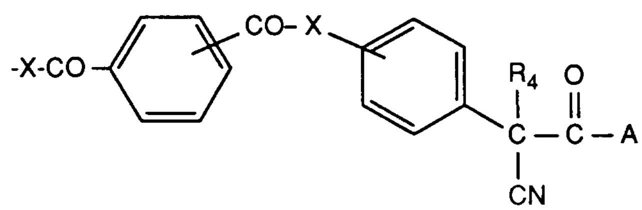

darstellt,
R′ und R″ unabhängig voneinander Wasserstoff, $C_1$-$C_{20}$-Alkyl, $C_2$-$C_{20}$-Alkenyl, Phenyl oder Naphthyl bedeuten oder zusammen mit dem Bindungs-N-Atom einen 5- oder 6-gliedrigen heterocyclischen Ring bilden,
a Null bis 10 ist, n 1 bis 6 ist,
X -O- oder -NH- bedeutet,
$R_4$ Wasserstoff oder bei n = 1 eine Gruppierung

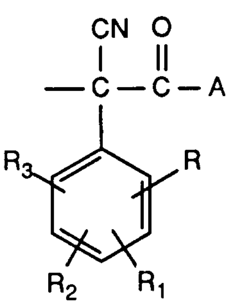

darstellt,
A bei n = 1, -OH, $-OR_5$, $-NR_6R_7$, $-NH-NR_8R_9$, $C_1$-$C_{30}$-Alkyl, $C_2$-$C_{30}$-Alkenyl, $C_5$-$C_{12}$-cycloalkyl, $C_1$-$C_4$-Alkyl-$C_5$-$C_{12}$-cycloalkyl,

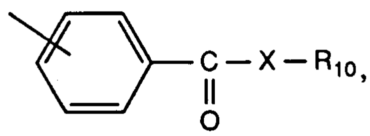

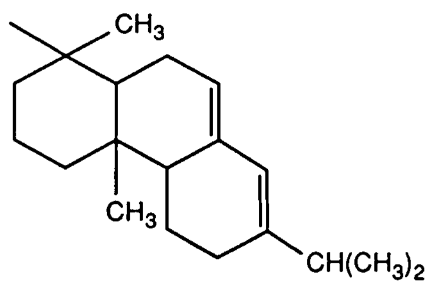

oder das entsprechende Dihydro- oder Tetrahydroderivat, $-C_aH_{2a}$-CO-X-$R_{10}$, $-C_bH_{2b}$-OCO-$R_{10}$, $-C_bH_{2b}$-OH,

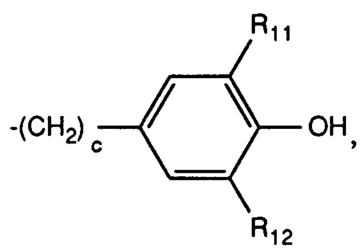

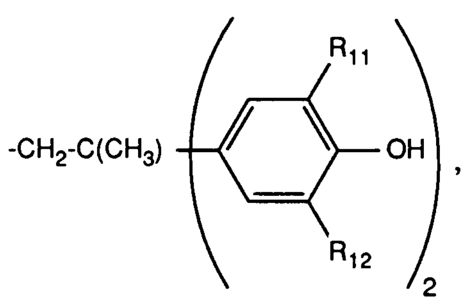

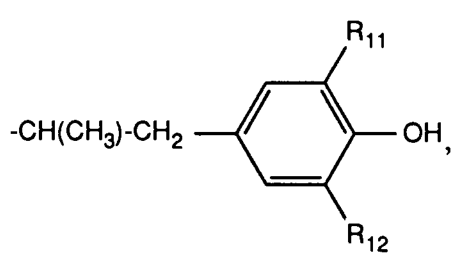

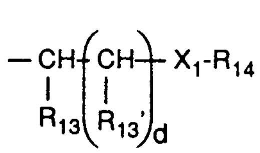

oder

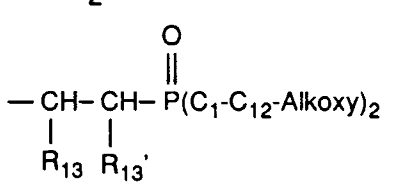

bedeutet, wobei
$R_5$ $C_1$-$C_{30}$-Alkyl, $C_2$-$C_{30}$-Alkenyl, $C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl, Biphenyl, der Rest eines Terpenalkohols oder ein Rest der Formeln

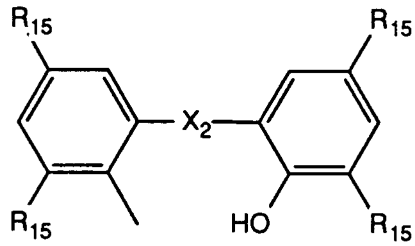, 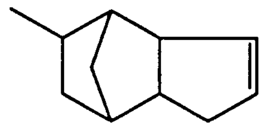, 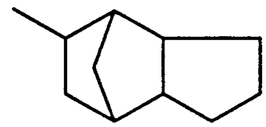,

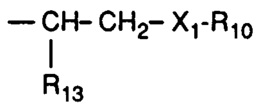 oder 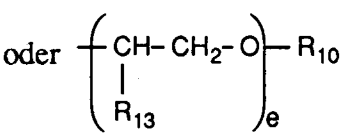

ist,
$R_6$ und $R_7$ unabhängig voneinander Wasserstoff, $C_1$-$C_{20}$-Alkyl, $C_2$-$C_{20}$-Alkenyl, $C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl,

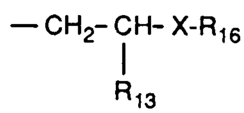

oder $-CH_2C(CH_2-O-R_{16})_3$ sind oder $R_6$ bei $R_7$ = H auch $-(CH_2)_f-X-R_{16}$ sein kann oder $R_6$ und $R_7$ zusammen mit dem N-Bindungsatom einen 5- oder 6-gliedrigen heterocyclischen Ring bilden,
$R_8$ und $R_9$ unabhängig voneinander Wasserstoff, $C_1$-$C_{20}$-Alkyl, Phenyl, -CO-$C_1$-$C_{20}$-Alkyl, -CO-$C_2$-$C_{18}$-Alkenyl, -CO-Phenyl oder -CO-Naphthyl sind oder zusammen $=CH-R_{17}$ darstellen,
$R_{10}$ Wasserstoff, $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl, Naphthyl, -CO-$C_1$-$C_{20}$-Alkyl, -CO-Phenyl, -CO-Naphthyl oder

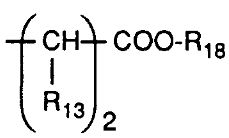

ist,
$R_{11}$ und $R_{12}$ unabhängig voneinander Wasserstoff, $C_1$-$C_4$-Alkyl, Cyclohexyl oder Phenyl sind,
$R_{13}$ Wasserstoff oder Methyl und $R_{13}'$ Wasserstoff, Methyl oder Phenyl sind,
$R_{14}$ $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl oder Naphthyl darstellt,
die $R_{15}$ unabhängig voneinander $C_1$-$C_4$-Alkyl sind,
$R_{16}$ Wasserstoff, -CO-$C_1$-$C_{20}$-Alkyl, -CO-$C_2$-$C_{20}$-Alkenyl, -CO-Phenyl oder -CO-Naphthyl darstellt,
$R_{17}$ $C_1$-$C_{20}$-Alkyl, $C_2$-$C_{20}$-Alkenyl, $C_5$-$C_{12}$-Cycloalkyl, Phenyl, Naphthyl, Furyl-2,

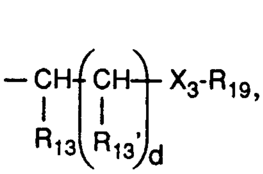, 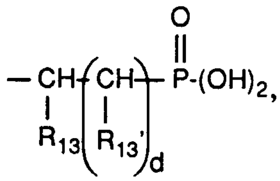, 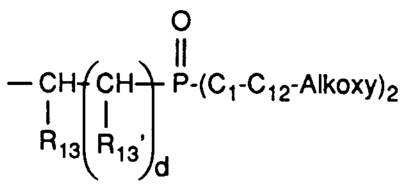

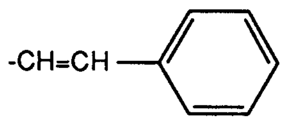, 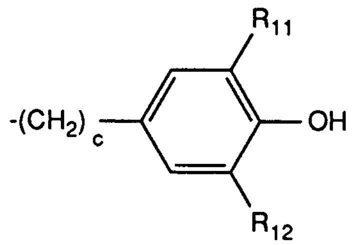 oder 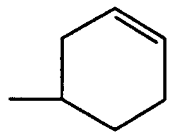

ist,
$R_{18}$ Wasserstoff, $C_1$-$C_{20}$-Alkyl oder $C_2$-$C_{20}$-Alkenyl darstellt,
$R_{19}$ $C_1$-$C_{20}$-Alkyl, $C_5$-$C_{12}$-Cycloalkyl, Phenyl oder $-CH_2$-COO-$C_{12}$-$C_{20}$-Alkyl ist,
X und a die oben angegebene Bedeutung haben,
$X_1$ -O-, -S-, -NH- oder $-NR_{14}$-,
$X_2$ die direkte Bindung, $-CH_2$-, $>$CH-$CH_3$ oder -S- und
$X_3$ -O-,-S-, -NH- oder $NR_{19}$- sind,
b 3 bis 5,
c 0 bis 2,
d 0 oder 1,
e 2 bis 10 und
f 2 bis 6 sind;
oder A, bei n = 2, -X'-$C_gH_{2g}$-X'-,

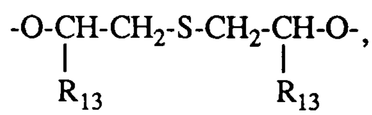

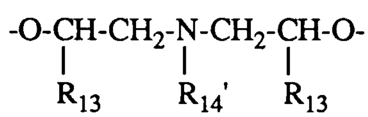, 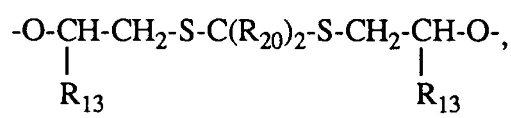

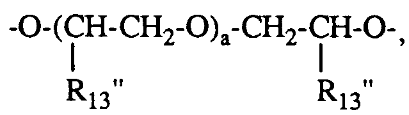

$-OCH_2CH=CHCH_2O-$, $-OCH_2C\equiv CCH_2O-$, $-O(CH_2)_h-NH-CO-X_4-OC-NH(CH_2)_h-O-$, $-NH(CH_2)_2-X(CH_2)_2-O-$ ,$-NH(CH_2CH_2NH)_i-CH_2CH_2NH-$, $-NH(CH_2CH_2CH_2NH)_iCH_2CH_2CH_2NH-$,

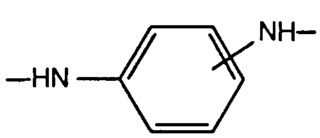, 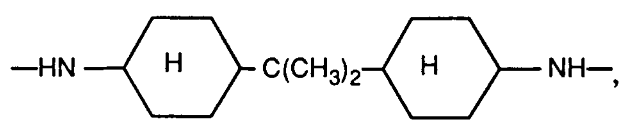

$-NH(CH_2)_h-O-CO-X_4-OC-O-(CH_2)_h-NH-$, -NH-NH-, $-NH-NH-CO-X_4-OC-NH-NH-$, $-NH-N=CH-X_5-CH=N-NH-$, $-C_aH_{2a}-$, $-(CH_2)_m-X_1-(CH_2)_m-$, Phenylen,

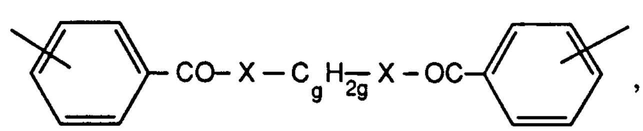,

$-C_pH_{2p}-CO-X-C_gH_{2g}-X-OC-C_pH_{2p}-$, $-(CH_2)_k-O-CO-X_4-CO-O-(CH_2)_k-$, $-CH=CH-CO-X-C_gH_{2g}-X-OC-CH=CH-$, $-CH(OH)CH_2-$, $-CH(OH)CH(OH)-$,

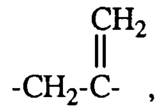 ,

$-CH=CH-$, $-CH(R_{21})$-oder

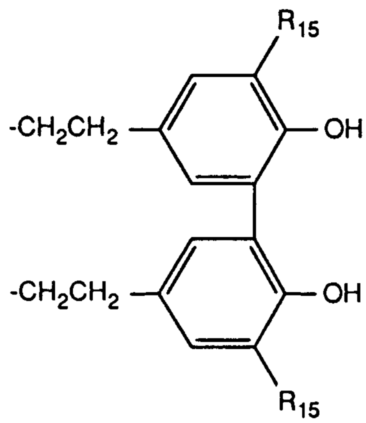

darstellt,
wobei X, $R_{13}$, $R_{15}$, a und f die oben angegebene Bedeutung haben,
die X' unabhängig voneinander -O- oder -NH- bedeuten,
$R_{13}''$ Wasserstoff oder Methyl ist und bei a = Null auch $C_2$-$C_{18}$-Alkyl oder Phenyl sein kann,
$R_{14}'$ dieselbe Bedeutung wie $R_{14}$ haben kann oder Wasserstoff ist,
die $R_{20}$ unabhängig voneinander Wasserstoff, $C_1$-$C_{20}$-Alkyl, Phenyl oder zusammen einen 5-12-gliedrigen cycloaliphatischen Ring darstellen,
$R_{21}$ $C_5$-$C_{12}$-Cycloalkyl, Phenyl-$C_1$-$C_4$-alkyl, Phenyl oder

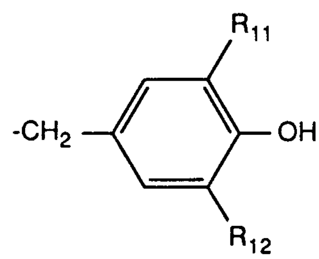

darstellt,
g 2 bis 12, h 2 bis 6, i 1 bis 5, k 3 bis 5, m 1 oder 2 und p 1 bis 10 sind,
$X_4$ $-C_aH_{2a}-$, Phenylen oder $-CH=CH-$ ist und
$X_5$ die direkte Bindung, $-(CH_2)_3-$ oder $-C(CH_3)_2-S-S-C(CH_3)_2-$ darstellt;
oder A, bei n = 3,

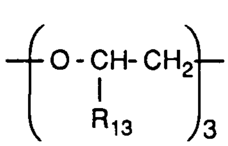 , 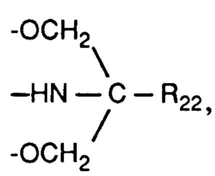 , 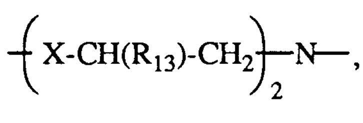 ,

$-(OCH_2)_3-C-R_{13}'$, $N(CH_2)_3-$ oder

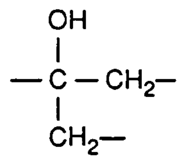

bedeutet, wobei $R_{13}$ und $R_{13}'$ die oben angegebene Bedeutung haben und $R_{22}$ Methyl oder Ethyl ist; oder A, bei n = 4, $C(CH_2O)_4-$,

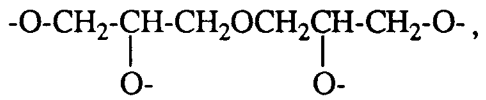

$-(OCH_2)_3-C-NH-$ oder $-(CH_2)_2NCH_2CH_2N(CH_2)_2-$ ist; oder A, bei n = 5,

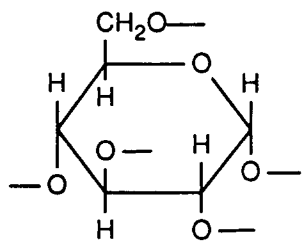

darstellt und bei n = 6

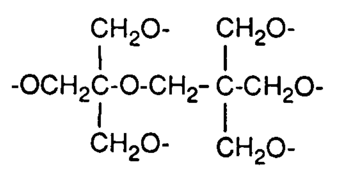

ist.

Durch irgendwelche Reste dargestellte Alkyl-, Alkoxy- oder Alkenylgruppen oder in solchen Resten enthaltene Alkyl-, Alkoxy- oder Alkenylteile können geradkettig oder verzweigt sein.

Als Beispiele von Alkylgruppen A, R, R′, R″, $R_1$, $R_5$ bis $R_{10}$, $R_{14}$, $R_{14}'$ und $R_{17}$ bis $R_{20}$ mit bis zu 20 bzw. bis zu 30 C-Atomen (A bzw. $R_5$) seien genannt: Methyl, Ethyl, Propyl, Isopropyl, n-Butyl, Isobutyl, t-Butyl, Pentyl, Isopentyl, Hexyl, Heptyl, 3-Heptyl, Octyl, Nonyl, Decyl, Undecyl, Dodecyl, Tridecyl, Tetradecyl, Pentadecyl, Hexadecyl, Heptadecyl, 2-Ethylbutyl, 1 -Methylpentyl, 1,3-Dimethylbutyl, 1, 1,3,3-Tetramethylbutyl, 1-Methylhexyl, 2-Ethylhexyl, 1 -Methylheptyl, 1, 1,3-Trimethylhexyl, 1 - Methylundecyl, Eicosyl, Heneicosyl, Docosyl, Tricosyl, Tetracosyl, Pentacosyl, Octacosyl und Tricontyl.

Alkylgruppen R, R′, R″, $R_1$ und $R_{20}$ sowie Alkylteile in Resten R oder $R_1$ = - $S-C_1-C_{20}$-Alkyl, $-O-CO-C_1-C_{20}$-Alkyl, $-CO-O-C_1-C_{20}$-Alkyl und $-NHCO-C_1-C_{20}$-Alkyl weisen bevorzugt 1-12 und insbesondere 1-4 C-Atome auf. Besonders bevorzugt sind, einschliesslich der Alkylgruppen $R_2$, geradkettige Alkylgruppen mit 1-4 C-Atomen, besonders Ethyl und vor allem Methyl.

Alkylgruppen A, $R_5$ bis $R_{10}$, $R_{14}$, $R_{14}'$, $R_{17}$, $R_{18}$ und $R_{19}$ oder in Resten $R_8$, $R_9$ oder $R_{16}$ enthaltene Alkylteile sind bevorzugt geradkettig und weisen insbesondere 1-18, vor allem 8- 18 C-Atome auf. Alkylgruppen $R_{17}$ sind insbesondere geradkettige Alkylgruppen mit 1-11C-Atomen.

Als Beispiele von Alkenylgruppen A, R, R′, R″, $R_1$, $R_5$ bis $R_7$, $R_{10}$, $R_{14}$, $R_{14}'$, $R_{17}$ und $R_{18}$ oder für in solchen Gruppen oder in Resten $R_8$, $R_9$ und $R_{16}$ enthaltene Alkenylteile mit 2- 18 ($R_8$, $R_9$), 2-20 bzw. 2-30 C-Atomen (A und $R_5$) seien erwähnt: Vinyl, Allyl, Methallyl, Hexenyl, Decenyl, Undecenyl, Heptadecenyl

und Oleyl.

Alkenylgruppen R, R', R" und $R_1$ weisen bevorzugt 3-6 C-Atome auf, während Alkenylgruppen A, $R_5$ bis $R_7$, $R_{10}$, $R_{14}$, $R_{14}'$, $R_{17}$ und $R_{18}$ sowie Alkenylteile in Resten $R_8$, $R_9$ und $R_{16}$ mit Vorteil 2-18 C-Atome aufweisen.

Beispiele für $C_5$-$C_{12}$-Cycloalkylgruppen A, R, $R_1$, $R_5$, $R_6$, $R_7$, $R_{14}$, $R_{14}'$, $R_{17}$, $R_{19}$ und $R_{21}$ sowie $C_1$-$C_4$-Alkyl-$C_5$-$C_{12}$-cycloalkylgruppen A, R, $R_1$, $R_5$, $R_6$ und $R_7$ sind: Cyclopentyl, Cyclohexyl, Methylcyclohexyl, 4-Butylcyclohexyl, Cycloheptyl, Cyclooctyl und Cyclododecyl. $C_5$-$C_7$-Cycloalkyl und Methyl-$C_5$-$C_7$-cycloalkyl, besonders Cyclopentyl, Methylcyclohexyl und vor allem Cyclohexyl sind bevorzugt.

$R_5$, $R_6$, $R_7$, $R_{10}$, $R_{14}$, $R_{14}'$ und $R_{21}$ als Phenyl-$C_1$-$C_4$-alkyl sind besonders Phenylethyl und vor allem Benzyl.

Alkoxygruppen R und $R_1$ oder Alkoxysubstituenten in Gruppen A können geradkettig oder verzweigt sein. Beispiele hierfür sind: Methoxy, Ethoxy, Propoxy, Isopropoxy, Butoxy, Isobutoxy, Pentyloxy, Hexyloxy, Heptyloxy, Octyloxy, Decyloxy, Dodecyloxy, Tetradecyloxy, Hexadecyloxy und Octadecyloxy. Bevorzugt sind geradkettige Alkoxygruppen mit 1 bis 4 C-Atomen, besonders Ethoxy und vor allem Methoxy.

Bilden R' und R" oder $R_6$ und $R_7$ zusammen mit dem N-Bindungsatom einen 5- oder 6-gliedrigen heterocyclischen Ring, so handelt es sich z.B. um einen Pyrolidin-, Piperidin-, 2,6-Dimethylpiperidin-, Piperazin-, 4-Methylpiperazin- oder Morpholinring.

Stellen R, $R_1$, $R_2$ oder $R_3$ Halogen dar, so handelt es sich z.B. um Fluor- oder Bromatome, insbesondere um Chloratome.

$R_2$ und $R_3$ sind bevorzugt Wasserstoff, R und $R_1$ stellen vorzugsweise unabhängig voneinander Wasserstoff, $C_1$-$C_4$-Alkyl, $C_1$-$C_4$-Alkoxy oder Halogen, besonders Chlor, dar. Besonders bevorzugt sind $R_1$, $R_2$ und $R_3$ Wasserstoff und R ist Wasserstoff, $C_1$-$C_4$-Alkyl, besonders Methyl, $C_1$-$C_4$-Alkoxy, besonders Methoxy, oder Chlor. Ganz besonders bevorzugt sind R, $R_1$, $R_2$ und $R_3$ Wasserstoff.

Bedeutet $R_4$ eine Gruppierung

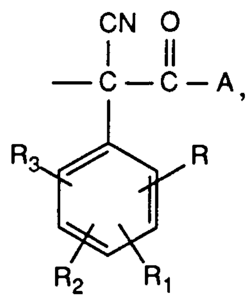

so gelten für R, $R_1$, $R_2$ und $R_3$ die oben angegebenen bevorzugten Bedeutungen und A ist vorzugsweise -O$C_1$-$C_{18}$-Alkyl, besonders -O$C_1$-$C_4$-Alkyl.

n ist vorzugsweise 1 oder 2.

$R_{11}$ ist vorzugsweise Wasserstoff, Methyl oder tert.Butyl und $R_{12}$ ist insbesondere Methyl oder tert.Butyl. Besonders bevorzugt ist eines von $R_{11}$ und $R_{12}$ Methyl oder tert.Butyl und das andere tert.Butyl.

A als Alkyl oder Alkenyl weist bevorzugt 1-17 bzw. 2-17 C-Atome auf. Bedeutet A eine Gruppe

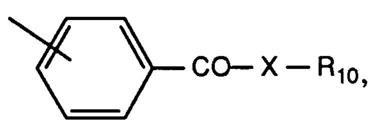

so ist sie bevorzugt in m- oder p-Stellung an die Carbonylgruppe gebunden. X ist dabei insbesondere -O- und $R_{10}$ stellt bevorzugt Wasserstoff, Cyclohexyl, Methylcyclohexyl, Benzyl oder $C_2$-$C_{18}$-Alkenyl und insbesondere Alkyl mit 1-18 und vor allem 1-4 C-Atomen dar.

Gruppierungen $-C_aH_{2a}-$, $-C_bH_{2b}-$, $-C_gH_{2g}-$ und $-C_pH_{2p}-$ können geradkettig oder verzweigt sein. Bevorzugt sind diese Gruppen geradkettig oder stellen bei n = 2 und A = $-X'-C_gH_{2g}-X'-$ $-CH_2C(CH_3)_2CH_2-$ dar.

c in Gruppen

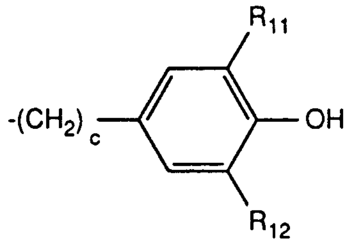

ist bevorzugt 1 und vor allem 2.

d ist vorzugsweise Null. $R_{13}$, $R_{13'}$ und $R_{13''}$ stellen bevorzugt Wasserstoff dar.

In Gruppen $-C_aH_{2a}-CO-X-R_{10}$ ist a bevorzugt 1-10, X stellt insbesondere -O- dar und $R_{10}$ ist Wasserstoff, $C_1$-$C_{18}$-Alkyl, $C_2$-$C_{18}$-Alkenyl, Cyclohexyl, Methylcyclohexyl oder Benzyl. In Gruppen $-C_bH_{2b}-OCO-R_{10}$ ist X ebenfalls bevorzugt -O-, während $R_{10}$ bevorzugt $C_1$-$C_{17}$-Alkyl, $C_2$-$C_{17}$-Alkenyl oder Phenyl bedeutet.

$X_1$ und $X_2$ sind bevorzugt -O- und insbesondere -S-. $X_2$ ist insbesondere die direkte Bindung oder $>CH-CH_3$. $X_4$ stellt vorzugsweise $-(CH_2)_a-$ dar, wobei a bevorzugt 2-8 und insbesondere 4-8 ist. $X_5$ ist vorzugsweise $-C(CH_3)_2-S-S-C(CH_3)_2-$.

$R_{14}$ ist bevorzugt Alkyl mit 1-18 und insbesondere 8-18 C-Atomen.

Als Reste von Terpenalkoholen $R_5$ kommen insbesondere Reste von acyclischen oder monocyclischen Verbindungen in Betracht, wie z.B. Reste des Geraniols, Nerols, Linalools, Citronellols, Thymols oder Menthols.

$R_{10}$ in Gruppen $R_5 = -CH(R_{13})-CH_2-X_1-R_{10}$ oder

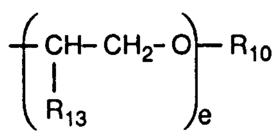

ist bevorzugt Wasserstoff, $C_1$-$C_{18}$-Alkyl oder $C_2$-$C_{18}$-Alkenyl. e ist dabei insbesondere 2-6 und $X_1$ und $R_{13}$ haben die oben angegebene bevorzugte Bedeutung.

$R_5$ stellt vorzugsweise $C_1$-$C_{18}$-Alkyl, $C_2$-$C_{18}$-Alkenyl, Cyclohexyl, Methylcyclohexyl, Benzyl, eine Gruppierung $-CH(R_{13})-CH_2-X_1-R_{10}''$ oder

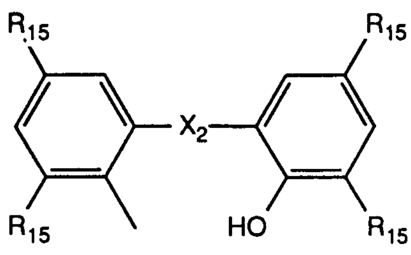

dar. Dabei ist $R_{13}$ bevorzugt Wasserstoff, $X_1$ ist bevorzugt -O- und insbesondere -S- und $R_{10}''$ ist Wasserstoff, $C_1$-$C_{18}$-Alkyl oder $C_2$-$C_{18}$-Alkenyl. Ganz besonders bevorzugt ist $R_5$ $C_1$-$C_{18}$-Alkyl, vor allem $C_8$-$C_{18}$-Alkyl, Cyclohexyl, Methylcyclohexyl, Benzyl oder eine Gruppierung

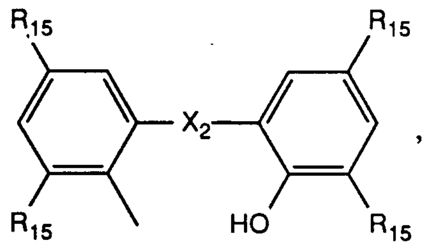
,

worin $X_2$ die direkte Bindung oder $>CH=CH_3$ bedeutet und die $R_{15}$ unabhängig voneinander Methyl oder tert.Butyl sind.

Von $R_6$ und $R_7$ stellt bevorzugt eines Wasserstoff und das andere $C_1$-$C_{18}$-Alkyl, $C_2$-$C_{18}$-Alkenyl, Cyclohexyl, Methylcyclohexyl, Benzyl oder eine Gruppe $-CH_2CH_2-X-R_{16}$ mit X = -O- und $R_{16}$ = Wasserstoff, -CO-$C_1$-$C_{18}$-Alkyl oder -CO-$C_2$-$C_{18}$-Alkenyl dar.

Von $R_8$ und $R_9$ ist bevorzugt eines Wasserstoff und das andere Wasserstoff, $C_1$-$C_{12}$-Alkyl, -CO-$C_1$-$C_{18}$-Alkyl oder -CO-$C_2$-$C_{18}$-Alkenyl oder $R_8$ und $R_9$ sind zusammen $=CH-R_{17}$, worin $R_{17}$ vorzugsweise $C_1$-$C_{11}$-Alkyl, $C_2$-$C_{11}$-Alkenyl, $-(CH_2)_m$-S-$C_1$-$C_{18}$-Alkyl oder

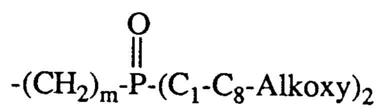

ist.

Besonders bevorzugt ist $R_9$ Wasserstoff und $R_8$ stellt Wasserstoff, -CO-$C_1$-$C_{18}$-Alkyl, besonders -CO-$C_8$-$C_{18}$-Alkyl, oder $-COCH=CH_2$ dar oder $R_8$ und $R_9$ sind zusammen $=CH-R_{17}$ mit $R_{17}$ = $C_1$-$C_{11}$ -Alkyl, $-(CH_2)_m$-S-$C_1$-$C_{18}$-Alkyl oder

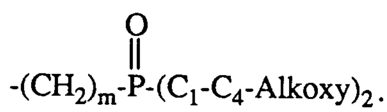

Dabei ist m vorzugsweise 2.

Bevorzugte Bedeutungen von $R_{14}$, $R_{15}$, $R_{16}$ und $R_{17}$ sind oben angegeben.

$R_{18}$ ist insbesondere Wasserstoff oder $C_1$-$C_{18}$-Alkyl, besonders $C_8$-$C_{18}$-Alkyl. $R_{19}$ stellt vorzugsweise $C_1$-$C_{18}$-Alkyl, besonders $C_8$-$C_{18}$-Alkyl dar.

Die X' haben bevorzugt dieselbe Bedeutung. $-C_gH_{2g}-$ ist insbesondere geradkettiges Alkylen mit 2-12 C-Atomen oder $-CH_2C(CH_3)_2CH_2-$.

$R_{14}'$ ist insbesondere $C_1$-$C_{18}$-Alkyl, besonders $C_8$-$C_{18}$-Alkyl und besonders bevorzugt Wasserstoff.

Die $R_{20}$ haben bevorzugt dieselbe Bedeutung und stellen insbesondere Wasserstoff oder Methyl dar.

h ist vorzugsweise 2 und $X_4$ ist vorzugsweise $-(CH_2)_a-$, worin a insbesondere 2-8 und besonders bevorzugt 4-8 ist. $X_5$ ist vorzugsweise $-C(CH_3)_2-S-S-C(CH_3)_2-$. k und f sind bevorzugt 5. $R_{21}$ ist bevorzugt Benzyl, Phenyl oder

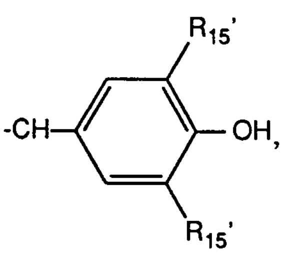

worin die $R_{15}'$ unabhängig voneinander Methyl oder tert.Butyl sind.

Bevorzugt sind erfindungsgemässe Zusammensetzungen, in welchen R und $R_1$ unabhängig voneinander Wasserstoff, $C_1$-$C_4$-Alkyl, $C_1$-$C_4$-Alkoxy oder Halogen, besonders Chlor, bedeuten und $R_2$ und $R_3$

Wasserstoff sind. Gemäss einer weiteren bevorzugten Ausführungsform werden Zusammensetzungen verwendet, in denen R Wasserstoff, $C_1$-$C_4$-Alkyl, besonders Methyl, $C_1$-$C_4$-Alkoxy, besonders Methoxy, oder Chlor ist und $R_1$, $R_2$ und $R_3$ Wasserstoff sind.

Von besonderem Interesse sind erfindungsgemässe Zusammensetzungen enthaltend Verbindungen der Formel I, worin R Wasserstoff, $C_1$-$C_4$-Alkyl, $C_1$-$C_4$-Alkoxy oder Chlor ist, $R_1$, $R_2$, $R_3$ und $R_4$ Wasserstoff sind, n 1 oder 2 ist,

A, bei n = 1, $-OR_5$, $-NHR_6$, $-NH-NH-R_8$, $-NH-N=CH-R_{17}$, $C_1$-$C_{17}$-Alkyl, $C_2$-$C_{17}$-Alkenyl,

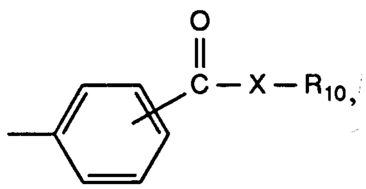

$-(CH_2)_pCO-X-R_{10}$, $CH_2-X_1-C_1-C_{18}$-Alkyl,

$$-(CH_2)_b-O-\underset{\underset{O}{\|}}{C}-R_{10}',$$

$-(CH_2)_b-OH$,

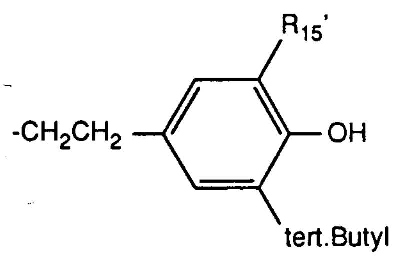 oder 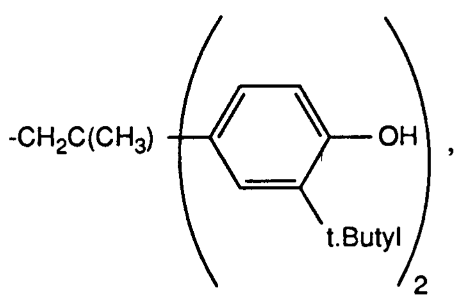 ,

$R_{10}$ Wasserstoff, $C_1$-$C_{18}$-Alkyl, $C_2$-$C_{18}$-Alkenyl, Cyclohexyl, Methylcyclohexyl oder Benzyl und

$R_{10}'$ $C_1$-$C_{17}$-Alkyl, $C_2$-$C_{17}$-Alkenyl oder Phenyl bedeuten,

$R_5$ $C_1$-$C_{18}$-Alkyl, $C_2$-$C_{18}$-Alkenyl, Cyclohexyl, Methylcyclohexyl, Benzyl,

$-\underset{\underset{R_{13}}{|}}{CH}-CH_2-X_1-R_{10}''$ oder 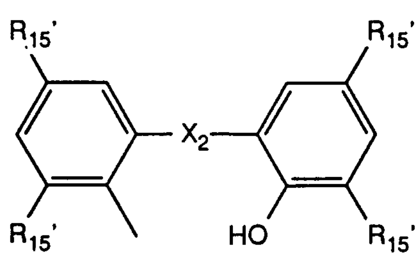

bedeutet, worin $R_{10}''$ Wasserstoff, $C_1$-$C_{18}$-Alkyl oder $C_2$-$C_{18}$-Alkenyl und die $R_{15}'$ unabhängig voneinander Methyl oder tert.Butyl sind,

$R_6$ $C_1$-$C_{18}$-Alkyl, $C_2$-$C_{18}$-Alkenyl, Cyclohexyl, Methylcyclohexyl, Benzyl, $-CH_2CH_2OH$,

$$-CH_2CH_2O-\underset{\underset{O}{\|}}{C}-C_1\text{-}C_{18}\text{-Alkyl oder } -CH_2CH_2O-\underset{\underset{O}{\|}}{C}-C_2\text{-}C_{18}\text{-Alkenyl}$$

ist,
$R_8$ Wasserstoff, $C_1$-$C_{12}$-Alkyl, -CO-$C_1$-$C_{18}$-Alkyl oder -CO-$C_2$-$C_{18}$-Alkenyl darstellt,
$R_{17}$ $C_1$-$C_{11}$-Alkyl, $C_2$-$C_{11}$-Alkenyl, -$(CH_2)_m$-S-$C_1$-$C_{18}$-Alkyl oder

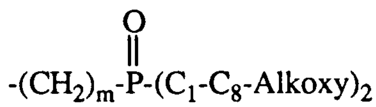

ist;
oder A, bei n = 2, -O-$(CH_2)_g$-O-, -NH-$(CH_2)_g$-NH-,

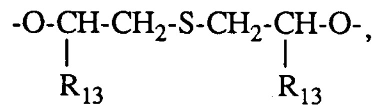

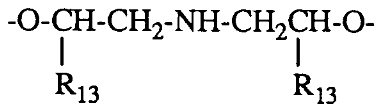 , 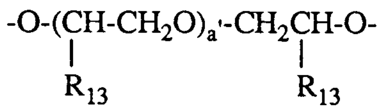

mit a' = Null bis 5, oder
-$OCH_2$-$C(CH_3)_2CH_2$-O-, -$NHCH_2CH_2$-X-$CH_2CH_2O$-, -$NH(CH_2CH_2NH)_i$-$CH_2CH_2NH$-, -$NH(CH_2CH_2CH_2NH)_i$-$CH_2CH_2CH_2$-NH-, -NH-$C(CH_3)_2$-$CH_2O$-

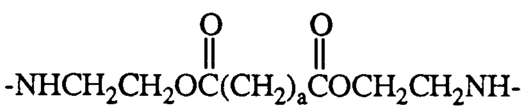 , 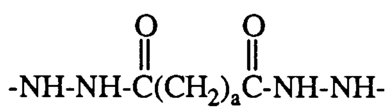

-NH-N=CH-$X_5$-CH=N-NH-, -$(CH_2)_p$-, Phenylen,

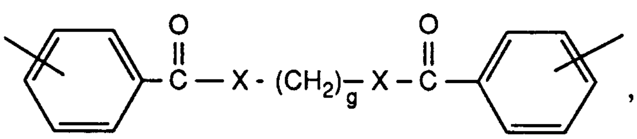 , 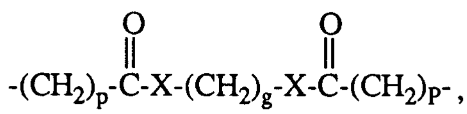

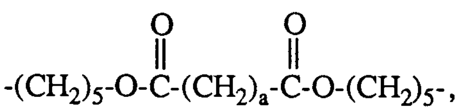

-CH($R_{21}$)- mit $R_{21}$ = Benzyl, Phenyl oder

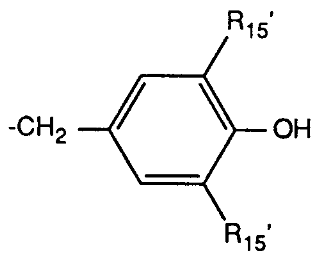 oder A 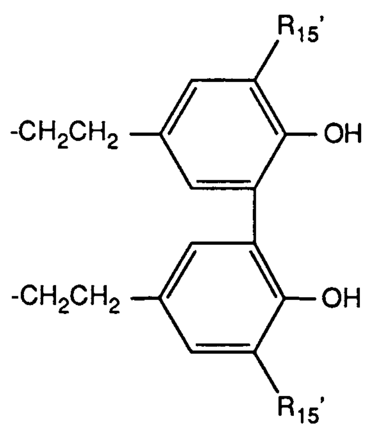

darstellt,
und a, b, g, i, m, p, X, $X_1$, $X_2$, $X_5$ und $R_{13}$ die oben angegebene Bedeutung haben.

Zweckmässige erfindungsgemässe Zusammensetzungen enthalten Verbindungen der Formel I, Worin R Wasserstoff, Chlor, Methyl oder Methoxy ist und $R_1$, $R_2$, $R_3$ und $R_4$ Wasserstoff sind,
n 1 oder 2 ist,
A, bei n = 1, $-OR_5$, $-NHR_6$, $-NH-NH-R_8$, $-NH-N=CH-R_{17}$, $C_1$-$C_{17}$-Alkyl, $-(CH_2)_p-COOH$,

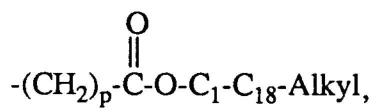

$-(CH_2)_bOH$, $-(CH_2)_b-OCO-C_1-C_{18}$-Alkyl, $-CH_2-S-C_1-C_{18}$-Alkyl,

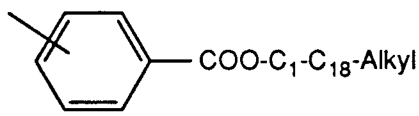 oder

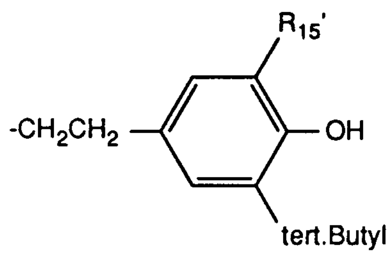

darstellt, wobei $R_5$ $C_1$-$C_{18}$-Alkyl, Cyclohexyl, Methylcylohexyl, Benzyl oder

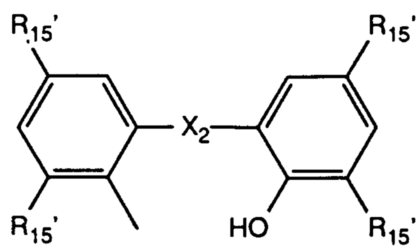

ist,
$R_6$ $C_1$-$C_{18}$-Alkyl, Cyclohexyl, Benzyl, $-(CH_2)_f-OH$ oder $-CH_2CH_2O-CO-C_1-C_{18}$-Alkyl bedeutet,
$R_8$ Wasserstoff, $-CO-C_1-C_{18}$-Alkyl oder $-COCH=CH_2$ ist,
die $R_{15}'$ unabhängig voneinander Methyl oder tert.Butyl sind,
$R_{17}$ $C_1$-$C_{11}$-Alkyl, $-(CH_2)m-S-C_1-C_{18}$-Alkyl oder

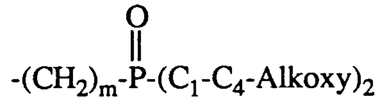

darstellt, oder A, bei n = 2, $-O-(CH_2)_g-O-$, $-NH-(CH_2)_g-NH-$, $-OCH_2CH_2SCH_2CH_2O-$, $-O(CH_2CH_2O)_c-CH_2CH_2-O-$, $OCH_2C(CH_3)_2CH_2O-$, $NHCH_2CH_2OCH_2CH_2O-$,

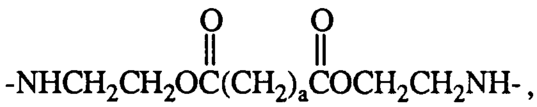, 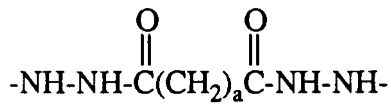,

-NH-N = CH-C($CH_3$)$_2$-S-S-C($CH_3$)$_2$-CH = N-NH-, Phenylen oder -($CH_2$)$_p$- bedeutet, wobei a, b, c, f, g, m, p und $X_2$ die oben angegebene Bedeutung haben.

Besonders bevorzugt sind Zusammensetzungen, worin R Chlor, Methyl oder Methoxy und insbesondere Wasserstoff darstellt, $R_1$, $R_2$, $R_3$ und $R_4$ Wasserstoff sind,
n 1 oder 2 ist,
A, bei n = 1, -$OR_5$, -$NHR_6$, -NH-NH-$R_8$, -NH-N = CH-$R_{17}$, $C_8$-$C_{17}$-Alkyl, -($CH_2$)$_{p'}$-CO-O-$C_8$-$C_{18}$-Alkyl mit p' = 2 bis 6, -($CH_2$)$_b$-OH, -($CH_2$)$_b$-OCO-$C_8$-$C_{18}$-Alkyl, -$CH_2$-S-$C_8$-$C_{18}$-Alkyl,

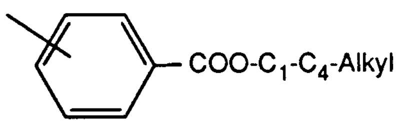 oder 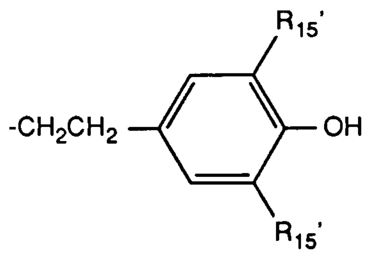

darstellt,

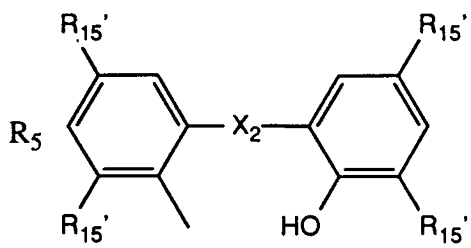

und insbesondere $C_8$-$C_{18}$-Alkyl ist,
$R_6$ $C_8$-$C_{18}$-Alkyl, -($CH_2$)$_{f''}$-OH mit f'' = 2 oder 3 oder -$CH_2CH_2OCO$-$C_8$-$C_{18}$-Alkyl bedeutet,
$R_8$ -CO-$C_8$-$C_{18}$-Alkyl oder -COCH = $CH_2$ ist,
$R_{17}$ $C_8$-$C_{11}$-Alkyl, ($CH_2$)$_m$-S-$C_8$-$C_{18}$-Alkyl oder

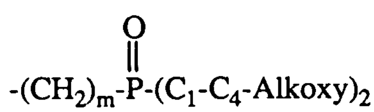

darstellt,
oder A, bei n = 2, -O($CH_2$)$_g$O-, -NH($CH_2$)$_g$NH-, -$OCH_2CH_2SCH_2CH_2O$-, -O($CH_2CH_2O$)$_c$$CH_2CH_2O$-, -$OCH_2C$($CH_3$)$_2$$CH_2O$-, -$NHCH_2CH_2OCH_2CH_2O$-,-$NHCH_2CH_2OCO$-($CH_2$)$_{a''}$-$OCOCH_2CH_2NH$- oder -NH-NHCO-($CH_2$)$_{a''}$-CONH-NH- mit
a'' = 2 bis 8, -NH-N = CH-C($CH_3$)$_2$-S-S-C($CH_3$)$_2$-CH = N-NH-, -($CH_2$)$_{p''}$- mit p'' = 5-10 oder Phenylen bedeutet,
b 3 bis 5, besonders 5 ist, $X_2$ die direkte Bindung oder $>$CH = $CH_3$ ist und $R_{15}'$, c, g und m die oben angegebene Bedeutung haben. Ganz besonders bevorzugt sind Zusammensetzungen, worin R, $R_1$, $R_2$, $R_3$ und $R_4$ Wasserstoff bedeuten, n 1 oder 2 ist,
A, bei n = 1, $C_8$-$C_{17}$-Alkyl, -$OR_5$, -$NHR_6$ oder -NH-NH-CO-$C_8$-$C_{17}$-Alkyl ist,
$R_5$ und $R_6$ unabhängig voneinander $C_8$-$C_{18}$-Alkyl darstellen,
oder A, bei n = 2, -O($CH_2$)$_g$-O-, -NH($CH_2$)$_g$NH-, -$OCH_2C$($CH_3$)$_2$$CH_2O$-, -NH-NHCO-$CH_2CH_2$-OCNH-NH-, -($CH_2$)$_{p''}$- mit p'' = 5 bis 10 oder Phenylen bedeutet, wobei g die oben angegebene Bedeutung hat.

Ebenfalls bevorzugt sind Zusammensetzungen enthaltend eine Verbindung der Formel Ia

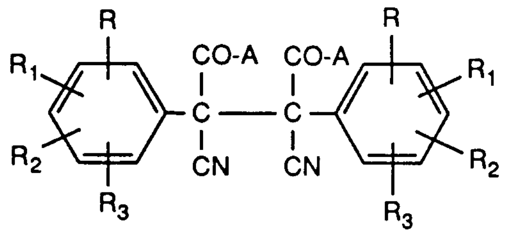

(Ia),

worin R Wasserstoff, $C_1$-$C_4$-Alkyl, $C_1$-$C_4$-Alkoxy oder Chlor ist, $R_1$, $R_2$ und $R_3$ Wasserstoff sind und A -$OC_1$-$C_{18}$-Alkyl, besonders -$OC_1$-$C_4$-Alkyl bedeutet, insbesondere solche mit Verbindungen der Formel Ia, worin R, $R_1$, $R_2$ und $R_3$ Wasserstoff sind und A -$OC_1$-$C_4$-Alkyl bedeutet.

Die in den erfindungsgemässen Zusammensetzungen enthaltenen Materialien sind solche, die gegen oxidativen, thermischen oder/und aktinischen Abbau empfindlich sind. Als nicht unter diese organischen Materialien fallend sind lebende Organismen zu verstehen.

Beispielhaft für organische Materialien, die erfindungsgemäss mit Hilfe der Verbindungen der Formel I stabilisiert werden können, seien erwähnt:

1. Polymere von Mono- und Diolefinen, beispielsweise Polypropylen, Polyisobutylen, Polybuten- 1, Polymethylpenten- 1, Polyisopren oder Polybutadien sowie Polymerisate von Cycloolefinen wie z.B. von Cyclopenten oder Norbomen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten- 1 -Copolymere, Propylen-Isobutylen-Copolymere, Ethylen-Buten- 1 -Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbomen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder a-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo-und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, - benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xyloldiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyhamstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly- 1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate,

POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die erfindungsgemässen Zusammensetzungen enthalten als organisches Material vorzugsweise einen Schmierstoff, eine Metallbearbeitungsflüssigkeit, eine Hydraulikflüssigkeit, ein natürliches, halbsynthetisches oder synthetisches Polymer. Bevorzugt sind halogenfreie Polymere. Besonders bevorzugt sind Zusammensetzungen, die einen halogenfreien thermoplastischen Kunststoff oder ein Elastomer enthalten. Gemäss einer weiteren bevorzugten Ausführungsform ist das organische Material ein Polyolefin. Beispiele für solche Polymere sind der vorstehenden Aufzählung von geeigneten Materialien zu entnehmen.

Ebenfalls bevorzugt sind Zusammensetzungen, die als organisches Material einen Schmierstoff, eine Metallbearbeitungsflüssigkeit oder eine Hydraulikflüssigkeit, insbesondere einen Schmierstoff, enthalten.

Die in Frage kommenden Schmierstoffe basieren beispielsweise auf mineralischen oder synthetischen Oelen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in ScheweKobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Oele und Fette, beispielsweise basierend auf einem Mineralöl. Bevorzugt sind Oele.

Eine weitere Gruppe von Schmierstoffen, die zur Anwendung gelangen können, sind pflanzliche oder tierische Oele, Fette, Talge und Wachse oder deren Gemische untereinander oder Gemische mit den erwähnten mineralischen oder synthetischen Oelen. Pflanzliche und tierische Oele, Fette, Talge und Wachse sind beispielsweise Palmkernöl, Palmöl, Olivenöl, Rueböl, Rapsöl, Leinöl, Erdnussöl, Sojabohnenöl, Baumwollöl, Sonnenblumenöl, Kürbiskernöl, Kokosnussöl, Maisöl, Rizinusöl, Baumnussöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-$\alpha$-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropantricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Caprylund Sebacinsäure oder von einem Gemisch davon. Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Metallbearbeitungsflüssigkeiten und Hydraulikflüssigketen können auf Basis der gleichen Substanzen hergestellt werden wie vorstehend für die Schmiermittel beschrieben. Häufig handelt es sich dabei auch um Emulsionen solcher Substanzen in Wasser oder anderen Flüssigkeiten.

Die Verbindungen der Formel I werden dem organischen Material im allgemeinen in Mengen von 0,01 bis 10 Gew.%, z.B. in Mengen von 0,05 bis 5, vorzugsweise 0,05 bis 3 Gew.% und insbesondere in Mengen von 0,1 bis 2 Gew.%, bezogen auf das organische Material, zugesetzt. Es können auch Gemische verschiedener Verbindungen der Formel I eingesetzt werden.

Die Einarbeitung in die organischen Materialien kann beispielsweise durch Einmischen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, während oder unmittelbar nach

der Polymerisation der entsprechenden Monomeren bzw. vor der Vernetzung. Die Verbindungen der Formel I können dabei als solche, aber auch in encapsulierter Form (z.B. in Wachsen, Oelen oder Polymeren) zugesetzt werden. Im Falle der Zugabe vor oder während der Polymerisation können die Verbindungen der Formel I auch als Regulatoren für die Kettenlänge der Polymeren (Kettenabbrecher) wirken.

Die Verbindungen der Formel I oder Mischungen davon können auch in Form eines Masterbatches, der diese Verbindungen beispielsweise in einer Konzentration von 2,5 bis 25 Gew.-% enthält, den zu stabilisierenden Kunststoffen zugesetzt werden.

Zweckmässig kann die Einarbeitung der Verbindungen der Formel I nach folgenden Methoden erfolgen:

- als Emulsion oder Dispersion (z.B. zu Latices oder Emulsionspolymeren)
- Als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Erfindungsgemässe Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Erfindungsgemässe Schmierstoffzusammensetzungen finden z.B. Verwendung in Verbrennungsmotoren, z.B. in Kraftfahrzeugen.

Zusätzlich zu den Verbindungen der Formel I bzw. Mischungen davon können die erfindungsgemässen Zusammensetzungen, insbesondere wenn sie organische, vorzugsweise synthetische, Polymere enthalten, noch weitere Stabilisatoren, besonders Antioxidantien, Lichtschutzmittel und Verarbeitungsstabilisatoren (Hitzestabilisatoren) enthalten. Beispiele für solche Additive sind:

1. Antioxidantien

1. 1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert.-butylphenol), 4,4′-Methylen-bis-(6-tert.butyl-2-methylphenol), 1, 1-Bis-(5-tert.butyl-4-hydroxy-2-methylphenyl)-butan, 2,6-Bis-(3-tert.butyl-5-methyl-2-hydroxybenzyl)-4-methylphenol, 1, 1,3-Tris-(5-tert.butyl-4-hydroxy-2-methylphenyl)-butan, 1, 1-Bis-(5-tert.butyl-4-hydroxy-2-methyl-phenyl)-3-n-dodecylmercaptobutan, Ethylenglycol-bis-[3,3-bis-(3′-tert.-butyl-4′hydroxyphenyl)-butyrat], Bis-(3-tert.butyl-4-hydroxy-5-methyl-phenyl)-dicyclopentadien, Bis-[2-(3′-tert.butyl-2′-hydroxy-5′-methyl-benzyl)-6-tert.butyl-4-methyl-phenyl] -terephthalat.

1.5 Benzylverbindungen, z.B. 1,3,5-Tris-(3,5-di-tert.butyl-4-hydroxy-benzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5 -Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino )-s-triazin, N-(3,5-di-tert.-butyl-4-hydroxyphenyl)-carbaminsäureoctylester.

1.7. Ester der β-(3,5-Di-tert.butyl-4-hydroxyphenyl)-propionsäure mit ein-oder mehrwertigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris-(hydroxyethyl)-isocyanurat, N,N′-Bis-(hydroxyethyl)-oxalsäurediamid.

1. 8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.9. Ester der β-(3,5-Dicyclohexyl-4-hydroxyphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N′-Bis-(hydroxyethyl)-oxalsäurediamid.

1.10. Amide der β-(3,5-Di-tert.butyl-4-hydroxyphenyl)-propionsäure, wie z.B. N,N′-wie z.B. N,N' -Bis-(3,5-di-tert.butyl-4-hydroxyphenylpropionyl)-hexamethylendiamin, N,N′-Bis-(3,5-di-tert.-butyl-4-hydroxyphenylpropionyl)-trimethylendiamin, N,N′-Bis-(3,5-di-tert.butyl-4-hydroxyphenylpropionyl)-hydrazin.

2. UV-Absorber und Lichtschutzmittel

2. 1. 2-(2′-Hydroxyphenyl)-benztriazole, wie z.B. das 5′-Methyl-, 3′,5′-3',5'-Di-tert.butyl-, 5′-tert.Butyl-, 5′-(1, 1,3,3-Tetramethylbutyl)-, 5-Chlor-3′,5′-di-tert.butyl-, 5-Chlor-3′-tert.-butyl-5′-methyl-, 3′-sec.Butyl-5′-tert.butyl, 4′-Octoxy-, 3′,5′-Di-tert.amyl-, 3′,5′-Bis(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2.3. Ester von gegebenenfalls substituierten Benzoesäuren, wie z.B. 4-tert.Butyl-phenyl-salicylat, Phenylsalicylat, Octylphenyl-salicylat, Dibenzoylsesorcin, Bis-(4-tert.butyl-benzoyl)-resorcin, Benzoylsesorcin, 3,5-Di-tert.butyl-4-hydroxybenzoesäure-2,4-di-tert.-butylphenylester, 3,5-Di-tert.butyl-4-hydroxybenzoesäurehexadecylester.

2.4. Acrylate, wie z.B. α-Cyan-β,β-diphenylacrylsäure-ethylester bzw. - isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbo-methoxy-β-cyanovinyl)-2-methyl-indolin.

2.5. Nickelverbindungen, wie z.B. Nickelkomplexe des 2,2′-Thio-bis-[4-(1, 1,3,3-tetramethylbutyl)-phenols], wie der 1: 1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzylmalonsäure-bis( 1,2, 2,6,6-pentamethylpiperidyl)-ester, Kondensationsprodukt aus 1 -Hydroxyethyl-2,2,6,6-tetramethyl-4-hydroxypiperidin und Bernsteinsäure, Kondensationsprodukt aus N,N′-Bis-(2,2,6,6-Tetramethyl-4-piperidyl)-hexamethylendiamin und 4-tert.Octylamino-2,6-dichlor- 1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1, 1′-( 1,2-Ethandiyl)-bis-(3,3,5,5-tetramethyl-piperazinon).

2.7. Oxalsäurediamide, wie z.B. 4,4′-Di-octyloxy-oxanilid, 2,2′-Di-octyloxy-5,5′-di-tert.-butyl-oxanilid, 2,2′-Di-dodecyloxy-5,5′ di-tert.butyl-oxanilid, 2-Ethoxy-2′-ethyl-oxanilid, N,N′-Bis-(3-dimethylaminopropyl)-oxalamid, 2-Ethoxy-5-tert.butyl-2′-ethyloxanilid und dessen Gemisch mit 2-Ethoxy-2′-ethyl-5,4′-di-tert.butyl-oxanilid, Gemische von o-und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)- 1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.

3. Metalldesaktivatoren, wie z.B. N,N′-Diphenyloxalsäurediamid, N-Salicylal-N′-salicyloylhydrazin, N,N′-Bis-(salicyloyl)-hydrazin, N,N′-Bis-(3,5-di-tert.butyl-4-hydroxyphenylpropionyl)-hydrazi n, 3-Salicyloylamino- 1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit,Diisodecylpentaerythrit-diphosphit, Bis-(2,4-di-tert.butylphenyl)-pentaerythritdiphosphit, Tri-stearyl-sorbit-triphosphit, Tetrakis-(2,4-di-tert.butylphenyl)-4,4′-biphenylen-diphosphonit.

5. Peroxidzerstörende Verbindungen, wie z.B. Ester der β-Thio-dipropionsäure, beispiels-weise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.
8. Nukleierunpsmittel, wie z.B. 4-tert.Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.
10. Sonstige Zusätze, wie z.B. Weichmacher, Gleitmittel, Emulgatoren, Pigmente, Optische Aufheller, Flammschutzmittel, Antistatika, Treibmittel.

Handelt es sich bei den erfindungsgemässen Zusammensetzungen um solche auf Basis von Schmierstoffen und Hydraulikflüssigkeiten bzw. Metallbearbeitungsflüssigkeiten,können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Verschleissschutzadditive.

Beispiele für Antioxidantien sind der weiter oben wiedergegebenen Auflistung unter dem Titel "1. Antioxidantien", insbesondere Punkte 1.1 bis 1.10 zu entnehmen. Beispiele für weitere zusätzliche Additive sind die folgenden:

Beispiele für aminische Antioxidantien:

N,N′-Di-isopropyl-p-phenylendiamin, N,N′-Di-sec-butyl-p-phenylendiamin, N,N′-Bis-(1,4-dimethyl-pentyl)-p-phenylendiamin, N,N′-Bis( 1 -ethyl-3-methyl-pentyl)-p-phenylendiamin, N,N′-Bis(1-methyl-heptyl)-p-phenylendiamin, N,N′-Dicyclohexyl-p-phenylendiamin, N,N′-Diphenyl-p-phenylendiamin, N,N′-Di-(naphthyl-2)-p-phenylendiamin, N-Isopropyl-N′-phenyl-p-phenylendiamin, N-( 1,3-Dimethyl-butyl)-N′-phenyl-p-phenylendiamin, N-(1-Methyl-heptyl)-N′-phenyl-p-phenylendiamin, N-Cyclohexyl-N′-phenyl-p-phenylendiamin, 4-(p-Toluol-sulfonamido)-diphenylamin, N,N′-Dimethyl-N,N′-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p′-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methylphenol, 2,4′-Diamino-diphenylmethan, 4,4′-Diamino-diphenylmethan, N,N,N′,N′-Tetramethyl-4,4′-diamino-diphenylmethan, 1,2-Di-[(2-methyl-phenyl)-amino]-ethan, 1,2-Di-(phenylamino)-propan, (o-Tolyl)-biguanid, Di-[4-( 1′,3′-dimethyl-butyl)-phenyl]amin, tert-octyliertes N-Phenyl- 1 -naphthylamin, Gemisch aus mono- und dialkylierten tert-Butyl/tert-Octyldiphenylaminen, 2,3-Dihydro-3,3-dimethyl-4H- 1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol,5,5′-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbemsteinsäureanhydrid, z.B. Dodecenylbemsteinsäure-anhydrid, Alkenylbemsteinsäure-Teilester und -Teilamide, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.

d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbemsteinsäureamide oder -imide, Polybutenylphosphonsäurederivate, basische Magnesium-, Calcium-, und Bariumsulfonate und -phenolate.

Beispiele für Verschleissschutz-Additive sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Die Verbindungen der Formel I eignen sich insbesondere als Verarbeitungsstabilisatoren (Hitzestabilisatoren) für vorzugsweise halogenfreie synthetische Polymere oder Schmierstoffe. In den genannten Polymeren zeichnen sie sich zudem oft durch eine farbverbessemde Wirkung aus.

Die Erfindung betrifft ferner neue Verbindungen der Formel Ib

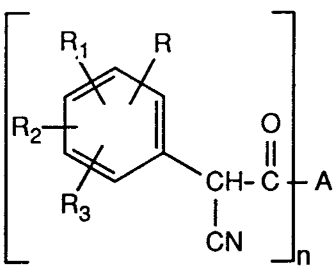

(Ib),

worin R Wasserstoff, -OH, Halogen, $C_1$-$C_{20}$-Alkyl, $C_2$-$C_{20}$-Alkenyl, $C_5$-$C_{12}$-Cycloalkyl, $C_1$-$C_4$-Alkyl-$C_5$-$C_{12}$-cycloalkyl, Phenyl, Naphthyl, $C_1$-$C_{20}$-Alkoxy, Phenyloxy, Naphthyloxy, -S-$C_1$-$C_{20}$-Alkyl, -S-Phenyl, -S-Naphthyl, -O-CO-$C_1$-$C_{20}$-Alkyl, -O-CO-Phenyl, -O-CO-Naphthyl, -COOH, -COO-$C_1$-$C_{20}$-Alkyl, -COO-$C_2$-$C_{20}$-Alkenyl, -COO-Phenol, -COO-Naphthyl, -CONR'R", -CO-$C_1$-$C_{20}$-Alkyl, -CO-Phenyl, -CO-Naphthyl, -NHCO-$C_1$-$C_{20}$-Alkyl, NHCO-$C_2$-$C_{20}$-Alkenyl, -NHCO-Phenyl oder -NHCO-Naphthyl bedeutet,
$R_1$ dieselbe Bedeutung wie R haben kann,
$R_2$ Wasserstoff, Halogen oder $C_1$-$C_4$-Alkyl ist und
$R_3$ Wasserstoff oder Halogen bedeutet,
oder zwei in ortho-Stellung zueinander gebundene Reste R, $R_1$, $R_2$ oder $R_3$ zusammen Tetramethylen oder -CH = CH-CH = CH- bilden, wobei die restlichen zwei von R, $R_1$, $R_2$ und $R_3$ Wasserstoff sind,
oder $R_1$ bis $R_3$ Wasserstoff sind und R bei n = 1 eine Gruppierung

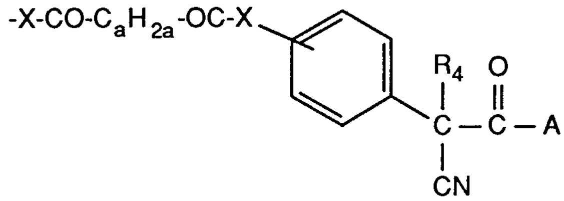 oder

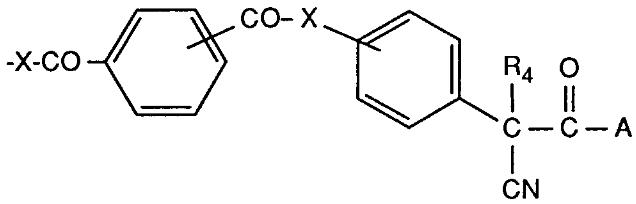

darstellt, worin $R_4$ jeweils H ist,

R' und R'' unabhängig voneinander Wasserstoff, $C_1$-$C_{20}$-Alkyl, $C_2$-$C_{20}$-Alkenyl, Phenyl oder Naphthyl bedeuten oder zusammen mit dem Bindungs-N-Atom einen 5- oder 6-gliedrigen heterocyclischen Ring bilden,

a Null bis 10 ist, n 1 bis 6 ist,

X -O- oder -NH- bedeutet,

A bei n = 1, $-OR_5$, $-NR_6R_7$, $-NH-NR_8R_9$, $C_1$-$C_{30}$-Alkyl, $C_2$-$C_{30}$-Alkenyl, $C_5$-$C_{12}$-Cycloalkyl, $C_1$-$C_4$-Alkyl-$C_5$-$C_{12}$-cycloalkyl,

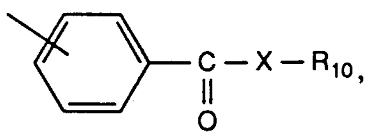

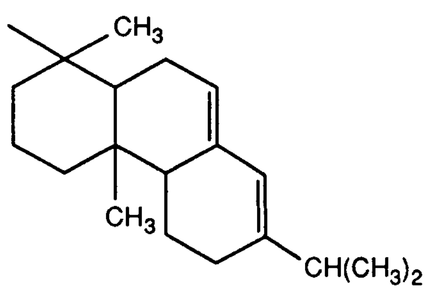

oder das entsprechende Dihydro- oder Tetrahydroderivat, $-C_aH_{2a}-CO-X-R_{10}$, $-C_bH_{2b}-OCO-R_{10}$, $-C_bH_{2b}-OH$,

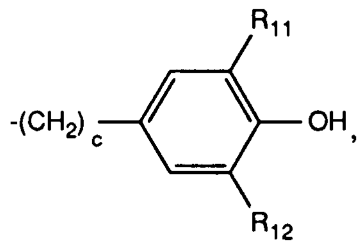

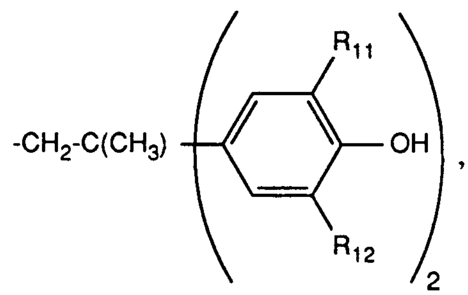 , 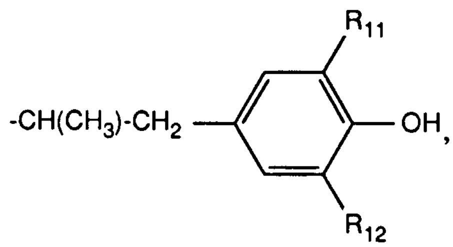

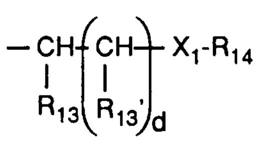 oder 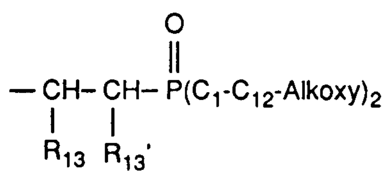

bedeutet, wobei

$R_5$ $C_1$-$C_{30}$-Alkyl, $C_2$-$C_{30}$-Alkenyl, -$C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl, Biphenyl, der Rest eines Terpenalkohols oder ein Rest der Formeln

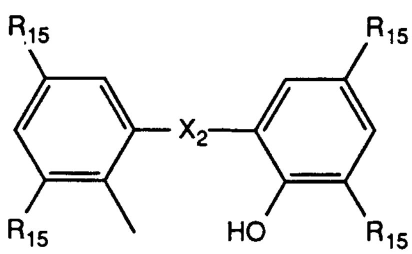 , 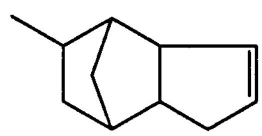 , 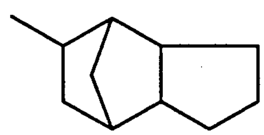 ,

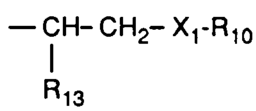 oder 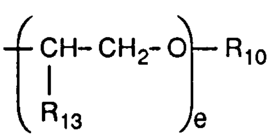

ist,

$R_6$ und $R_7$ unabhängig voneinander Wasserstoff, $C_1$-$C_{20}$ -Alkyl, $C_2$-$C_{20}$-Alkenyl, $C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl,

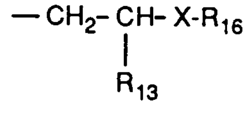

oder $-CH_2C(CH_2-O-R_{16})_3$ sind oder $R_6$ bei $R_7$ = H auch $-(CH_2)_f-X-R_{16}$ sein kann oder $R_6$ und $R_7$ zusammen mit dem N-Bindungsatom einen 5- oder 6-gliedrigen heterocyclischen Ring bilden,

$R_8$ und $R_9$ unabhängig voneinander Wasserstoff, $C_1$-$C_{20}$-Alkyl, Phenyl, $-CO-C_1-C_{20}$-Alkyl, $-CO-C_2-C_{18}$-Alkenyl, -CO-Phenyl oder -CO-Naphthyl sind oder zusammen $=CH-R_{17}$ darstellen,

$R_{10}$ Wasserstoff, $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl, Naphthyl, -CO-$C_1$-$C_{20}$-Alkyl, -CO-Phenyl, -CO-Naphthyl oder

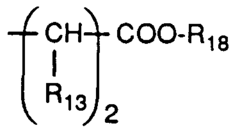

ist,
$R_{11}$ und $R_{12}$ unabhängig voneinander Wasserstoff, $C_1$-$C_4$-Alkyl, Cyclohexyl oder Phenyl sind,
$R_{13}$ Wasserstoff oder Methyl und $R_{13}'$ Wasserstoff, Methyl oder Phenyl sind,
$R_{14}$ $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl oder Naphthyl darstellt,
die $R_{15}$ unabhängig voneinander $C_1$-$C_4$-Alkyl sind,
$R_{16}$ Wasserstoff, -CO-$C_1$-$C_{20}$-Alkyl, -CO-$C_2$-$C_{20}$-Alkenyl, -CO-Phenyl oder -CO-Naphthyl darstellt,
$R_{17}$ $C_1$-$C_{20}$-Alkyl, $C_2$-$C_{20}$-Alkenyl, $C_5$-$C_{12}$-Cycloalkyl, Phenyl, Naphthyl, Furyl-2,

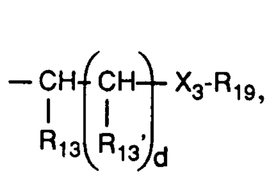

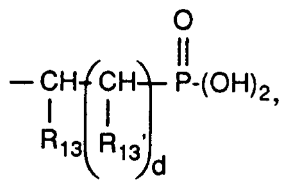

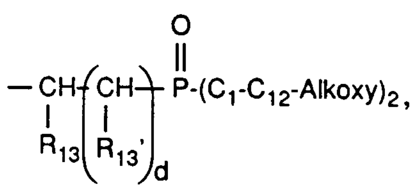

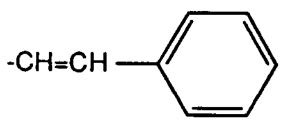

,

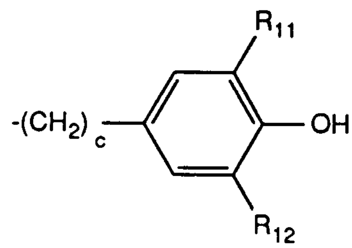

oder

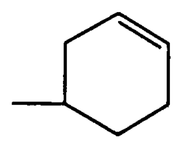

ist,
$R_{18}$ Wasserstoff, $C_1$-$C_{20}$-Alkyl oder $C_2$-$C_{20}$-Alkenyl darstellt,
$R_{19}$ $C_1$-$C_{20}$-Alkyl, $C_5$-$C_{12}$-Cycloalkyl, Phenyl oder -$CH_2$-COO-$C_{12}$-$C_{20}$-Alkyl ist,
X und a die oben angegebene Bedeutung haben,
$X_1$ -O-, -S-, -NH- oder -$NR_{14}$-,
$X_2$ die direkte Bindung, -$CH_2$-, >CH-$CH_3$ oder -S- und
$X_3$ -O-, -S-, -NH- oder -$NR_{19}$- sind,
b 3 bis 5,
c 0 bis 2,
d 0 oder 1,
e 2 bis 10 und
f 2 bis 6 sind;
mit der Massgabe, dass A ungleich -$OC_1$-$C_{12}$-Alkyl, -$NH_2$, -NH-Methyl, -NH-Cyclohexyl, -NH-Phenyl,

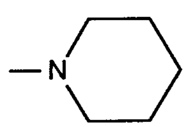

,

-NH-$NH_2$, $C_1$-$C_7$-Alkyl, -$CH_2N(CH_3)_2$, -$CH_2$N(Methyl)-(Phenyl) oder -$C_{a''}H_{2a''}$-CO-O-$R_{10'}$ mit a'' = 2 oder 4 und $R_{10'}$ = Wasserstoff, Methyl oder Ethyl, ist, **wenn einer der Reste R, $R_1$, $R_2$ oder $R_3$ Wasserstoff, Chlor, Methyl oder Methoxy ist, und die übrigen Reste R, $R_1$, $R_2$ oder $R_3$ Wasserstoff darstellen;**
, und daß Verbindungen der Formel Ib ausgeschlossen sind, worin
R, $R_1$, $R_2$, $R_3$ = H und A = $C_1$-$C_{24}$-Alkyl, -O-$C_1$-$C_{24}$-Alkyl, -$NH_2$, NH-$C_1$-$C_{20}$-Alkyl oder -N($C_1$-$C_{20}$-Alkyl)$_2$, Piperidino, Morpholino, Pyrrolidino, Dimethylamino und Diethylamino sind, sowie Verbindungen, worin $R_{16}$ = Wasserstoff und f = 2 sind, und daß die Verbindungen der Formeln

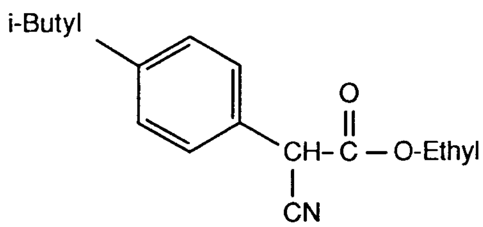

,

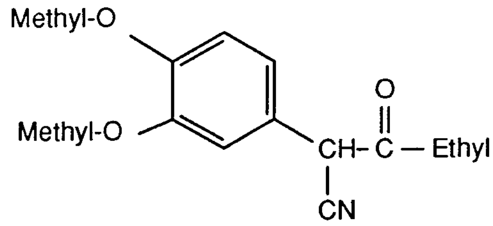

,

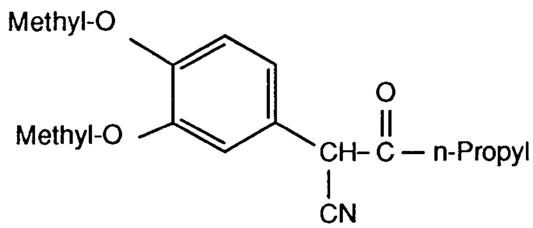

,

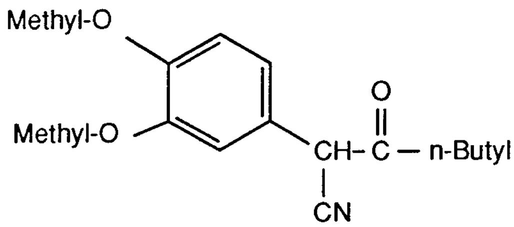

,

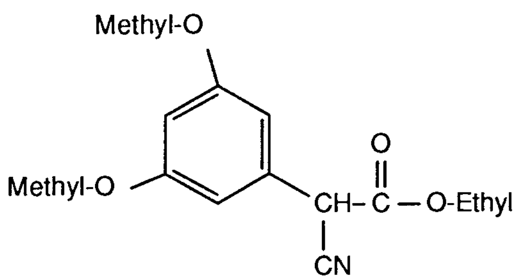

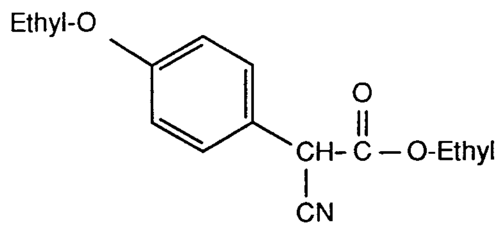

,

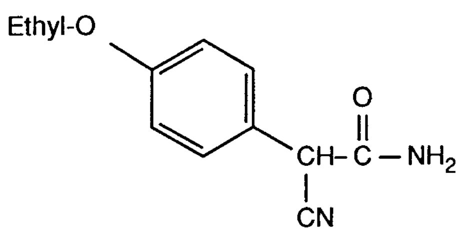

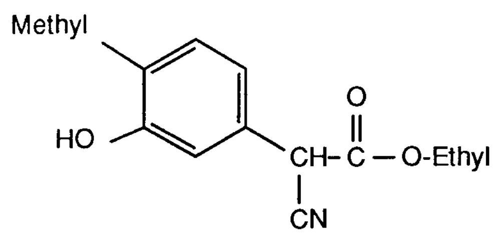

,

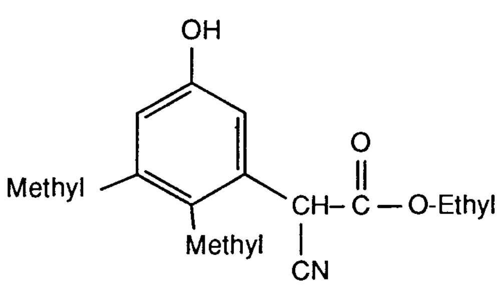

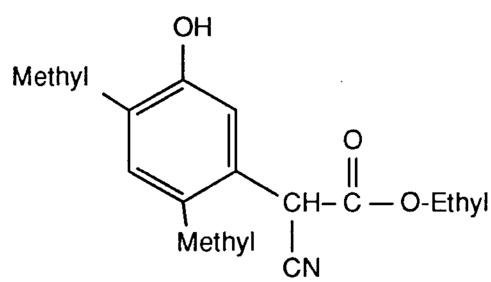

,

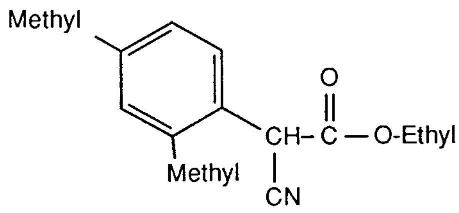
,
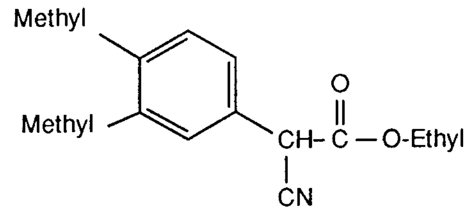
,
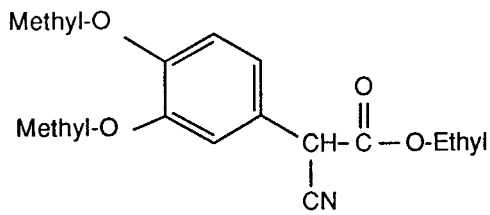
,
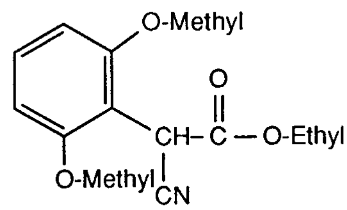
,
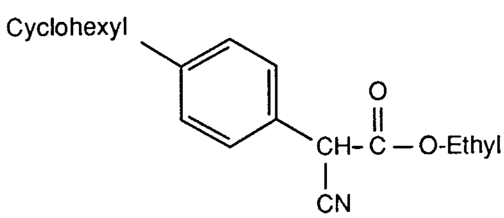
,
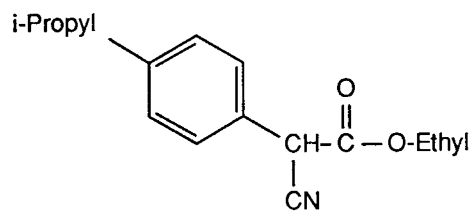
,
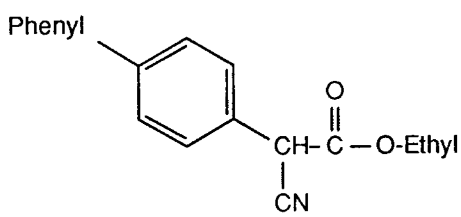
,
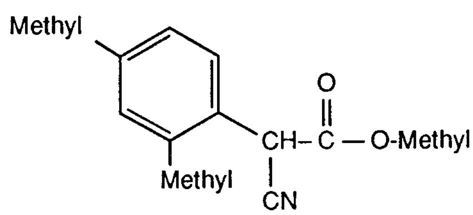
,
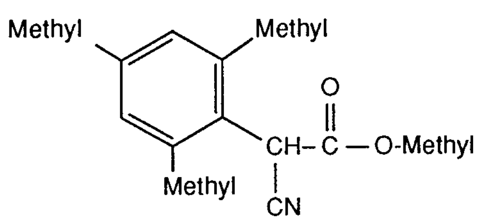
,
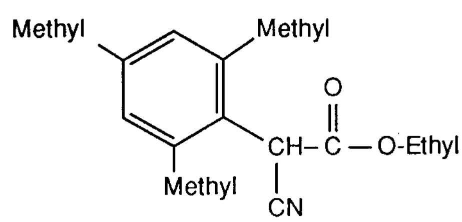
und
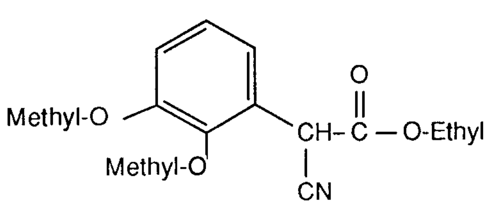

ebenfalls ausgeschlossen sind ;
oder A, bei n = 2, $-X'-C_gH_{2g}-X'-$,

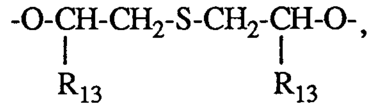

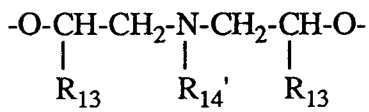, 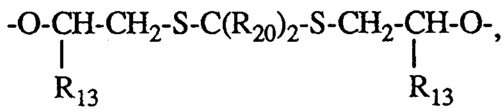

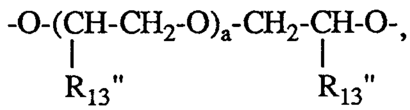

$-OCH_2CH=CHCH_2O-$, $-OCH_2C{\equiv}CCH_2O-$, $-O(CH_2)_h-NH-CO-X_4-OC-NH(CH_2)_h-O-$, $NH(CH_2)_2-X(CH_2)_2-O-$,$-NH-(CH_2CH_2NH)_i-CH_2CH_2NH-$, $NH(CH_2CH_2CH_2NH)_iCH_2CH_2CH_2NH-$,

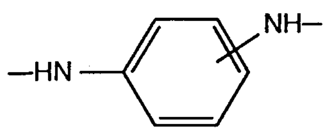, 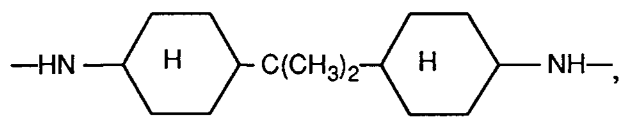

$-NH(CH_2)_h-O-CO-X_4-OC-O-(CH_2)_h-NH-$, $NH-NH-$, $NH-NH-CO-X_4-OC-NH-NH-$, $-NH-N=CH-X_5-CH=N-NH-$, $-C_pH_{2p}-$, $-(CH_2)_m-X_1-(CH_2)_m-$,

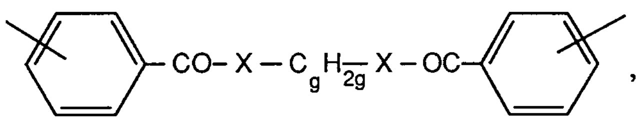,

$-C_pH_{2p}-CO-X-C_gH_{2g}-X-OC-C_pH_{2p}-$, $-(CH_2)_k-O-CO-X_4-CO-O-(CH_2)_k-$, $CH=CH-CO-X-C_gH_{2g}-X-OC-CH=CH-$, $-CH(OH)CH_2-$, $-CH(OH)CH(OH)-$,

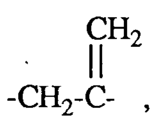,

$-CH=CH-$, $-CH(R_{21})-$ oder

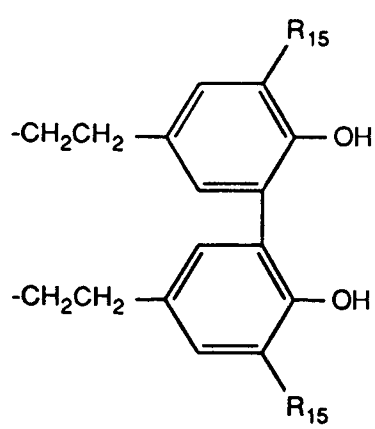

darstellt,
wobei X, $R_{13}$, $R_{15}$, a und f die oben angegebene Bedeutung haben,
die X′ unabhängig voneinander -O- oder -NH- bedeuten,
$R_{13}''$ Wasserstoff oder Methyl ist und bei a = Null auch $C_2$-$C_{18}$-Alkyl oder Phenyl sein kann,
$R_{14}'$ dieselbe Bedeutung wie $R_{14}$ haben kann oder Wasserstoff ist,
die $R_{20}$ unabhängig voneinander Wasserstoff, $C_1$-$C_{20}$-Alkyl, Phenyl oder zusamm en einen 5-12-gliedrigen cycloaliphatischen Ring darstellen,
$R_{21}$ $C_5$-$C_{12}$-Cycloalkyl, Phenyl-$C_1$-$C_4$-alkyl, Phenyl oder

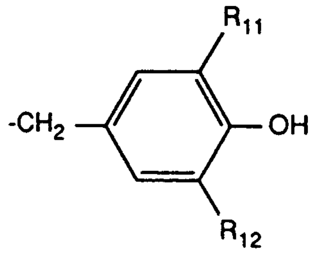

darstellt,
g 2 bis 12, h 2 bis 6, i 1 bis 5, k 3 bis 5, m 1 oder 2 und p 1 bis 10 sind,
$X_4$ -$C_aH_{2a}$-, Phenylen oder -CH=CH- ist und
$X_5$ die direkte Bindung, -$(CH_2)_3$- oder -$C(CH_3)_2$-S-S-$C(CH_3)_2$- darstellt;
oder A, bei n = 3,

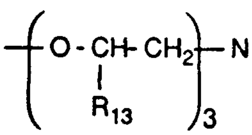 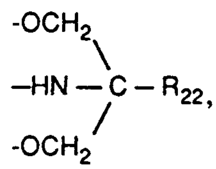 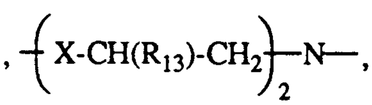

-$(OCH_2)_3$-C-$R_{13}'$, $N(CH_2)_3$- oder

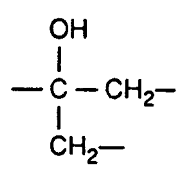

bedeutet, wobei $R_{13}$ und $R_{13}'$ die oben angegebene Bedeutung haben und $R_{22}$ Methyl oder Ethyl ist;
oder A, bei n = 4, $CH(CH_2O)_4$-,

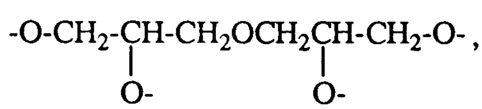

$\{OCH_2)_3$-C-NH-oder -$(CH_2)_2NCH_2CH_2N(CH_2)_2$- ist;
oder A, bei n = 5,

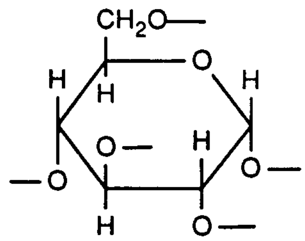

darstellt und
bei n = 6

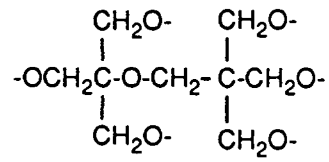

ist.

Bevorzugt sind Verbindungen der Formel Ib, worin R und $R_1$ unabhängig voneinander Wasserstoff, $C_1$-$C_4$-Alkyl, $C_1$-$C_4$-Alkoxy oder Halogen, besonders Chlor, bedeuten und $R_2$ und $R_3$ Wasserstoff sind. Gemäss einer weiteren bevorzugten Ausführungsform werden Verbindungen der Formel Ib eingesetzt, worin R Wasserstoff, $C_1$-$C_4$-Alkyl, besonders Methyl, $C_1$-$C_4$-Alkoxy, besonders Methoxy, oder Chlor ist und $R_1$, $R_2$ und $R_3$ Wasserstoff sind.

Von besonderem Interesse sind Verbindungen der Formel Ib, worin R Wasserstoff, $C_1$-$C_4$-Alkyl, $C_1$-$C_4$-Alkoxy oder Chlor ist, $R_1$, $R_2$ und $R_3$ Wasserstoff sind, n 1 oder 2 ist, A, bein n = 1, $-OR_5$, $-NHR_6$, $-NH-NH-R_8$, $-NH-N=CH-R_{17}$, $C_1$-$C_{17}$-Alkyl, $C_2$-$C_{17}$-Alkenyl,

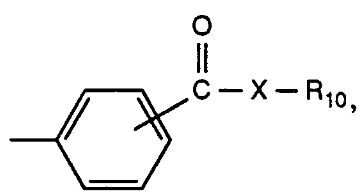

$-(CH_2)_pCO-X-R_{10}$, $-CH_2-X_1-C_1-C_{18}$-Alkyl,

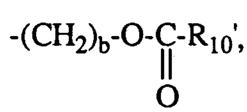

$-(CH_2)_b-OH$,

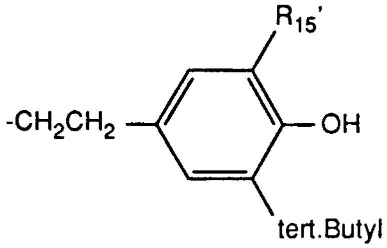 oder 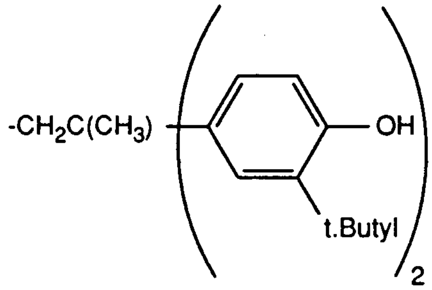 ,

$R_{10}$ Wasserstoff, $C_1$-$C_{18}$-Alkyl, $C_2$-$C_{18}$-Alkenyl, Cyclohexyl, Methylcyclohexyl oder Benzyl und
$R_{10}'$ $C_1$-$C_{17}$-Alkyl, $C_2$-$C_{17}$-Alkenyl oder Phenyl bedeuten,
$R_5$ $C_1$-$C_{18}$-Alkyl, $C_2$-$C_{18}$-Alkenyl, Cyclohexyl, Methylcyclohexyl, Benzyl,

$$-\underset{\underset{R_{13}}{|}}{CH}-CH_2-X_1-R_{10}''$$

oder

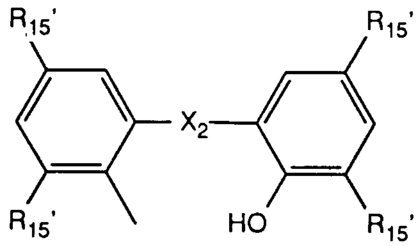

bedeutet, worin $R_{10}''$ Wasserstoff, $C_1$-$C_{18}$-Alkyl oder $C_2$-$C_{18}$-Alkenyl und die $R_{15}'$ unabhängig voneinander Methyl oder tert.Butyl sind,
$R_6$ $C_1C_{18}$-Alkyl, $C_2$-$C_{18}$-Alkenyl, Methylcyclohexyl, Benzyl, $-CH_2CH_2OH$,

$$-CH_2CH_2O-\underset{\underset{O}{\|}}{C}-C_1-C_{18}\text{-Alkyl} \text{ oder } -CH_2CH_2O-\underset{\underset{O}{\|}}{C}-C_2-C_{18}\text{-Alkenyl}$$

ist.
$R_8$ Wasserstoff, $C_1$-$C_{12}$-Alkyl, $-CO-C_1$-$C_{18}$-Alkyl oder $-CO-C_2$-$C_{18}$-Alkenyl darstellt,
$R_{17}$ $C_1$-$C_{11}$-Alkyl, $C_2$-$C_{11}$-Alkenyl, $-(CH_2)_m-S-C_1$-$C_{18}$-Alkyl oder

$$-(CH_2)_m-\overset{\overset{O}{\|}}{P}-(C_1\text{-}C_8\text{-Alkoxy})_2$$

ist;
oder A, bei n = 2, $-O-(CH_2)_g-O-$, $NH-(CH_2)_g-NH-$,

$$-O-\underset{\underset{R_{13}}{|}}{CH}-CH_2-S-CH_2-\underset{\underset{R_{13}}{|}}{CH}-O-,$$

$$-O-\underset{\underset{R_{13}}{|}}{CH}-CH_2-NH-CH_2\underset{\underset{R_{13}}{|}}{CH}-O-, \quad -O-(\underset{\underset{R_{13}}{|}}{CH}-CH_2O)_{a'}-CH_2\underset{\underset{R_{13}}{|}}{CH}-O-$$

mit a' = Null bis 5, oder $-OCH_2-C(CH_3)_2CH_2-O-$, $NHCH_2CH_2-X-CH_2CH_2O-$, $NH(CH_2CH_2NH)_i-CH_2CH_2NH-$, $-NH(CH_2CH_2CH_2NH)_i-CH_2CH_2CH_2-NH-$, $-NH-C(CH_3)_2-CH_2-O-$,

$$-NHCH_2CH_2O\overset{\overset{O}{\|}}{C}(CH_2)_a\overset{\overset{O}{\|}}{C}OCH_2CH_2NH-, \quad -NH-NH-\overset{\overset{O}{\|}}{C}(CH_2)_a\overset{\overset{O}{\|}}{C}-NH-NH-,$$

$-NH-N=CH-X_5-CH=N-NH-$, $-(CH_2)_p-$,

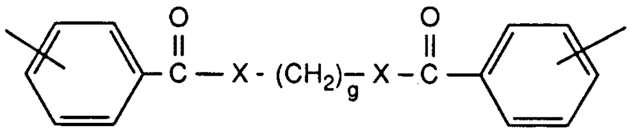,

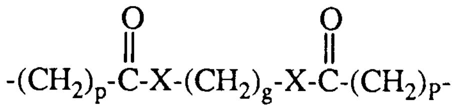, 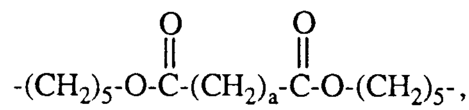,

$-CH(R_{21})-$ mit $R_{21}$ = Benzyl, Phenyl oder

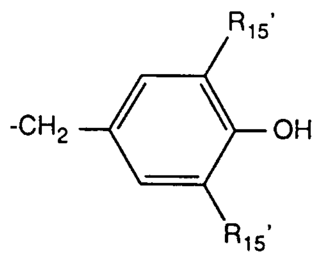 oder A 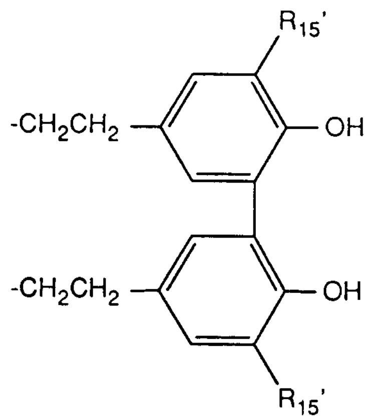

darstellt,
und a, b, g, i, m, p, X, $X_1$, $X_2$, $X_5$ und $R_{13}$ die oben angegebene Bedeutung haben.

Zweckmässig sind auch Verbindungen der Formel Ib, worin R Wasserstoff, Chlor, Methyl oder Methoxy ist und $R_1$, $R_2$ und $R_3$ Wasserstoff sind,
n 1 oder 2 ist,
A, bei n = 1, $-OR_5$, $-NHR_6$, $-NH-NH-R_8$, $-NH-N=CH-R_{17}$, $C_8$-$C_{17}$-Alkyl, $-(CH_2)_p-COOH$,

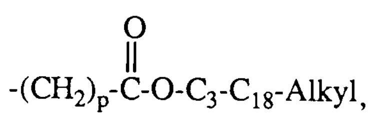,

$-(CH_2)_bOH$, $-(CH_2)_b-OCO-C_1-C_{18}$-Alkyl, $-CH_2-S-C_1-C_{18}$-Alkyl,

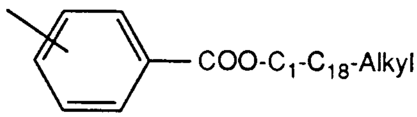 oder 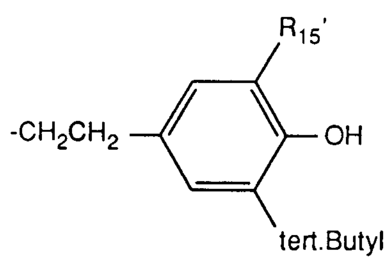

darstellt, wobei $R_5$ $C_{13}$-$C_{18}$-Alkyl, Cyclohexyl, Methylcyclohexyl, Benzyl oder

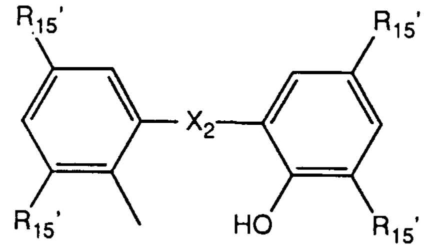

ist,
$R_6$ $C_2$-$C_{18}$-Alkyl, Benzyl, $-(CH_2)_f-OH$ oder $-CH_2CH_2O-CO-C_1$-$C_{18}$-Alkyl bedeutet,
$R_8$ $-CO-C_1$-$C_{18}$-Alkyl oder $-COCH=CH_2$ ist,
die $R_{15}$' unabhängig voneinander Methyl oder tert.Butyl sind,
$R_{17}$ $C_1$-$C_{11}$-Alkyl, $-(CH_2)_m-S-C_1$-$C_{18}$-Alkyl oder

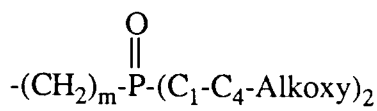

darstellt, oder A, bei n = 2, $-O-(CH_2)_g-O-$, $-NH-(CH_2)_g-NH-$, $-OCH_2CH_2SCH_2CH_2O--O(CH_2CH_2O)_c-CH_2CH_2-O-$, $-OCH_2C(CH_3)_2CH_2O-$, $-NHCH_2CH_2OCH_2CH_2O-$,

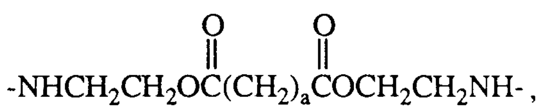, 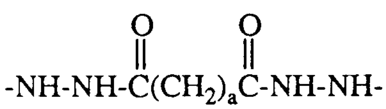,

$-NH-N=CH-C(CH_3)_2-S-S-C(CH_3)_2-CH=N-NH-$ oder $-(CH_2)_p-$ bedeutet,
wobei a, b, c, f, g, m, p und $X_2$ die oben angegebene Bedeutung haben.

Besonders bevorzugt sind Verbindungen der Formel Ib, worin R Chlor, Methyl oder Methoxy und insbesondere Wasserstoff darstellt, $R_1$, $R_2$ und $R_3$ Wasserstoff sind,
n 1 oder 2 ist,
A, bei n = 1, $-OR_5$, $-NHR_6$, $-NH-NH-R_8$, $-NH-N=CH-R_{17}$, $C_8$-$C_{17}$-Alkyl, $-(CH_2)_{p'}-CO-O-C_8$ -$C_{18}$-Alkyl mit p' = 2 bis 6, $-(CH_2)_b-OH$, $-(CH_2)_b-OCO-C_8$-$C_{18}$-Alkyl, $-CH_2-S-C_8$-$C_{18}$ Alkyl,

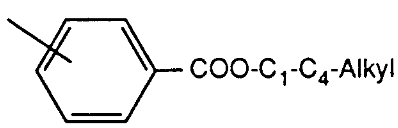 oder 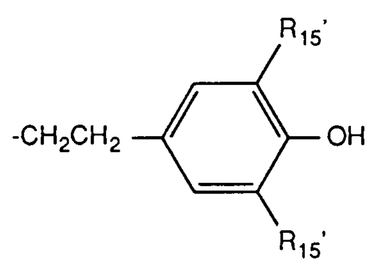

darstellt,

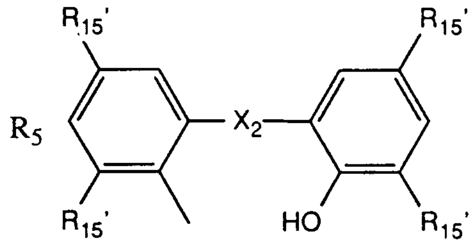

und insbesondere $C_{13}$-$C_{18}$-Akyl ist,
$R_6$ $C_8$-$C_{18}$-Alkyl, -$(CH_2)_{f''}$-OH mit f″ = 2 oder 3 oder -$CH_2CH_2OCO$-$C_8$-$C_{18}$-Alkyl bedeutet,
$R_8$ -CO-$C_8$-$C_{18}$-Alkyl oder -$COCH=CH_2$ ist,
$R_{17}$ $C_8$-$C_{11}$-Alkyl, -$(CH_2)_m$-S-$C_8$-$C_{18}$-Alkyl oder

$$-(CH_2)_m-\overset{\displaystyle O}{\overset{\|}{P}}-(C_1\text{-}C_4\text{-Alkoxy})_2$$

darstellt, oder A, bei n = 2, -$O(CH_2)_gO$-, -$NH(CH_2)_gNH$-, -$OCH_2CH_2SCH_2CH_2O$-, -$O(CH_2CH_2O)_cCH_2CH_2O$-, -$OCH_2C(CH_3)_2CH_2O$-,-$NHCH_2CH_2OCH_2CH_2O$-,-$NHCH_2CH_2OCO$-$(CH_2)_{a''}$-$OCOCH_2CH_2NH$- oder -NH-NHCO-$(CH_2)_{a''}$ -CONH-NH- mit a″ = 2 bis 8, -NH-N=CH-$C(CH_3)_2$-S-S-$C(CH_3)_2$-CH=N-NH- oder -$(CH_2)_{p''}$- mit p″ = 5- 10 bedeutet,
b 3 bis 5, besonders 5 ist, $X_2$ die direkte Bindung oder $>$CH-$CH_3$ ist und $R_{15}'$, c, g und m die oben angegebene Bedeutung haben. Ganz besonders bevorzugt sind Verbindungen der Formel Ib, worin R, $R_1$, $R_2$ und $R_3$ Wasserstoff bedeuten, n 1 oder 2 ist,
A, bei n = 1, $C_8$-$C_{17}$-Alkyl, -$OR_5$, -$NHR_6$ oder -NH-NH-CO-$C_8$-$C_{17}$-Alkyl ist,
$R_5$ $C_{13}$-$C_{18}$-Alkyl ist und $R_6$ $C_8$-$C_{18}$-Alkyl darstellt,
oder A, bei n = 2, -$O(CH_2)_g$-O-, -$NH(CH_2)_gNH$-, $OCH_2C(CH_3)_2CH_2O$-, -NH-NHCO-$CH_2CH_2$-OCNH-NH- oder -$(CH_2)_{p''}$- mit p" 5 bis 10 bedeutet, wobei g die oben angegebene Bedeutung hat.

Ein weiterer Gegenstand der Erfindung ist die Verwendung von Verbindungen der Formel I oder Ib zum Stabilisieren von organischem Material gegen oxidativen, thermischen und/oder aktinischen Abbau. Bevorzugt ist die Verwendung von Verbindungen der Formel I oder Ia als Stabilisatoren in Schmierstoffen, Metallbearbeitungsflüssigkeiten, Hydraulikflüssigkeiten, in natürlichen, halbsynthetischen oder synthetischen Polymeren, bevorzugt halogenfreien Polymeren.

Die Erfindung besteht demzufolge auch in einem Verfahren zum Stabilisieren organischer Materialien, besonders von Schmierstoffen, Metallbearbeitungsflüssigkeiten, Hydraulikflüssigkeiten, natürlichen, halbsynthetischen oder synthetischen Polymeren, indem man dem organischen Material als Stabilisatoren Verbindungen der Formel I oder Ib zusetzt bzw. auf diese aufbringt.

Die Verbindungen der Formel I bzw. Ib können auf an sich bekannte Weise hergestellt werden, beispielsweise wie folgt:

A) Definitionsgemässe Ester können z.B. durch Umsetzung von $\alpha$-Cyanophenylessigsäureestern der Formel II

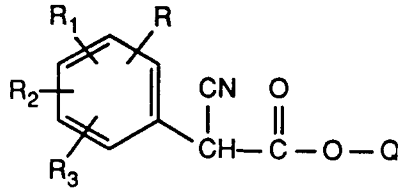

(II)

Q = z.B. $C_1$-$C_6$-Alkyl, besonders Methyl, mit geeigneten Alkoholen HO-$[A]_n$ in Gegenwart katalytischer Mengen einer starken Säure, wie Methan- oder p-Toluolsulfonsäure, erhalten werden. Höhere Ester werden zweckmässig durch säurekatalysierte Umesterung hergestellt.

Ester der Formel I bzw. Ib, worin A den Rest eines Bisphenols darstellt, können z.B. auch dadurch hergestellt werden, dass man ein Benzylcyanid der Formel III

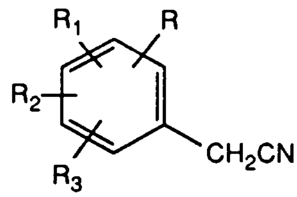

(III)

unter basischen Bedingungen mit einem cyclischen Kohlensäureester des entsprechenden Bisphenols umsetzt.

B) Amide können analog A) durch Umsetzung von α-Cyanophenylessigsäureestern der Formel II mit Aminen $H_2N\text{-}[A]_n$ oder Aminoalkoholen in einem geeigneten Lösungsmittel, wie Ethanol, Toluol oder Xylolen, oder in der Schmelze erhalten werden. Amidester können durch Umsetzung von α-Cyanophenylessigsäureestem der Formel II mit geeigneten Aminoalkoholen und anschliessende Veresterung, z.B. mit einem Säurechlorid in Anwesenheit einer Base, wie Pyridin, hergestellt werden.

C) Hydrazide können ebenfalls analog A) durch Umsetzung von α-Cyanophenylessigsäureestern der Formel II mit Hydrazinen, z.B. solchen der Formel $H_2N\text{-}NH\text{-}[A]_n$, oder durch Umsetzung eines α-Cyanophenylessigsäurehydrazids der Formel IV

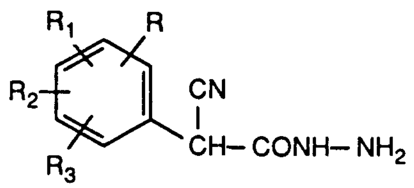

(IV)

mit geeigneten Säurechloriden oder Aldehyden hergestellt werden.

D) α-Ketophenylacetonitrile bzw. α-Cyanophenylessigsäuren (A = -OH) können z.B. durch basisch katalysierte Umsetzung von Benzylcyaniden der Formel III mit Carbonsäureestem $Q\text{-}O\text{-}CO\text{-}[A]_n$ oder Dimethylcarbonat erhalten werden. Als Basen eignen sich insbesondere Alkalimetallalkoholate und -hydride. Die Umsetzung wird zweckmässig in einem organischen Lösungsmittel, z.B. Alkoholen, aliphatischen oder aromatischen Kohlenwasserstoffen, wie n-Pentan, Toluol oder Xylolen, Dimethylsulfoxid oder aliphatischen oder cyclischen Ethern, wie Methylisopropylether und Tetrahydrofuran, vorgenommen. Die bei der Umsetzung von mehrwertigen Carbonsäureestem (n = 2, 3 oder 4) mit nur 1 Aequivalent Benzylcyanid erhaltenen Cyanoketocarbonsäureester können weiter umgesetzt, z.B. mit höheren Alkoholen verestert werden.

Durch Umsetzung von Benzylcyaniden der Formel III mit Lactonen werden α-Cyanoketoalkohole erhalten, die noch weiter verestert werden können, z.B. mit einem Säurechlorid in Gegenwart einer Base, wie Pyridin.

E) Dimere Verbindungen der Formel I

(n = 1, $R_4$ = 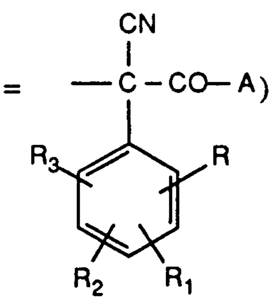

können auf an sich bekannte Weise durch oxidative Kupplung von Verbindungen der Formel V

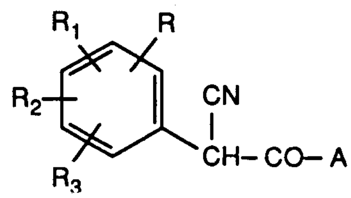

(V)

erhalten werden (vgl. z.B. J.Org.Chem., 36, 3160-3168 (1971) und DE-A 2 033 910). A stellt dabei bevorzugt den Rest eines einwertigen Alkohols, Amins oder Ketons dar.

Verbindungen der Formel I mit funktionellen Gruppen, wie Hydroxylgruppen, primären oder sekundären Aminogruppen oder Carbonamidgruppen, können auf an sich bekannte Weise in andere definitionsgemässe Derivate umgewandelt werden, z.B. durch Veresterung, N-Alkylierung u. ä.

Die als Ausgangsprodukte verwendeten Alkohole, Amine, Aminoalkohole, Hydrazine, Carbonsäureester, Säurechloride, Aldehyde und Lactone sowie die Verbindungen der Formeln II-V sind an sich bekannt oder können auf an sich bekannte Weise hergestellt werden.

Die nachfolgenden Beispiele illustrieren die Erfindung näher, ohne sie auf die Beispiele beschränken zu wollen. Darin sowie in der übrigen Beschreibung und in den Patentansprüchen bedeuten Teile und Prozente Gewichtsteile und Gewichtsprozente, sofern nichts anderes angegeben ist. EA bedeutet Elementaranalyse.

Herstellunpsbeispiele 1-39

A) Ester

1. 39,4 g 1,6-Hexandiol und 133,9 g α-Cyanophenylessigsäuremethylester werden mit 0,32 g Methansulfonsäure versetzt und anschliessend bei 150 °C unter schwachem Vakuum (ca. 200 mbar) während 12 Std. gerührt. Danach wird bei 150 °C Badtemperatur und einem Druck von ca. $2x10^{-3}$ mbar der überschüssige Methylester (20,2 g) abdestilliert. Der noch warme ölige Rückstand wird mit 200 ml Diethylether verdünnt, filtriert und danach in den Kühlschrank gestellt. Der auskristallisierte Feststoff wird abgesaugt, mit 200 ml kaltem Diethylether gewaschen und getrocknet. Man erhält so 111,4 g (82,6 % d. Th.) der in der folgenden Tabelle I angegebenen Verbindung Nr. 3 in Form eines weissen Feststoffes mit Schmelzbereich 60-65 °C.

Auf analoge Weise werden die in der folgenden Tabelle I angegebenen Verbindungen Nr. 1, 2, 4-8, 37, 38, 39 und 40 hergestellt, wobei im Falle der Verbindungen Nr. 37, 38 und 39 anstelle des α-Cyanophenylessigsäuremethylesters das entsprechende p-Methoxyphenyl-, p-Chlorphenyl- bzw. p-Methylphenylderivat eingesetzt wird.

2. 0,65 g Natriumhydrid (55 %) werden in 10 ml Dimethylsulfoxid und 12 ml Tetrahydrofuran suspendiert. Danach werden bei -5 °C 1,9 g Benzylcyanid zugetropft. Anschliessend werden 6,5 g des cyclischen Carbonats des 4,4',6,6'-Tetra-t.butyl-2,2'-bisphenols zugegeben, und das Gemisch wird während 23 Std. bei Raumtemperatur gerührt. Anschliessend werden 600 ml Eiswasser zugegeben, das Gemisch wird mit 3 ml konz. HCl angesäuert, und der Niederschlag wird abgesaugt und aus n-Hexan umkristallisiert. Man erhält so 6,3 g (76 % d. Th.) der Verbindung Nr. 34 in Form eines weissen Feststoffes mit Schmelzpunkt 189- 192 °C.

Auf analoge Weise wird die Verbindung Nr. 35 hergestellt.

B) Amide

1. 17,5 g α-Cyanophenylessigsäuremethylester und 5,8 g 1,6-Diaminohexan werden in 75 ml Xylol 3 Std. am Rückfluss gekocht. Danach wird das Reaktionsgemisch abgekühlt, mit 100 ml Petrolether verdünnt, und der Feststoff wird abgesaugt, mit kaltem Methanol gewaschen und getrocknet. Das erhaltene rohe Diamid (17,0 g = 84,5 % d. Th.) wird aus 80 ml Essigsäure umkristallisiert. Man erhält so 12,0 g (59,6 % d. Th.) der Verbindung Nr. 10 in Form eines weissen Feststoffes vom Smp. 185-190 °C.

Auf analoge Weise werden die Verbindungen Nr. 9, 11 und 12 hergestellt.

2. 6,12 g der Verbindung Nr. 12 werden in 30 ml Pyridin gelöst. Danach werden 9,45 ml Palmitinsäurechlorid zugegeben, und das Gemisch wird 3 Std. bei Raumtemperatur gerührt. Anschliessend werden 200 ml Wasser zugegeben, der Niederschlag wird abgesaugt, mit Wasser gewaschen und in Methylenchlorid gelöst. Die erhaltene Lösung wird über Magnesiumsulfat getrocknet, eingedampft, und der Rückstand wird aus Acetonitril umkristallisiert. Man erhält so 11,6 g (87 % d. Th.) der Verbindung Nr. 13

in Form eines weissen Feststoffes vom Smp. 81 -84 °C. Auf analoge Weise werden die Verbindungen Nr. 14 und 15 hergestellt.

C) Hydrazide

1. 8,76 g α-Cyanophenylessigsäurehydrazid werden in 50 ml Pyridin gelöst. Danach werden 13,75 g Palmitinsäurechlorid zugegeben, und das Gemisch wird 1 Std. bei Raumtemperatur gerührt. Anschliessend werden 400 ml Eiswasser zugegeben, der ausgefallene Niederschlag wird abgesaugt, mit Wasser gewaschen und dann zuerst aus Acetonitril/Toluol und danach aus Toluol umkristallisiert. Es werden 14,2 g (69 % d. Th.) der Verbindung Nr. 16 in Form eines weissen Feststoffes vom Smp. 141-143 °C erhalten.

Auf analoge Weise werden die Verbindungen Nr. 17 und 41 hergestellt.

2. 5,84 g α-Cyanophenylessigsäurehydrazid werden in 50 ml Methylenchlorid suspendiert. Danach werden 3,44 g Bis-2,2′-thioisobutyraldehyd zugegeben, und das Gemisch wird 3 Std. unter Stickstoff bei Raumtemperatur gerührt. Das ausgeschiedene Reaktionswasser wird mit Magnesiumsulfat weggetrocknet, das Gemisch wird filtriert und eingedampft. Der Rückstand wird in 50 ml Toluol gelöst und dann mit 250 ml n-Hexan ausgefällt. Diese Operation wird einmal wiederholt, worauf man 5,9 g (68 % d. Th.) der Verbindung Nr. 20 in Form eines weissen Feststoffes vom Smp. 100-104 °C erhält.

Auf analoge Weise werden die Verbindungen Nr. 18, 19 und 21 hergestellt.

D) Ketone

1. 17,8 g Benzylcyanid und 17,8 g Sebacinsäuredimethylester werden in 10 ml Tetrahydrofuran gelöst und bei -3 °C bis +3 °C zu einer Suspension von 6,7 g Natriumhydrid (55 % in Oel) in 60 ml Tetrahydrofuran und 50 ml Dimethylsulfoxid zugetropft. Das Gemisch wird anschliessend 5 Std. bei Raumtemperatur gerührt und dann mit 1200 ml Eiswasser und 20 ml Salzsäure verdünnt. Der ausgefallene Feststoff wird mit Methylenchlorid extrahiert, und der Extrakt wird dreimal mit Wasser gewaschen, über Magnesiumsulfat getrocknet und eingedampft. Nach dreimaliger Kristallisation aus Toluol/n-Hexan werden 17,9 g (66 % d. Th.) der Verbindung Nr. 23 in Form eines weissen Feststoffes vom Smp. 70-74 °C erhalten.

Auf analoge Weise werden die Verbindungen Nr. 22, 27-32 und 36 hergestellt.

2. 5,25 g Natriumhydrid (55 %) werden in 45 ml Dimethylsulfoxid und 55 ml Tetrahydrofuran suspendiert. Danach werden bei -3 °C gleichzeitig 14,1 g ε-Caprolacton und 14,1 g Benzylcyanid zugetropft. Nach 5,5 Std. Rühren bei Raumtemperatur werden 800 ml Eiswasser zugegeben, und das Gemisch wird mit 20 ml konz. HCl angesäuert. Das ausgeschiedene Oel wird mit Methylenchlorid extrahiert, und der Extrakt wird mit Wasser gewaschen und über Magnesiumsulfat getrocknet. Nach dem Abdampfen des Lösungsmittels wird der Rückstand bei $6x10^{-3}$ mbar destilliert. Zwischen 138-173 °C gehen 13,2 g (47 % d. Th.) der praktisch reinen Verbindung Nr. 24 über, Smp. 97-102 °C. Die Verbindung erstarrt nach dem Abkühlen.

6,95 g des obigen Ketoalkohols (Verbindung Nr. 24) werden in 30 ml Pyridin gelöst und mit 8,5 g Palmitinsäurechlorid versetzt. Nach 22 Std. Rühren bei Raumtemperatur werden 300 ml Eiswasser zugegeben, und das Gemisch wird mit Diethylether extrahiert. Aus dem Rückstand werden nach dem Eindampfen des Diethylethers durch Chromatographie an Kieselgel (Methylenchlorid/Hexan = 4:1) und Kristallisation aus Acetonitril 4,6 g (33 % d. Th.) der Verbindung Nr. 25 erhalten; Smp. 36-41 °C.

3. 10,9 g Natriummethylat werden in 50 ml Methanol gelöst. Danach werden gleichzeitig 17,6 g Benzylcyanid und 36,0 g Dimethylglutarat zugetropft. Nach 5,5 Std. Rückfluss werden 800 ml Eiswasser und 30 ml Essigsäure zugegeben, und das Gemisch wird mit Diethylether extrahiert. Aus dem Rückstand werden nach dem Eindampfen des Diethylethers durch Destillation bei $4x10^{-3}$ mbar/140-147 °C 15,5 g des reinen Ketomethylesters erhalten. 10,8 g dieses Methylesters werden mit 9,8 g n-Octadecanol und 0,2 g p-Toluolsulfonsäure 4,5 Std. bei 150 °C in schwachem Vakuum (ca. 200 mbar) gerührt. Durch Chromatographie an Kieselgel (Methylenchlorid/Hexan = 3:1) und Kristallisation aus Acetonitril werden 8,9 g (46 % d. Th.) der Verbindung Nr. 26 vom Smp. 62-64 °C erhalten.

E) Dimeres

In eine Lösung von 2,3 g Natrium in 50 ml absolutem Methanol werden bei Raumtemperatur 17,5 g α-Cyanophenylessigsäuremethylester zugegeben. Anschliessend werden 12,4 g Iod portionenweise unter ständigem Rühren zugegeben, und das Gemisch wird noch 30 Minuten gerührt. Die erhaltene hellgelbe Lösung wird mit 100 ml Methylenchlorid verdünnt, mit Wasser gewaschen und über Natriumsulfat

getrocknet. Der Rückstand wird nach dem Eindampfen zweimal aus Methanol umkristallisiert. Es werden 6,8 g (39 % d. Th.) der Verbindung Nr. 33 als diastereomeres Gemisch vom Smp. 149-155 °C erhalten.

In der folgenden Tabelle I sind Struktur und physikalische Daten der Verbindungen Nr. 1-41 zusammengefasst.

Die mit (V) gekennzeichneten Verbindungen sind in DE-OS-2 033 910 vorbeschreiben.

Tabelle I:

| Nr. | Verbindung | Smp. °C | EA: C (berechnet/gefunden) | H | N |
|---|---|---|---|---|---|
| 1 (V) | 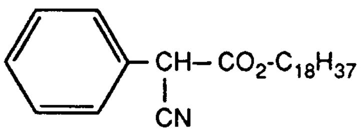 | 46-48 | 78,40<br>78,30 | 10,48<br>10,47 | 3,39<br>3,26 |
| 2 | 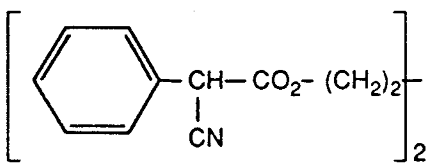 | 81-83 | 70,20<br>70,09 | 5,36<br>5,46 | 7,44<br>7,64 |
| 3 | 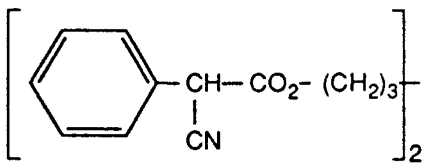 | 60-65 | 71,27<br>71,31 | 5,98<br>5,94 | 6,93<br>7,00 |
| 4 | 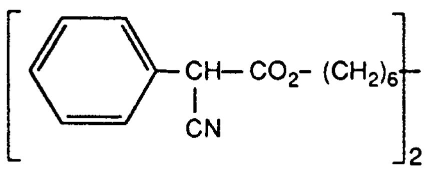 | 39-41 | 73,74<br>73,78 | 7,43<br>7,57 | 5,73<br>5,70 |
| 5 | 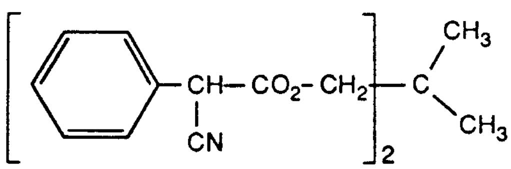 | Oel | 70,75<br>70,45 | 5,68<br>5,70 | 7,18<br>7,08 |
| 6 | 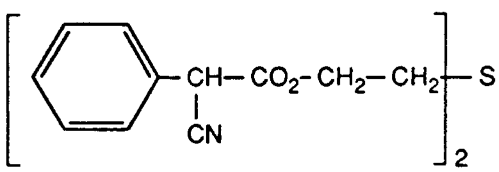 | 54-58 | 64,69<br>64,44 | 4,94<br>4,98 | 6,86<br>6,72 |
| 7 | 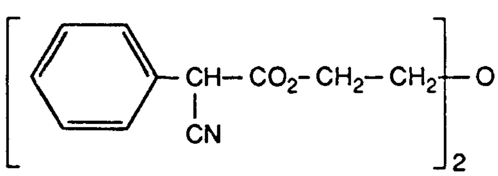 | Oel | 67,34<br>67,11 | 5,14<br>5,19 | 7,14<br>7,07 |
| 8 | 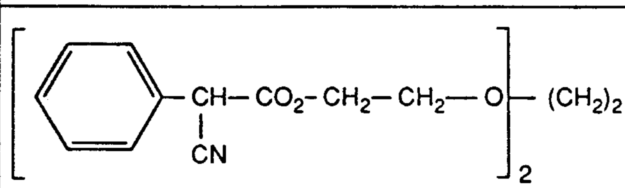 | Oel | 66,05<br>66,04 | 5,54<br>5,66 | 6,42<br>6,24 |

Tabelle I (Fortsetzung)

| Nr. | Verbindung | Smp. °C | EA: C H N (berechnet/gefunden) |
|---|---|---|---|
| 9 (V) | 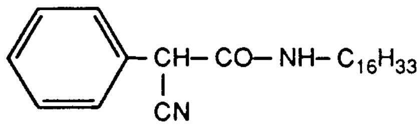 | 87-89 | 78,07 10,48 7,28<br>77,82 10,44 7,26 |
| 10 | 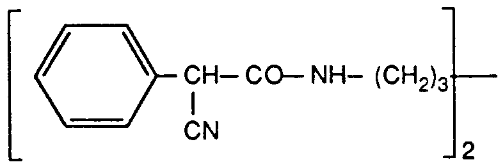 | 185-190 | 71,62 6,51 13,92<br>71,56 6,60 13,97 |
| 11 | 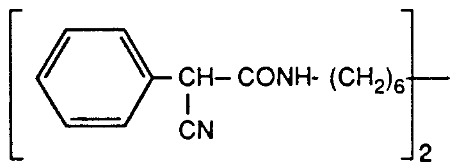 | 132-134 | 74,04 7,87 11,51<br>73,84 8,03 11,56 |
| 12 | 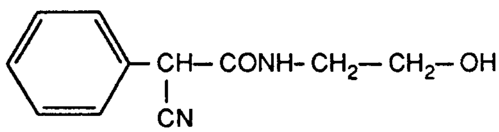 | 108-110 | 64,69 5,92 13,72<br>64,75 5,95 13,78 |
| 13 | 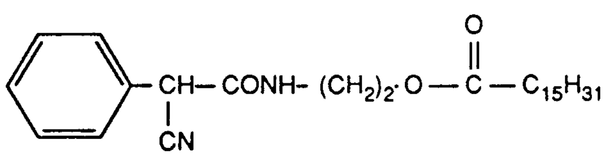 | 81-84 | 73,26 9,56 6,33<br>73,20 9,59 6,09 |
| 14 | 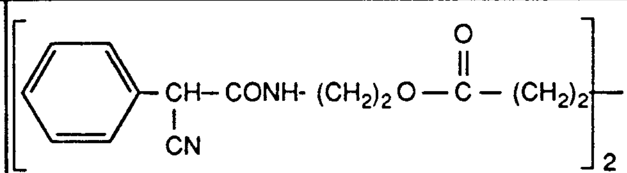 | 143-148 | 64,85 5,83 10,80<br>64,70 5,87 11,27 |
| 15 | 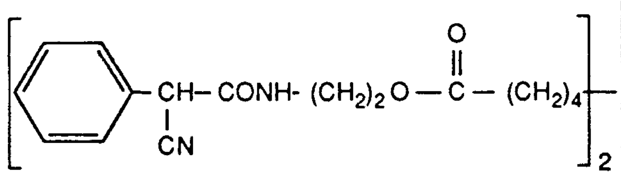 | Oel | 66,88 6,67 9,75<br>66,16 6,71 9,58 |
| 16 | 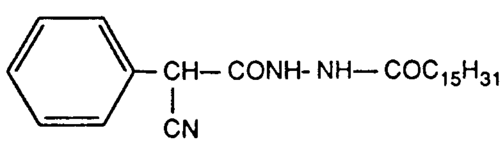 | 141-143 | 72,60 9,50 10,16<br>72,63 9,52 10,01 |

Tabelle I (Fortsetzung)

| Nr. | Verbindung | Smp. °C | EA: C H N (berechnet/gefunden) |
|---|---|---|---|
| 17 | 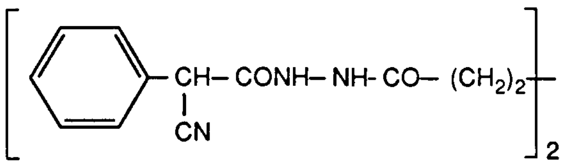 | 228-230 Zersetzung | 62,60 5,25 18,25<br>62,24 5,35 18,09 |
| 18 | 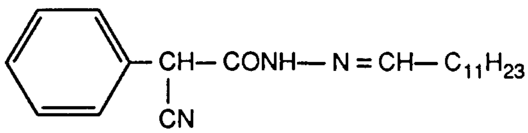 | 100-103 | 73,86 9,15 12,30<br>73,94 9,18 12,50 |
| 19 | 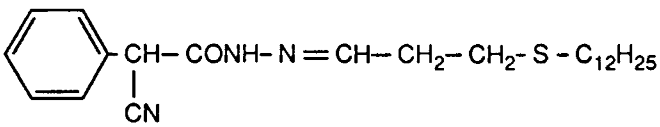 | 83-85 | 69,35 8,97 10,11<br>69,33 8,97 10,14 |
| 20 | 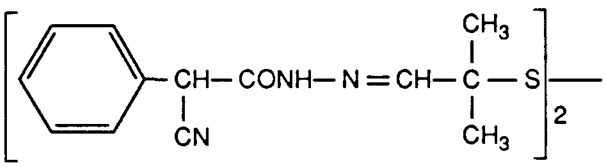 | 100-104 | 59,98 5,42 16,14<br>60,01 5,57 16,06 |
| 21 | 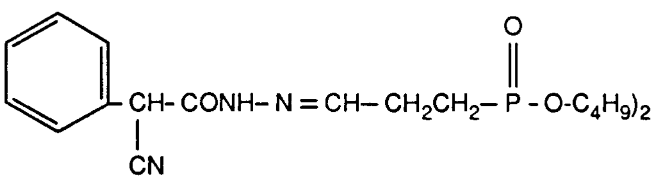 | Oel | 58,96 7,42 10,31<br>58,72 7,63 9,83 |
| 22 | 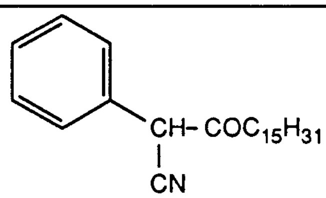 | 69-72 | 81,07 10,49 3,94<br>80,90 10,56 3,83 |
| 23 | 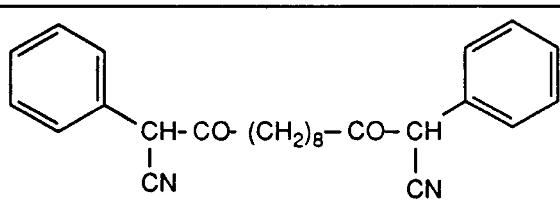 | 70-74 | 77,97 7,05 6,99<br>77,76 7,17 6,94 |
| 24 | 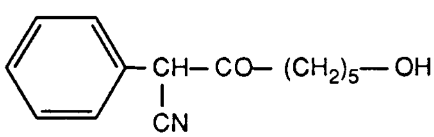 | 97-102 | 72,70 7,41 6,06<br>72,71 7,23 6,19 |

Tabelle I (Fortsetzung)

| Nr. | Verbindung | Smp. °C | EA: C (berechnet/gefunden) | H | N |
|---|---|---|---|---|---|
| 25 | 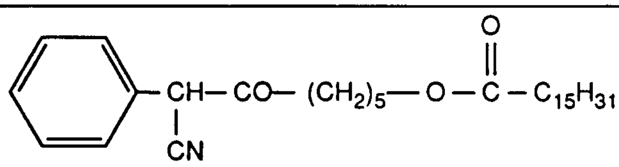 | 36-41 | 76,71<br>76,67 | 10,09<br>10,00 | 2,98<br>2,99 |
| 26 | 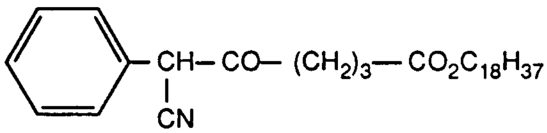 | 62-64 | 76,97<br>77,00 | 10,21<br>10,23 | 2,90<br>3,16 |
| 27 | 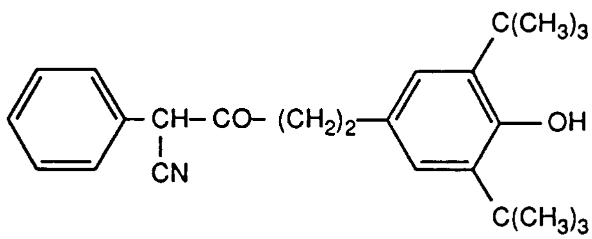 | 96-97 | 79,54<br>79,48 | 8,28<br>8,28 | 3,71<br>3,65 |
| 28 | 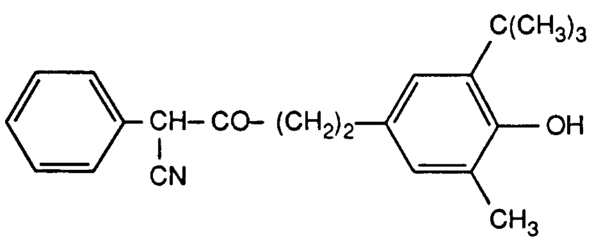 | 105-107 | 78,77<br>78,80 | 7,51<br>7,52 | 4,18<br>4,27 |
| 29 | 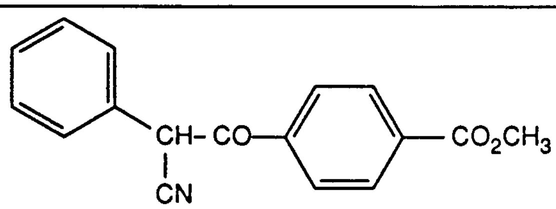 | 98-100 | 73,11<br>72,96 | 4,69<br>4,74 | 5,02<br>4,94 |
| 30 | 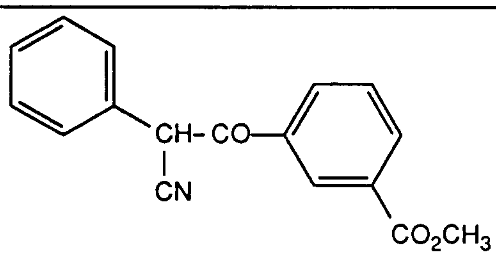 | 90-92 | 73,11<br>72,86 | 4,69<br>4,76 | 5,02<br>5,04 |

Tabelle I (Fortsetzung)

| Nr. | Verbindung | Smp. °C | EA: C (berechnet/gefunden) | H | N |
|---|---|---|---|---|---|
| 31 | 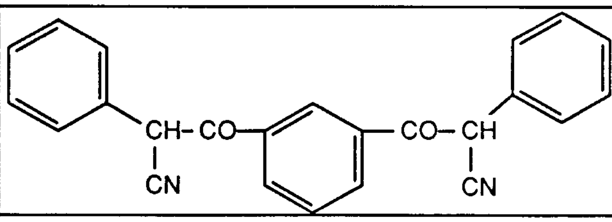 | 105-118 | 79,11<br>78,71 | 4,43<br>4,75 | 7,69<br>7,42 |
| 32 | 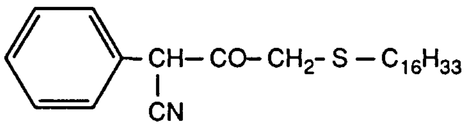 | 59-62 | 75,13<br>75,03 | 9,94<br>9,48 | 3,37<br>3,59 |
| 33 | 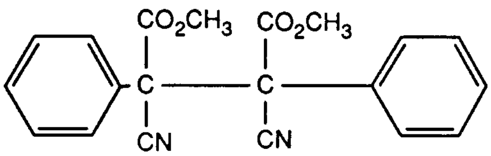 | 149-155 | 68,96<br>69,04 | 4,63<br>4,68 | 8,04<br>8,06 |
| 34 | 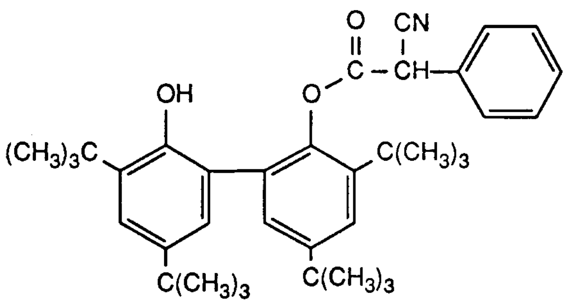 | 189-192 | 80,25<br>80,24 | 8,55<br>8,47 | 2,53<br>2,59 |
| 35 | 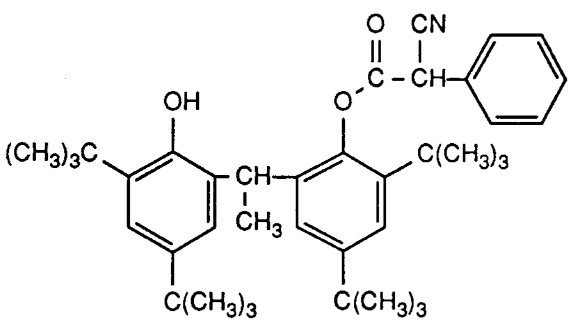 | 158-162 | 80,51<br>80,50 | 8,84<br>8,92 | 2,41<br>2,31 |
| 36 | 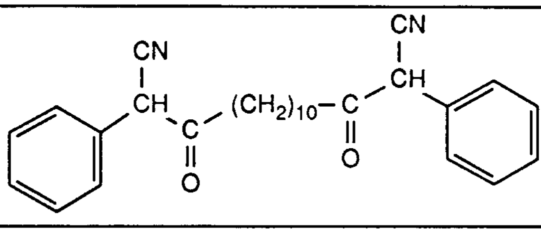 | 55-78 | 78,47<br>78,44 | 7,53<br>7,66 | 6,54<br>6,28 |

Tabelle I (Fortsetzung)

| Nr. | Verbindung | Smp. °C | EA: C (berechnet/gefunden) | H | N |
|---|---|---|---|---|---|
| 37 | 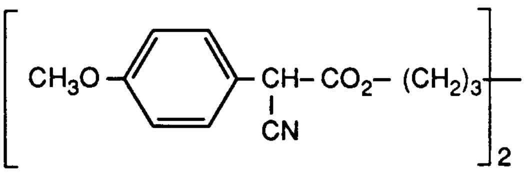 | Oel | 67,22<br>67,10 | 6,08<br>6,13 | 6,03<br>6,39 |
| 38 | 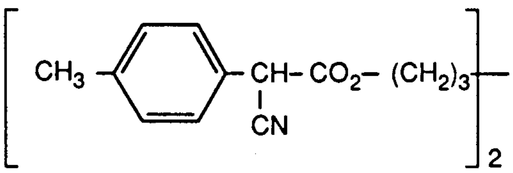 | 56-63 | 72,20<br>72,06 | 6,53<br>6,56 | 6,48<br>6,22 |
| 39 | 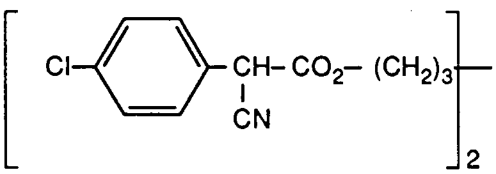 | Oel | 60,90<br>60,96 | 4,68<br>4,69 | 5,92<br>5,94 |
| 40 | 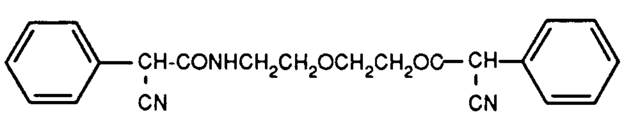 | Oel | 67,51<br>67,36 | 5,41<br>5,49 | 10,74<br>10,68 |
| 41 | 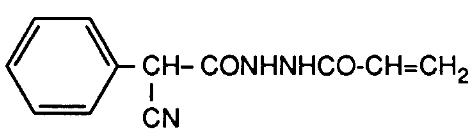 | 179-183 | 62,87<br>62,50 | 4,84<br>4,87 | 18,33<br>18,24 |

Verwendungsbeispiele: Stabilisierung von Polypropylen.

1,3 kg Polypropylen-Pulver (Schmelzindex 3,2 g/10 min., gemessen bei 230 °C mit 2, 16 kg) werden mit 0,05 % Calciumstearat, 0,05 % IRGANOX® 1010 und 0,05 % der in Tabelle II angegebenen Verarbeitungsstabilisatoren der Formel I gemischt. Das Gemisch wird in einem Extruder mit einem Zylinderdurchmesser von 20 mm und einer Länge von 20 d = 400 mm mit 100 Umdrehungen/min. extrudiert, wobei die 3 Heizzonen auf 260 °C, 270 °C und 280 °C eingestellt werden. Das Extrudat wird zur Kühlung durch ein Wasserbad gezogen und anschliessend granuliert. Nach diesen drei Extrusionen wird der Schmelzindex gemessen (bei 230 °C mit 2, 16 kg). Auf analoge Weise wird Polypropylen ohne Verarbeitungsstabilisator extrudiert. Die Ergebnisse sind in der folgenden Tabelle II zusammengefasst.

Die Zunahme des Schmelzindex ist ein Mass für den Kettenabbau des Polymeren und somit der Stabilisatorwirkung.

Tabelle II:

| Verbindung Nr. | Schmelzindex nach 3 Extrusionen g/10 min. bei 230°C mit 2,16 kg |
|---|---|
| ohne Stabilisator | 17,8 |
| 1 | 8,8 |
| 2 | 6,4 |
| 3 | 6,4 |
| 4 | 6,4 |
| 5 | 6,5 |
| 6 | 6,8 |
| 7 | 7,2 |
| 8 | 6,9 |
| 9 | 8,5 |
| 10 | 6,4 |
| 11 | 6,9 |
| 13 | 8,7 |
| 14 | 7,7 |
| 15 | 8,3 |
| 16 | 6,9 |
| 17 | 5,8 |
| 18 | 7,0 |
| 19 | 8,4 |
| 20 | 4,9 |
| 21 | 8,8 |
| 22 | 7,7 |
| 23 | 6,6 |
| 25 | 8,3 |
| 26 | 7,9 |
| 27 | 7,4 |
| 28 | 6,6 |
| 29 | 7,3 |
| 30 | 7,2 |
| 31 | 6,6 |
| 32 | 7,2 |

Tabelle II (Fortsetzung)

| Verbindung Nr. | Schmelzindex nach 3 Extrusionen g/10 min. bei 230°C mit 2,16 kg |
|---|---|
| 33 | 7,1 |
| 36 | 8,4 |
| 37 | 7,4 |
| 38 | 8,3 |
| 39 | 8,8 |
| 40 | 7,3 |

## Patentansprüche

1. Zusammensetzung enthaltend thermischem, oxidativem und/oder aktinischem Abbau unterliegende Schmierstoffe, Metallbearbeitungsflüssigkeiten, Hydraulikflüssigkeiten oder natürliche halbsynthetische oder synthetische Polymere und mindestens eine Verbindung der Formel I

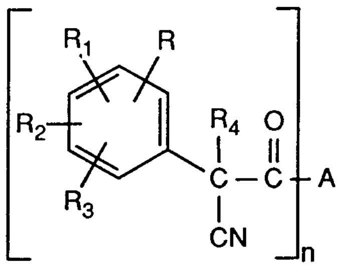

(I),

worin R Wasserstoff, -OH, Halogen, $C_1$-$C_{20}$-Alkyl, $C_2$-$C_{20}$-Alkenyl, $C_5$-$C_{12}$-Cycloalkyl, $C_1$-$C_4$-Alkyl-$C_5$-$C_{12}$-cycloalkyl, Phenyl, Naphthyl, $C_1$-$C_{20}$-Alkoxy, Phenyloxy, Naphthyloxy, -S-$C_1$-$C_{20}$-Alkyl, -S-Phenyl, -S-Naphthyl, -O-CO-$C_1$-$C_{20}$-Alkyl, -O-CO-Phenyl, -O-CO-Naphthyl, -COOH, COO-$C_1$-$C_{20}$-Alkyl, -COO-$C_2$-$C_{20}$-Alkenyl, -COO-Phenyl, -COO-Naphthyl, -CONR'R'', -CO-$C_1$-$C_{20}$-Alkyl, -CO-Phenyl, -CO-Naphthyl, -NHCO-$C_1$-$C_{20}$-Alkyl, -NHCO-$C_2$-$C_{20}$-Alkenyl, -NHCO-Phenyl oder -NHCO-Naphthyl bedeutet,
$R_1$ dieselbe Bedeutung wie R haben kann,
$R_2$ Wasserstoff, Halogen oder $C_1$-$C_4$-Alkyl ist und
$R_3$ Wasserstoff oder Halogen bedeutet,
oder zwei in ortho-Stellung zueinander gebundene Reste R, $R_1$, $R_2$ oder $R_3$ zusammen Tetramethylen oder -CH=CH-CH=CH- bilden, wobei die restlichen zwei von R, $R_1$, $R_2$ und $R_3$ Wasserstoff sind,
oder $R_1$ bis $R_3$ Wasserstoff sind und R bei n = 1 eine Gruppierung

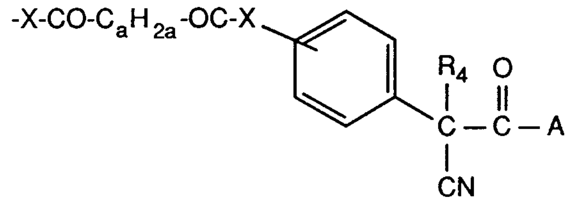

oder

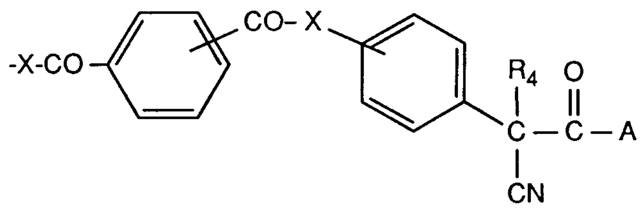

darstellt,
R' und R'' unabhängig voneinander Wasserstoff, $C_1$-$C_{20}$-Alkyl, $C_2$-$C_{20}$-Alkenyl, Phenyl oder Naphthyl bedeuten oder zusammen mit dem Bindungs-N-Atom einen 5- oder 6-gliedrigen heterocyclischen Ring bilden,
a Null bis 10 ist, n 1 bis 6 ist,
X -O- oder -NH- bedeutet,
$R_4$ Wasserstoff oder bei n = 1 eine Gruppierung

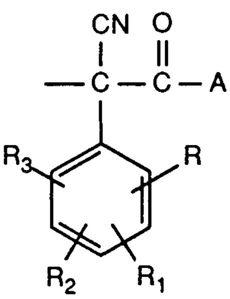

darstellt,
A bei n = 1,-OH, -$OR_5$, -$NR_6R_7$, -NH-$NR_8NR_9$, $C_1$-$C_{30}$-Alkyl, $C_2$-$C_{30}$-Alkenyl, $C_5$-$C_{12}$-Cycloalkyl, $C_1$-$C_4$-Alkyl-$C_5$-$C_{12}$-cycloalkyl,

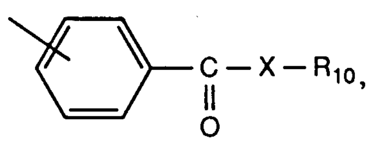

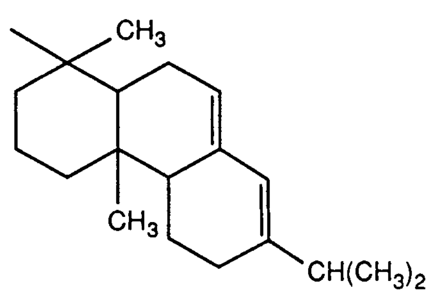

oder das entsprechende Dihydro- oder Tetrahydroderivat, $-C_aH_{2a}-CO-X-R_{10}$, $-C_bH_{2b}-OCO-R_{10}$, $-C_bH_{2b}-OH$,

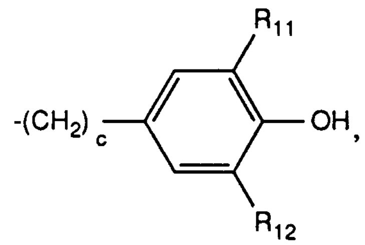

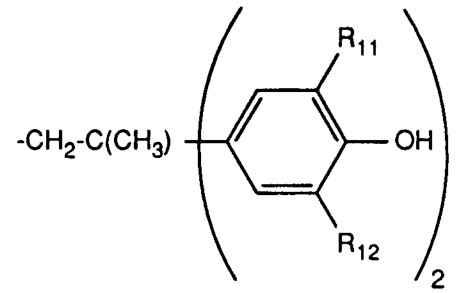
,
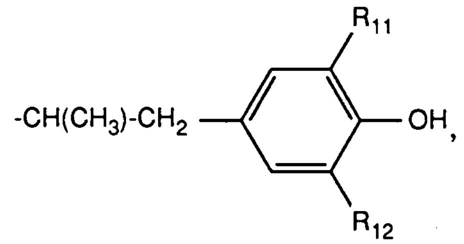

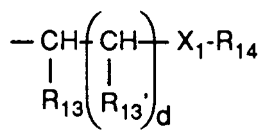
oder
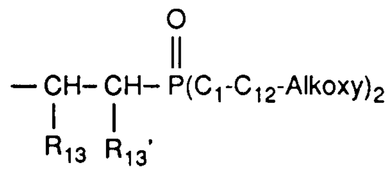

bedeutet, wobei

$R_5$ $C_1$-$C_{30}$-Alkyl, $C_2$-$C_{30}$-Alkenyl, $C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl, Biphenyl, der Rest eines Terpenalkohols oder ein Rest der Formeln

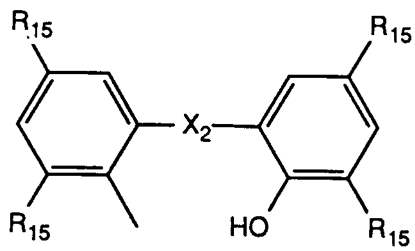
,
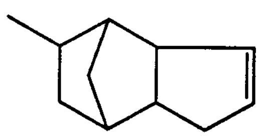
,
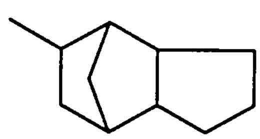
,

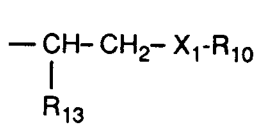
oder
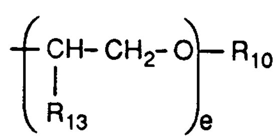

ist,

$R_6$ und $R_7$ unabhängig voneinander Wasserstoff, $C_1$-$C_{20}$-Alkyl, $C_2$-$C_{20}$-Alkenyl, $C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl,

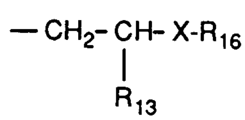

oder $-CH_2C(CH_2-O-R_{16})_3$ sind oder $R_6$ bei $R_7$ = H auch $-(CH_2)_f-X-R_{16}$ sein kann oder $R_6$ und $R_7$ zusammen mit dem N-Bindungsatom einen 5- oder 6-gliedrigen heterocyclischen Ring bilden,

$R_8$ und $R_9$ unabhängig voneinander Wasserstoff, $C_1$-$C_{20}$-Alkyl, Phenyl, $-CO-C_1-C_{20}$-Alkyl, $-CO-C_2-C_{18}$-

Alkenyl, -CO-Phenyl oder -CO-Naphthyl sind oder zusammen $=CH-R_{17}$ darstellen,
$R_{10}$ Wasserstoff, $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl, Naphthyl, -CO-$C_1$-$C_{20}$-Alkyl, -CO-Phenyl, -CO-Naphthyl oder

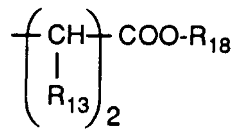

ist,
$R_{11}$ und $R_{12}$ unabhängig voneinander Wasserstoff, $C_1$-$C_4$-Alkyl, Cyclohexyl oder Phenyl sind,
$R_{13}$ Wasserstoff oder Methyl und $R_{13}$' Wasserstoff, Methyl oder Phenyl sind,
$R_{14}$ $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl oder Naphthyl darstellt,
die $R_{15}$ unabhängig voneinander $C_1$-$C_4$-Alkyl sind,
$R_{16}$ Wasserstoff, -CO-$C_1$-$C_{20}$-Alkyl, -CO-$C_2$-$C_{20}$-Alkenyl, -CO-Phenyl oder -CO-Naphthyl darstellt,
$R_{17}$ $C_1$-$C_{20}$-Alkyl, $C_2$-$C_{20}$-Alkenyl, $C_5$-$C_{12}$-Cycloalkyl, Phenyl, Naphthyl, Furyl-2,

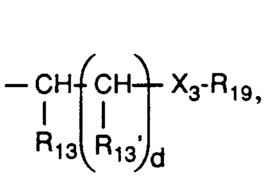 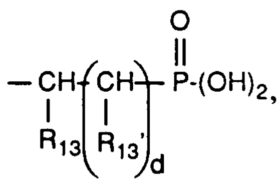 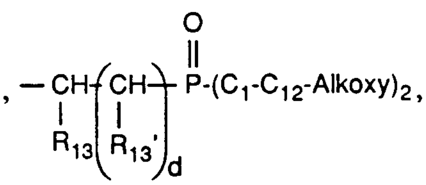

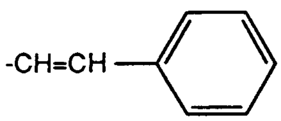, 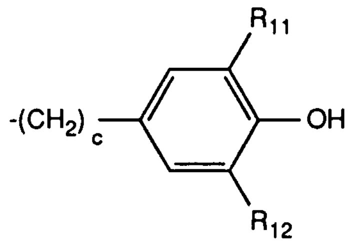 oder 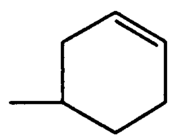

ist,
$R_{18}$ Wasserstoff, $C_1$-$C_{20}$-Alkyl oder $C_2$-$C_{20}$-Alkenyl darstellt,
$R_{19}$ $C_1$-$C_{20}$-Alkyl, $C_5$-$C_{12}$-Cycloalkyl, Phenyl oder $-CH_2-COO-C_{12}-C_{20}$-Alkyl ist,
X und a die oben angegebene Bedeutung haben,
$X_1$ -O-, -S-, -NH- oder $-NR_{14}-$,
$X_2$ die direkte Bindung, $-CH_2-$, $>CH-CH_3$ oder -S- und
$X_3$ -O-, -S-, -NH- oder $-NR_{19}-$ sind,
b 3 bis 5,
c 0 bis 2,
d 0 oder 1,
e 2 bis 10 und
f 2 bis 6 sind;
oder A, bei n = 2, $-X'-C_gH_{2g}-X'-$,

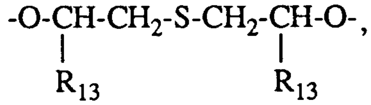

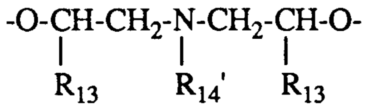, 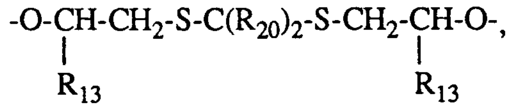

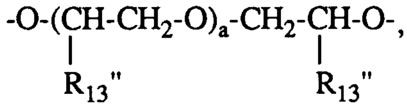

$-OCH_2CH=CHCH_2O-$, $-OCH_2C\equiv CCH_2O-$, $-O(CH_2)_h-NH-CO-X_4-OC-NH(CH_2)_h-O-$, $-NH(CH_2)_2-X(CH_2)_2-O-$, $-NH(CH_2CH_2NH)_i-CH_2CH_2NH-$, $-NH(CH_2CH_2CH_2NH)_iCH_2CH_2CH_2NH-$-$NH(CH_2)_h-O-CO-X_4-OC-O-(CH_2)_h-NH-$,

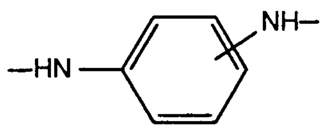, 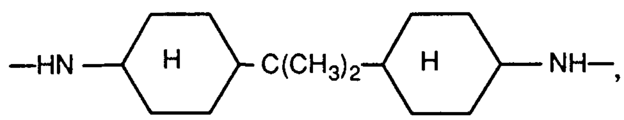

$-NH-NH-$, $-NH-NH-CO-X_4-OC-NH-NH-$, $-NH-N=CH-X_5-CH=N-NH-$, $-C_aH_{2a}-$, $-(CH_2)_m-X_1-(CH_2)_m-$, Phenylen,

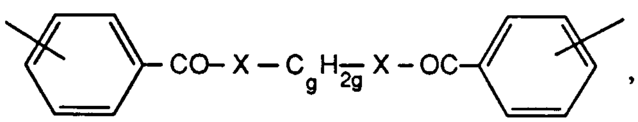,

$-C_pH_{2p}-CO-X-C_gH_{2g}-X-OC-C_pH_{2p}-$, $-(CH_2)_k-O-CO-X_4-CO-O-(CH_2)_k-$, $-CH=CH-CO-X-C_gH_{2g}-X-OC-CH=CH-$, $-CH(OH)CH_2-$, $-CH(OH)CH(OH)-$,

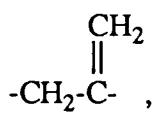,

$-CH=CH-$, $-CH(R_{21})-$ oder

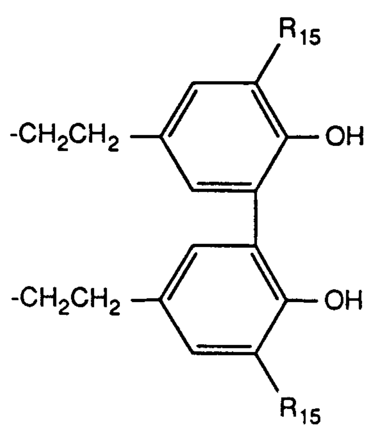

darstellt,
wobei X, $R_{13}$, $R_{15}$, a und f die oben angegebene Bedeutung haben,
die X' unabhängig voneinander -O- oder -NH- bedeuten,
$R_{13}''$ Wasserstoff oder Methyl ist und bei a = Null auch $C_2$-$C_{18}$-Alkyl oder Phenyl sein kann,
$R_{14}'$ dieselbe Bedeutung wie $R_{14}$ haben kann oder Wasserstoff ist,
die $R_{20}$ unabhängig voneinander Wasserstoff, $C_1$-$C_{20}$-Alkyl, Phenyl oder zusammen einen 5-12-gliedrigen cycloaliphatischen Ring darstellen,
$R_{21}$ $C_5$-$C_{12}$-Cycloalkyl, Phenyl-$C_1$-$C_4$-alkyl, Phenyl oder

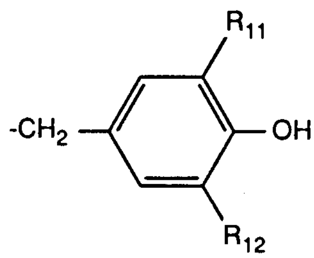

darstellt,
g 2 bis 12, h 2 bis 6, i 1 bis 5, k 3 bis 5, m 1 oder 2 und p 1 bis 10 sind,
$X_4$ $-C_aH_{2a}-$, Phenylen oder -CH=CH- ist und
$X_5$ die direkte Bindung, $-(CH_2)_3-$ oder $-C(CH_3)_2-S-S-C(CH_3)_2-$ darstellt;
oder A, bei n = 3,

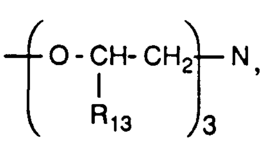, 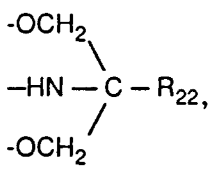, $-(X-CH(R_{13})-CH_2)_2N-$,

$-(OCH_2)_3-C-R_{13}'$, $N(CH_2)_3-$ oder

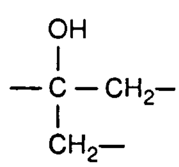

bedeutet, wobei $R_{13}$ und $R_{13}'$ die oben angegebene Bedeutung haben und $R_{22}$ Methyl oder Ethyl ist;
oder A, bei n = 4, $C(CH_2O)_4-$,

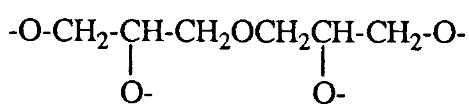,

$-(OCH_2)_3-C-NH-$oder $-(CH_2)_2NCH_2CH_2N(CH_2)_2-$ ist;
oder A, bei n = 5,

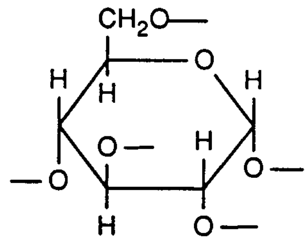

darstellt und
bei n = 6

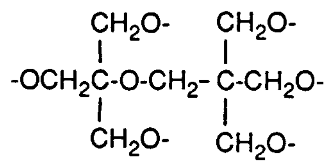

ist.

2. Zusammensetzung nach Anspruch 1, worin R und $R_1$ unabhängig voneinander Wasserstoff, $C_1$-$C_4$-Alkyl, $C_1$-$C_4$-Alkoxy oder Halogen, besonders Chlor, bedeuten und $R_2$ und $R_3$ Wasserstoff sind.

3. Zusammensetzung nach Anspruch 1, worin R Wasserstoff, $C_1$-$C_4$-Alkyl, $C_1$-$C_4$-Alkoxy oder Chlor ist, $R_1$, $R_2$, $R_3$ und $R_4$ Wasserstoff sind, n 1 oder 2 ist,
A, bei n = 1, $-OR_5$, $-NHR_6$, $-NH-NH-R_8$, $-NH-N=CH-R_{17}$, $C_1$-$C_{17}$-Alkyl, $C_2$-$C_{17}$-Alkenyl,

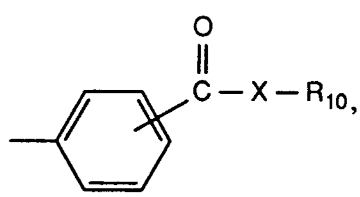

$-(CH_2)_pCO-X-R_{10}$, $-CH_2-X_1-C_1-C_{18}$-Alkyl,

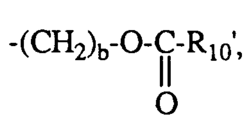

$-(CH_2)_b-OH$,

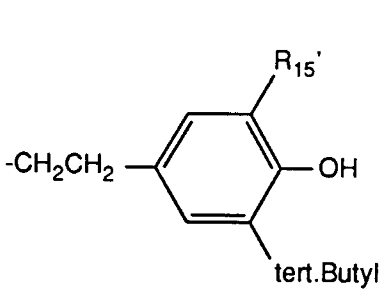 oder 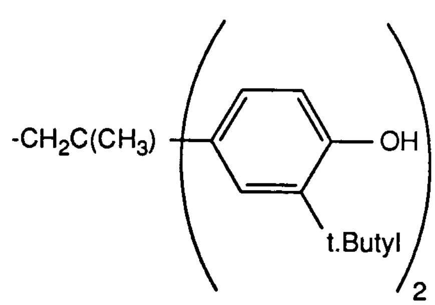 ,

$R_{10}$ Wasserstoff, $C_1$-$C_{18}$-Alkyl, $C_2$-$C_{18}$-Alkenyl, Cyclohexyl, Methylcyclohexyl oder Benzyl und $R_{10}'$ $C_1$-$C_{17}$-Alkyl, $C_2$-$C_{17}$-Alkenyl oder Phenyl bedeuten,
$R_5$ $C_1$-$C_{18}$-Alkyl, $C_2$-$C_{18}$-Alkenyl, Cyclohexyl, Methylcyclohexyl, Benzyl,

$$-\underset{R_{13}}{\underset{|}{CH}}-CH_2-X_1-R_{10}''$$

oder

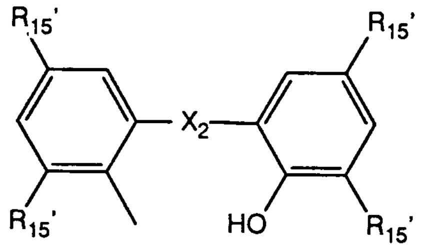

bedeutet, worin $R_{10}''$ Wasserstoff, $C_1$-$C_{18}$-Alkyl oder $C_2$-$C_{18}$-Alkenyl und die $R_{15}'$ unabhängig voneinander Methyl oder tert.Butyl sind,
$R_6$ $C_1$-$C_{18}$-Alkyl, $C_2$-$C_{18}$-Alkenyl, Cyclohexyl, Methylcyclohexyl, Benzyl, $-CH_2CH_2OH$,

$$-CH_2CH_2O-\underset{O}{\underset{\|}{C}}-C_1\text{-}C_{18}\text{-Alkyl} \text{ oder } -CH_2CH_2O-\underset{O}{\underset{\|}{C}}-C_2\text{-}C_{18}\text{-Alkenyl}$$

ist,
$R_8$ Wasserstoff, $C_1$-$C_{12}$-Alkyl, $-CO-C_1$-$C_{18}$-Alkyl oder $-CO-C_2$-$C_{18}$-Alkenyl darstellt, $R_{17}$ $C_1$-$C_{11}$-Alkyl, $C_2$-$C_{11}$-Alkenyl, $-(CH_2)_m-S-C_1$-$C_{18}$-Alkyl oder

$$-(CH_2)_m-\overset{O}{\overset{\|}{P}}-(C_1\text{-}C_8\text{-Alkoxy})_2$$

ist;
oder A, bei n = 2, $-O-(CH_2)_g-O-$, $-NH-(CH_2)_g-NH-$,

$$-O-\underset{R_{13}}{\underset{|}{CH}}-CH_2-S-CH_2-\underset{R_{13}}{\underset{|}{CH}}-O-,$$

$$-O-\underset{R_{13}}{\underset{|}{CH}}-CH_2-NH-CH_2\underset{R_{13}}{\underset{|}{CH}}-O-, \quad -O-(\underset{R_{13}}{\underset{|}{CH}}-CH_2O)_{a'}-CH_2\underset{R_{13}}{\underset{|}{CH}}-O-$$

mit a' = Null bis 5, oder $-OCH_2-C(CH_3)_2CH_2-O-$, $-NHCH_2CH_2-X-CH_2CH_2O-$, $-NH(CH_2CH_2NH)_i-CH_2CH_2NH-$, $-NH(CH_2CH_2CH_2NH)_i-CH_2CH_2CH_2-NH-$, $-NH-C(CH_3)_2-CH_2O-$,

$$-NHCH_2CH_2O\overset{O}{\overset{\|}{C}}(CH_2)_a\overset{O}{\overset{\|}{C}}OCH_2CH_2NH-, \quad -NH-NH-\overset{O}{\overset{\|}{C}}(CH_2)_a\overset{O}{\overset{\|}{C}}-NH-NH-,$$

$-NH-N=CH-X_5-CH=N-NH-$, $-(CH_2)_p-$, Phenylen,

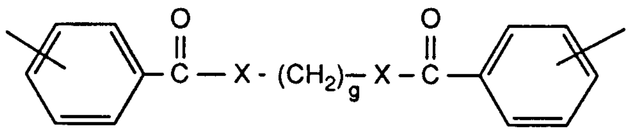, 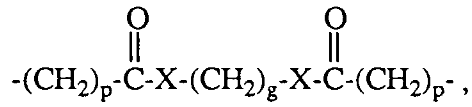,

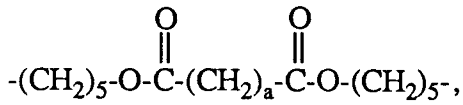,

$-CH(R_{21})-$ mit $R_{21}$ = Benzyl, Phenyl oder

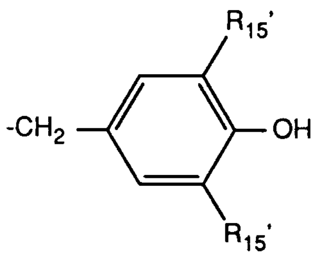 oder A 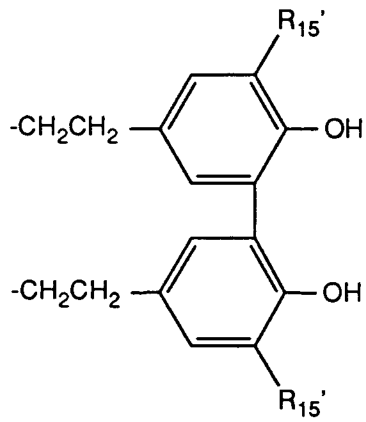

darstellt,
und a, b, g, i, m, p, X, $X_1$, $X_2$, $X_5$ und $R_{13}$ die in Anspruch 1 angegebene angegebene Bedeutung haben.

**4.** Zusammensetzung nach Anspruch 1, worin R Wasserstoff, Chlor, Methyl oder Methoxy ist und $R_1$, $R_2$, $R_3$ und $R_4$ Wasserstoff sind,
n 1 oder 2 ist,
A, bei n = 1, $-OR_5$, $-NHR_6$, $-NH-NH-R_8$, $-NH-N=CH-R_{17}$, $C_1$-$C_{17}$-Alkyl, $-(CH_2)_p-COOH$,

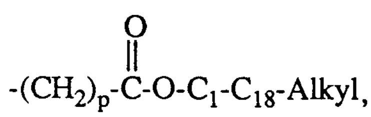,

$-(CH_2)_bOH$, $-(CH_2)_b-OCO-C_1-C_{18}$-Alkyl, $-CH_2-S-C_1-C_{18}$-Alkyl,

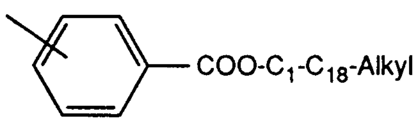 oder

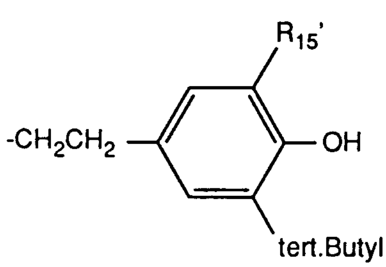

darstellt, wobei $R_5$ $C_1$-$C_{18}$-Alkyl, Cyclohexyl, Methylcyclohexyl, Benzyl oder

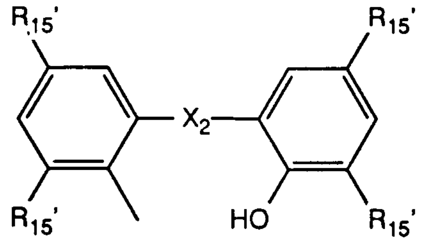

ist,
$R_6$ $C_1$-$C_{18}$-Alkyl, Cyclohexyl, Benzyl, $-(CH_2)_f$-OH oder $-CH_2CH_2O$-CO-$C_1$-$C_{18}$-Alkyl bedeutet,
$R_8$ Wasserstoff, -CO-$C_1$-$C_{18}$-Alkyl oder $-COCH=CH_2$ ist,
die $R_{15}$' unabhängig voneinander Methyl oder tert.Butyl sind,
$R_{17}$ $C_1$-$C_{11}$-Alkyl, $-(CH_2)_m$-S-$C_1$-$C_{18}$-Alkyl oder

$$-(CH_2)_m-\overset{O}{\overset{\|}{P}}-(C_1\text{-}C_4\text{-Alkoxy})_2$$

darstellt,
oder A, bei n = 2, $-O-(CH_2)_g-O-$, $-NH-(CH_2)_g-NH-$, $-OCH_2CH_2SCH_2CH_2O-$, $-O(CH_2CH_2O)_c-CH_2CH_2-O-$, $-OCH_2C(CH_3)_2CH_2O-$, $-NHCH_2CH_2OCH_2CH_2O-$,

$$-NHCH_2CH_2O\overset{O}{\overset{\|}{C}}(CH_2)_a\overset{O}{\overset{\|}{C}}OCH_2CH_2NH-,\quad -NH-NH-\overset{O}{\overset{\|}{C}}(CH_2)_a\overset{O}{\overset{\|}{C}}-NH-NH-,$$

$-NH-N=CH-C(CH_3)_2-S-S-C(CH_3)_2-CH=N-NH-$, Phenylen oder $-(CH_2)_p-$ bedeutet, wobei a, b, c, f, g, m, p und $X_2$ die in Anspruch 1 angegebene Bedeutung haben.

**5.** Zusammensetzung nach Anspruch 1, worin R Chlor, Methyl oder Methoxy und insbesondere Wasserstoff darstellt, $R_1$, $R_2$, $R_3$ und $R_4$ Wasserstoff sind,
n 1 oder 2 ist,
A, bei n = 1, $-OR_5$, $-NHR_6$, $-NH-NH-R_8$, $-NH-N=CH-R_{17}$, $C_8$-$C_{17}$-Alkyl, $-(CH_2)_{p'}$-CO-O-$C_8$-$C_{18}$-Alkyl mit p' = 2 bis 6, $-(CH_2)_b$-OH, $-(CH_2)_b$-OCO-$C_8$-$C_{18}$-Alkyl, $-CH_2$-S-$C_8$-$C_{18}$-Alkyl,

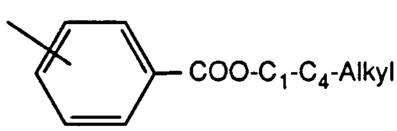
oder
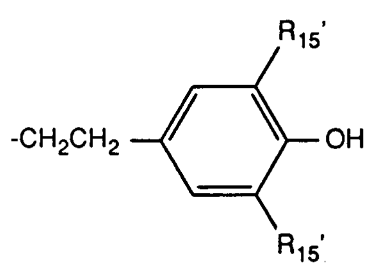

darstellt,

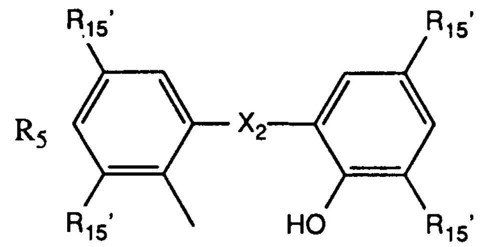

und insbesondere $C_8$-$C_{18}$-Alkyl ist,
$R_6$ $C_8$-$C_{18}$-Alkyl, $-(CH_2)_{f''}$-OH mit f" = 2 oder 3 oder $-CH_2CH_2OCO$-$C_8$-$C_{18}$-Alkyl bedeutet,
$R_8$ -CO-$C_8$-$C_{18}$-Alkyl oder $-COCH=CH_2$ ist,
$R_{17}$ $C_8$-$C_{11}$-Alkyl, $(CH_2)_m$-S-$C_8$-$C_{18}$-Alkyl oder

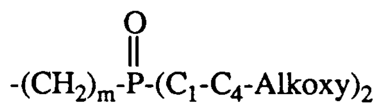

darstellt,
oder A, bein = 2, $-O(CH_2)_gO-$, $-NH(CH_2)_gNH-$, $-OCH_2CH_2SCH_2CH_2O-$, $-O(CH_2CH_2O)_cCH_2CH_2O-$, $-OCH_2C(CH_3)_2CH_2O-$, $-NHCH_2CH_2OCH_2CH_2O-$,$-NHCH_2CH_2OCO-(CH_2)_{a''}-OCOCH_2CH_2NH-$ oder $-NH-NHCO-(CH_2)_{a''}-CONH-NH-$ mit a″ = 2 bis 8, $-NH-N=CH-C(CH_3)_2-S-S-C(CH_3)_2-CH=N-NH-$, $-(CH_2)_{p''}-$ mit p″ = 5-10 oder Phenylen bedeutet,
b 3 bis 5, besonders 5 ist, $X_2$ die direkte Bindung oder $>CH-CH_3$ ist, die $R_{15}'$ unabhängig voneinander Methyl oder tert. Butyl sind und c, g und m die in Anspruch 1 angegebene Bedeutung haben.

**6.** Zusammensetzung nach Anspruch 1, worin R, $R_1$, $R_2$, $R_3$ und $R_4$ Wasserstoff bedeuten,
n 1 oder 2 ist,
A, bei n = 1, $C_8$-$C_{17}$-Alkyl, $-OR_5$, $-NHR_6$ oder -NH-NH-CO-$C_8$-$C_{17}$-Alkyl ist,
$R_5$ und $R_6$ unabhängig voneinander $C_8$-$C_{18}$-Alkyl darstellen,
oder A, bei n = 2, $-O(CH_2)_g-O-$, $-NH(CH_2)_gNH-$, $-OCH_2C(CH_3)_2CH_2O-$, $-NH-NHCO-CH_2CH_2-OCNH-NH-$, $-(CH_2)_{p''}-$ mit p″ = 5 bis 10 oder Phenylen bedeutet, wobei g die in Anspruch 1 angegebene Bedeutung hat.

**7.** Zusammensetzung nach Anspruch 1, enthaltend eine Verbindung der Formel Ia

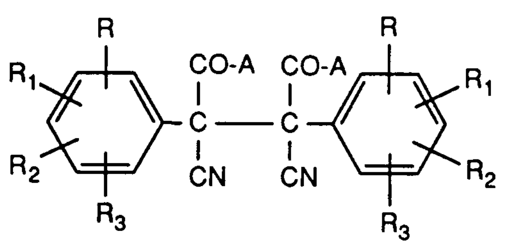

(Ia),

worin R Wasserstoff, $C_1$-$C_4$-Alkyl, $C_1$-$C_4$-Alkoxy oder Chlor ist, $R_1$, $R_2$ und $R_3$ Wasserstoff sind und A $-OC_1$-$C_{18}$-Alkyl, besonders $-OC_1$-$C_4$-Alkyl bedeutet.

**8.** Zusammensetzung nach Anspruch 1, worin das organische Material ein Schmierstoff, eine Metallbearbeitungsflüssigkeit, eine Hydraulikflüssigkeit, ein natürliches, halbsynthetisches oder synthetisches Polymer ist.

**9.** Zusammensetzung nach Anspruch 8, worin das organische Material ein Schmierstoff, ein Elastomer oder ein halogenfreier thermoplastischer Kunststoff, insbesondere ein halogenfreies Polyolefin ist.

**10.** Verbindungen der Formel Ib

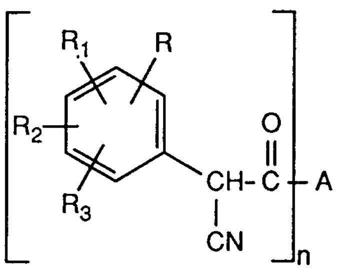

(Ib),

worin R Wasserstoff, -OH, Halogen, $C_1$-$C_{20}$-Alkyl, $C_2$-$C_{20}$-Alkenyl, $C_5$-$C_{12}$-Cycloalkyl, $C_1$-$C_4$-Alkyl-$C_5$-$C_{12}$-cycloalkyl, Phenyl, Naphthyl, $C_1$-$C_{20}$-Alkoxy, Phenyloxy, Naphthyloxy, -S-$C_1$-$C_{20}$-Alkyl, -S-Phenyl, -S-Naphthyl, -O-CO-$C_1$-$C_{20}$-Alkyl, -O-CO-Phenyl, -O-CO-Naphthyl, -COOH, -COO-$C_1$-$C_{20}$-Alkyl, -COO-$C_2$-$C_{20}$-Alkenyl, -COO-Phenyl, -COO-Naphthyl, -CONR'R", -CO-$C_1$-$C_{20}$-Alkyl, -CO-Phenyl, -CO-Naphthyl, -NHCO-$C_1$-$C_{20}$-Alkyl, -NHCO-$C_2$-$C_{20}$-Alkenyl, -NHCO-Phenyl oder -NHCO-Naphthyl bedeutet,
$R_1$ dieselbe Bedeutung wie R haben kann,
$R_2$ Wasserstoff, Halogen oder $C_1$-$C_4$-Alkyl ist und
$R_3$ Wasserstoff oder Halogen bedeutet,
oder zwei in ortho-Stellung zueinander gebundene Reste R, $R_1$, $R_2$ oder $R_3$ zusammen Tetramethylen oder -CH=CH-CH=CH- bilden, wobei die restlichen zwei von R, $R_1$, $R_2$ und $R_3$ Wasserstoff sind,
oder $R_1$ bis $R_3$ Wasserstoff sind und R bei n = 1 eine Gruppierung

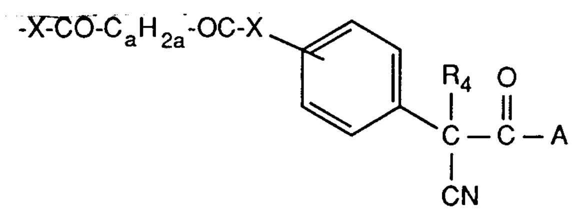

oder

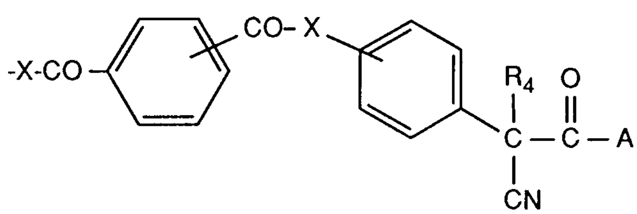

darstellt, worin $R_4$ jeweils H ist,
R' und R" unabhängig voneinander Wasserstoff, $C_1$-$C_{20}$-Alkyl, $C_2$-$C_{20}$-Alkenyl, Phenyl oder Naphthyl bedeuten oder zusammen mit dem Bindungs-N-Atom einen 5- oder 6-gliedrigen heterocyclischen Ring bilden,
a Null bis 10 ist, n 1 bis 6 ist,
X -O- oder -NH- bedeutet,
A bei n = 1, $-OR_5$, $-NR_6R_7$, $-NH-NR_8R_9$, $C_1$-$C_{30}$-Alkyl, $C_2$-$C_{30}$-Alkenyl, $C_5$-$C_{12}$-Cycloalkyl, $C_1$-$C_4$-Alkyl-$C_5$-$C_{12}$-cycloalkyl,

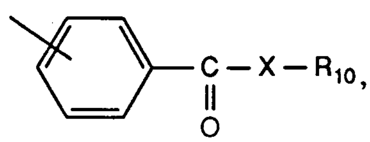

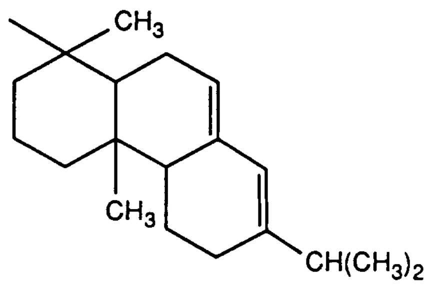

oder das entsprechende Dihydro- oder Tetrahydroderivat, $-C_aH_{2a}-CO-X-R_{10}$, $-C_bH_{2b}-OCO-R_{10}$, $-C_bH_{2b}-OH$,

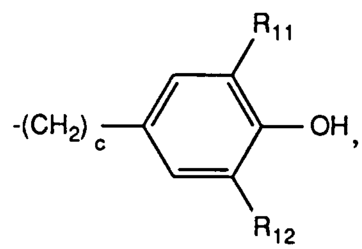

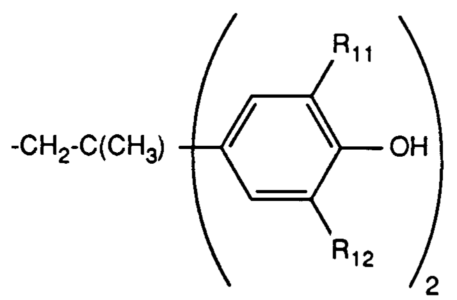, 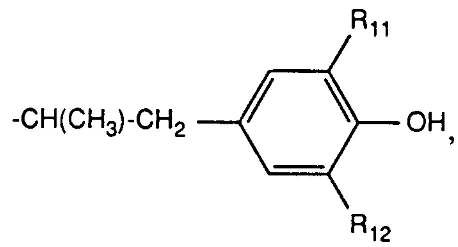,

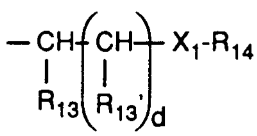 oder 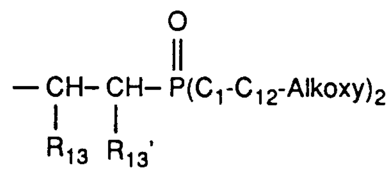

bedeutet, wobei
$R_5$ $C_1$-$C_{30}$-Alkyl, $C_2$-$C_{30}$-Alkenyl, $C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl, Biphenyl, der Rest eines Terpenalkohols oder ein Rest der Formeln

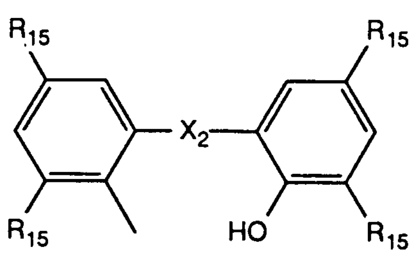, 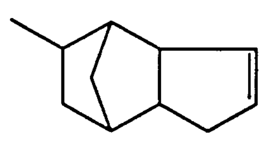, 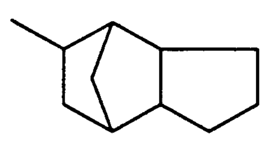,

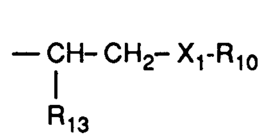 oder 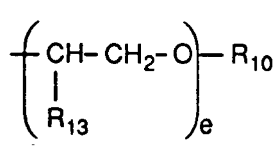

ist,
$R_6$ und $R_7$ unabhängig voneinander Wasserstoff, $C_1$-$C_{20}$-Alkyl, $C_2$-$C_{20}$-Alkenyl, $C_5$-$C_{12}$-Cycloalkyl, $C_1$-$C_4$-Alkyl-$C_5$-$C_{12}$-cycloalkyl, Phenyl-$C_1$-$C_4$-alkyl, Phenyl, Naphthyl,

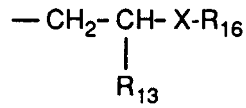

oder $-CH_2C(CH_2-O-R_{16})_3$ sind oder $R_6$ bei $R_7$ = H auch $-(CH_2)_f-X-R_{16}$ sein kann oder $R_6$ und $R_7$ zusammen mit dem N-Bindungsatom einen 5- oder 6-gliedrigen heterocyclischen Ring bilden,
$R_8$ und $R_9$ unabhängig voneinander Wasserstoff, $C_1$-$C_{20}$-Alkyl, Phenyl, -CO-$C_1$-$C_{20}$-Alkyl, -CO-$C_2$-$C_{18}$-Alkenyl, -CO-Phenyl oder -CO-Naphthyl sind oder zusammen $=CH-R_{17}$ darstellen,
$R_{10}$ Wasserstoff, $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl, Naphthyl, -CO-$C_1$-$C_{20}$-Alkyl, -CO-Naphthyl oder

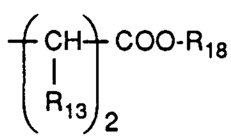

ist,
$R_{11}$ und $R_{12}$ unabhängig voneinander Wasserstoff, $C_1$-$C_4$-Alkyl, Cyclohexyl oder Phenyl sind,
$R_{13}$ Wasserstoff oder Methyl und $R_{13}'$ Wasserstoff, Methyl oder Phenyl sind,
$R_{14}$ $C_1$-$C_{20}$-Alkyl, $C_2$-$C_{20}$-Alkenyl, $C_5$-$C_{12}$-Cycloalkyl, Phenyl-$C_1$-$C_4$-alkyl, Phenyl oder Naphthyl darstellt,
die $R_{15}$ unabhängig voneinander $C_1$-$C_4$-Alkyl sind,
$R_{16}$ Wasserstoff, -CO-$C_1$-$C_{20}$-Alkyl, -CO-$C_2$-$C_{20}$-Alkenyl, -CO-Phenyl oder -CO-Naphthyl darstellt,
$R_{17}$ $C_1$-$C_{20}$-Alkyl, $C_2$-$C_{20}$-Alkenyl, $C_5$-$C_{12}$-Cycloalkyl, Phenyl, Naphthyl, Furyl-2,

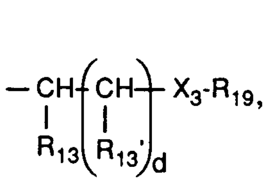, 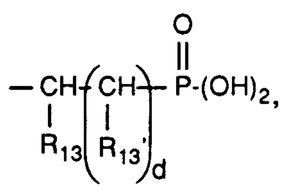, 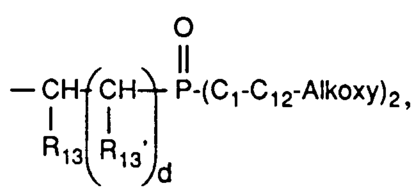,

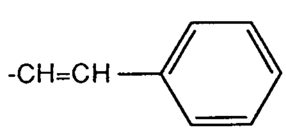, 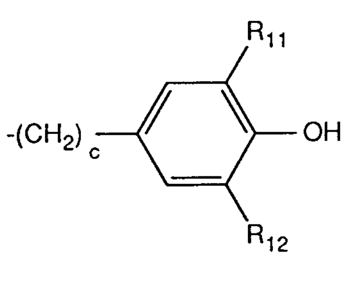 oder 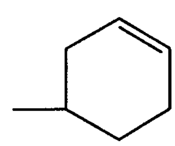

ist,
$R_{18}$ Wasserstoff, $C_1$-$C_{20}$-Alkyl oder $C_2$-$C_{20}$-Alkenyl darstellt,
$R_{19}$ $C_1$-$C_{20}$-Alkyl, $C_5$-$C_{12}$-Cycloalkyl, Phenyl oder $-CH_2-COO-C_{12}-C_{20}$-Alkyl ist,
X und a die oben angegebene Bedeutung haben,
$X_1$ -O-, -S-, -NH- oder $-NR_{14}-$,
$X_2$ die direkte Bindung, $-CH_2-$, $>CH-CH_3$ oder -S- und
$X_3$ -O-, -S-, -NH- oder $-NR_{19}-$ sind,
b 3 bis 5,

c 0 bis 2,
d 0 oder 1,
e 2 bis 10 und
f 2 bis 6 sind;
mit der Massgabe, dass A ungleich $-OC_1-C_{12}$-Alkyl, $-NH_2$, -NH-Methyl, -NH-Cyclohexyl, -NH-Phenyl,

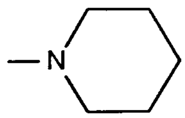 ,

$-NH-NH_2$, $C_1-C_7$-Alkyl, $-CH_2N(CH_3)_2$, $-CH_2N$(Methyl)-(Phenyl) oder $-C_{a''}H_{2a''}-CO-O-R_{10'}$ mit a'' = 2 oder 4 und $R_{10'}$ = Wasserstoff, Methyl oder Ethyl, ist, wenn einer der Reste R, $R_1$, $R_2$ oder $R_3$ Wasserstoff, Chlor, Methyl oder Methoxy ist, und die übrigen Reste R, $R_1$, $R_2$ oder $R_3$ Wasserstoff darstellen;
, und daß Verbindungen der Formel Ib ausgeschlossen sind, worin
R, $R_1$, $R_2$, $R_3$ = H und A = $C_1-C_{24}$-Alkyl, $-O-C_1-C_{24}$-Alkyl, $-NH_2$, $-NH-C_1-C_{20}$-Alkyl oder $-N(C_1-C_{20}$-Alkyl$)_2$, Piperidino, Morpholino, Pyrrolidino, Dimethylamino und Diethylamino sind, sowie Verbindungen, worin $R_{16}$ = Wasserstoff und f = 2 sind, und daß die Verbindungen der Formeln

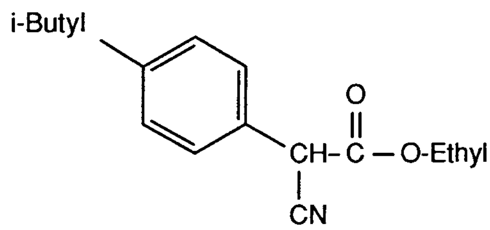 ,

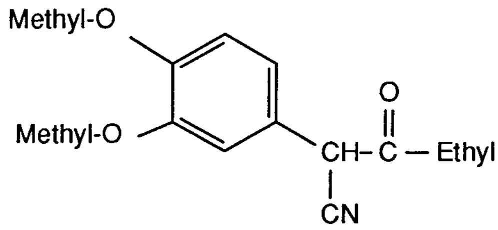 ,

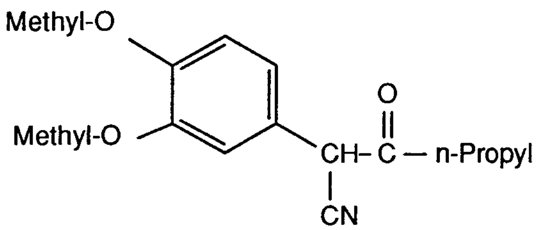 ,

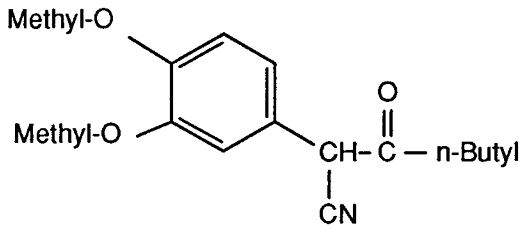 ,

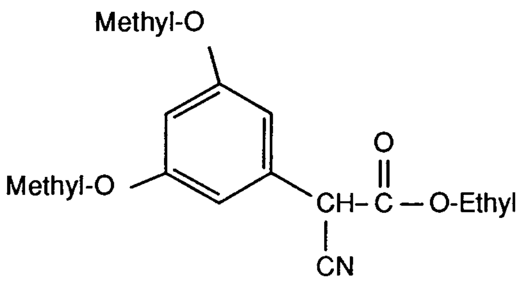

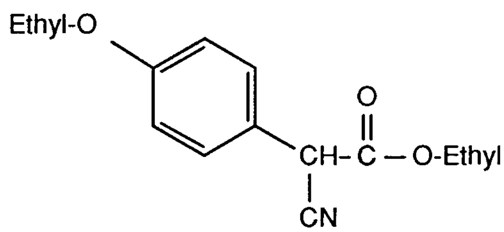 ,

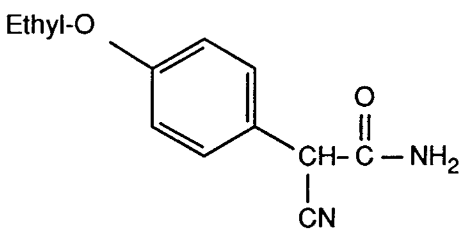

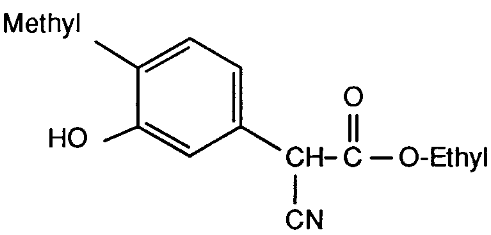 ,

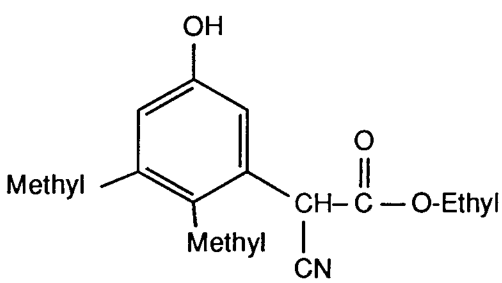

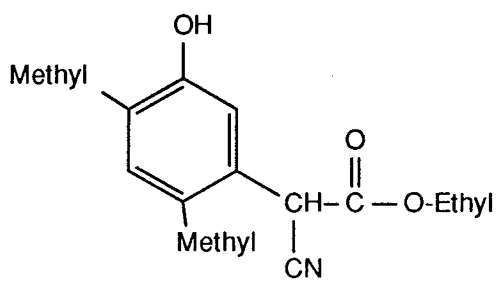 ,

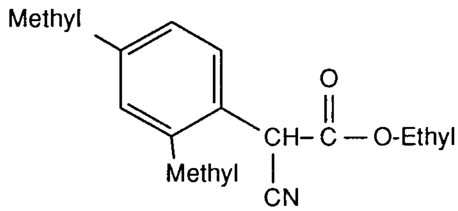 , 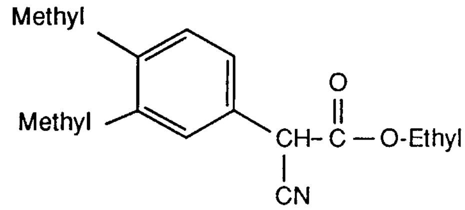 ,

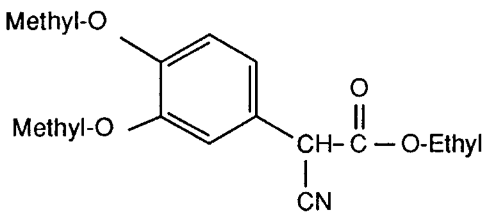 , 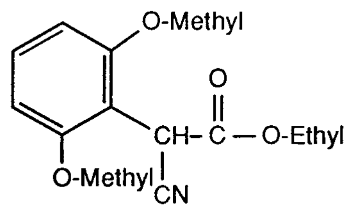 ,

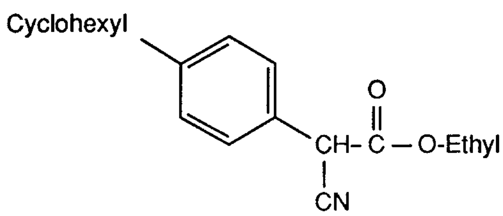 , 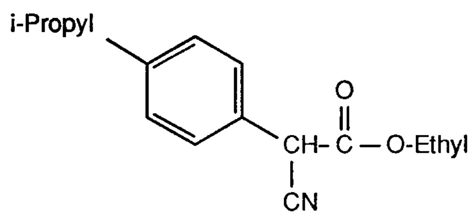 ,

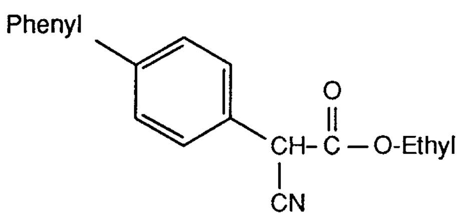 , 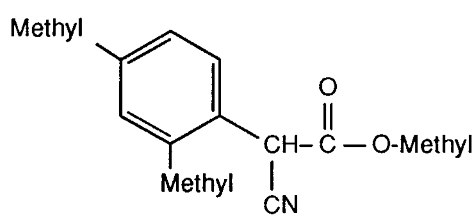 ,

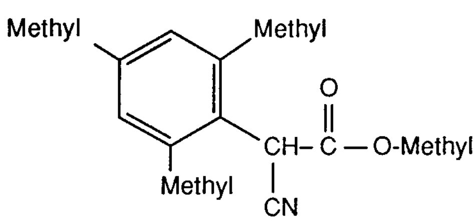 , 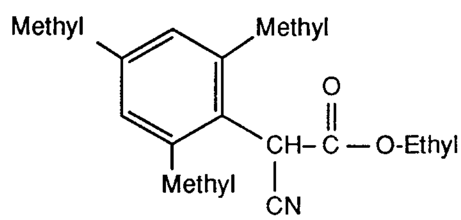 und

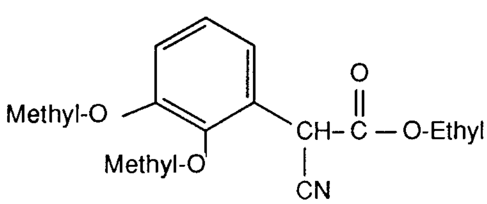

ebenfalls ausgeschlossen sind;
oder A, bei n = 2, $-X'-C_gH_{2g}-X'-$,

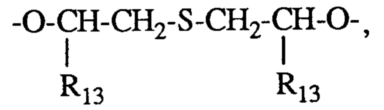

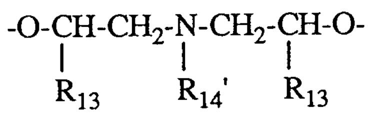, 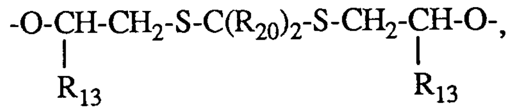

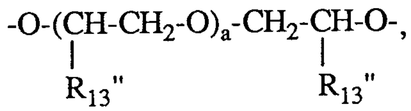

-OCH$_2$CH = CHCH$_2$O-, -OCH$_2$C≡CCH$_2$O-, -O(CH$_2$)$_h$-NH-CO-X$_4$-OC-NH(CH$_2$)$_h$-O-, -NH(CH$_2$)$_2$-X(CH$_2$)$_2$-O-,-NH(CH$_2$CH$_2$NH)$_i$-CH$_2$CH$_2$NH-, -NH(CH$_2$CH$_2$CH$_2$NH)$_i$CH$_2$CH$_2$CH$_2$NH-,

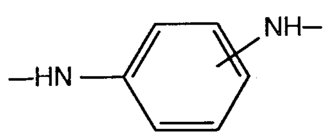, 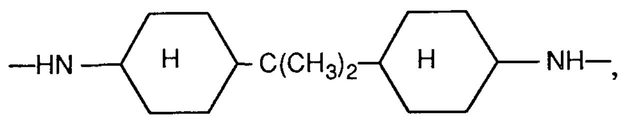

-NH(CH$_2$)$_h$-O-CO-X$_4$-OC-O-(CH$_2$)$_h$-NH-, -NH-NH-, -NH-NH-CO-X$_4$-OC-NH-NH-, -NH-N = CH-X$_5$-CH = N-NH-, -C$_p$H$_{2p}$-, -(CH$_2$)$_m$-X$_1$-(CH$_2$)$_m$-,

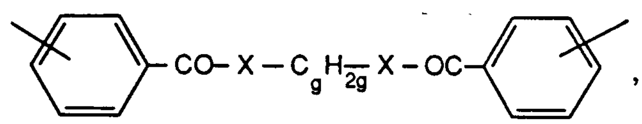,

-C$_p$-H$_{2p}$-CO-X-C$_g$H$_{2g}$-X-OC-C$_p$H$_{2p}$-, -(CH$_2$)$_k$-O-CO-X$_4$-CO-O-(CH$_2$)$_k$-, -CH = CH-CO-X-C$_g$H$_{2g}$-X-OC-CH = CH-, -CH(OH)CH$_2$-, -CH(OH)CH(OH)-,

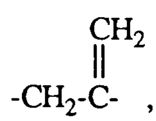,

-CH = CH-, CH(R$_{21}$)- oder

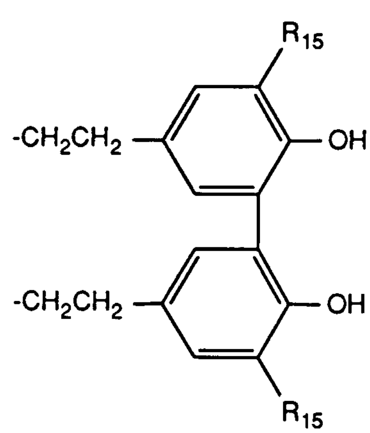

darstellt,
wobei X, $R_{13}$, $R_{15}$, a und f die oben angegebene Bedeutung haben,
die X' unabhängig voneinander -O- oder -NH- bedeuten,
$R_{13}''$ Wasserstoff oder Methyl ist und bei a = Null auch $C_2$-$C_{18}$-Alkyl oder Phenyl sein kann,
$R_{14}'$ dieselbe Bedeutung wie $R_{14}$ haben kann oder Wasserstoff ist,
die $R_{20}$ unabhängig voneinander Wasserstoff, $C_1$-$C_{20}$-Alkyl, Phenyl oder zusammen einen 5-12-gliedrigen cycloaliphatischen Ring darstellen,
$R_{21}$ $C_5$-$C_{12}$-Cycloalkyl, Phenyl-$C_1$-$C_4$-alkyl, Phenyl oder

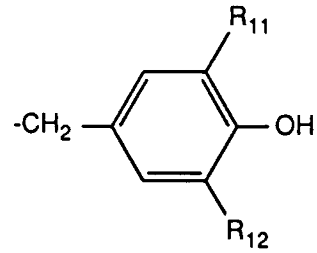

darstellt,
g 2 bis 12, h 2 bis 6, i 1 bis 5, k 3 bis 5, m 1 oder 2 und p 1 bis 10 sind,
$X_4$ -$C_aH_{2a}$-, Phenylen oder -CH=CH- ist und
$X_5$ die direkte Bindung, -$(CH_2)_3$- oder -$C(CH_3)_2$-S-S-$C(CH_3)_2$- darstellt;
oder A, bei n = 3,

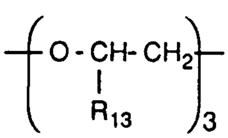, 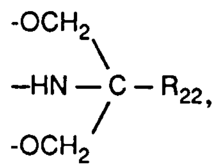, 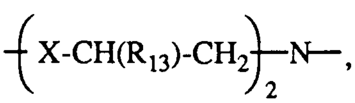,

-$(OCH_2)_3$-C-$R_{13}'$, $N(CH_2)_3$- oder

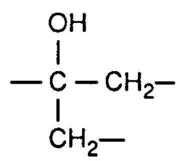

bedeutet, wobei $R_{13}$ und $R_{13}'$ die oben angegebene Bedeutung haben und $R_{22}$ Methyl oder Ethyl ist;
oder A, bei n = 4, $C(CH_2O)_4$-,

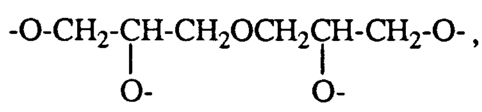

-$(OCH_2)_3$-C-NH- oder -$(CH_2)_2NCH_2CH_2N(CH_2)_2$- ist;
oder A, bei n = 5,

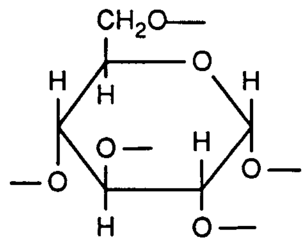

darstellt und
bei n = 6

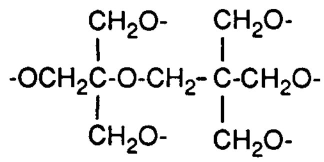

ist.

**11.** Verbindungen nach Anspruch 10, worin R und $R_1$ unabhängig voneinander Wasserstoff, $C_1$-$C_4$-Alkyl, $C_1$-$C_4$-Alkoxy oder Halogen, besonders Chlor, bedeuten und $R_2$ und $R_3$ Wasserstoff sind.

**12.** Verbindungen nach Anspruch 10, worin R Wasserstoff, $C_1$-$C_4$-Alkyl, $C_1$-$C_4$-Alkoxy oder Chlor ist, $R_1$, $R_2$ und $R_3$ Wasserstoff sind, n 1 oder 2 ist,
A, bei n = 1, $-OR_5$, $-NHR_6$, $-NH-NH-R_8$, $-NH-N=CH-R_{17}$, $C_1$-$C_{17}$-Alkyl, $C_2$-$C_{17}$-Alkenyl,

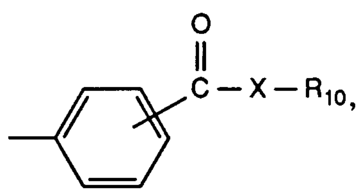

$-(CH_2)_pCO-X-R_{10}$, $-CH_2-X_1-C_1-C_{18}$-Alkyl,

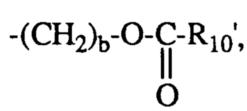

$-(CH_2)_b-OH$,

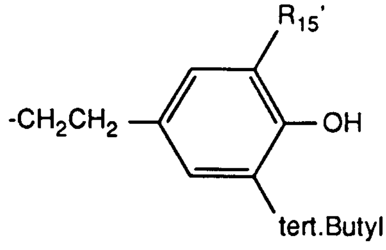 oder 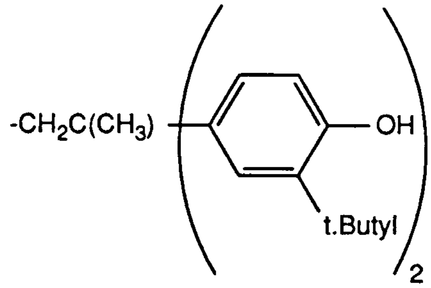,

$R_{10}$ Wasserstoff, $C_1$-$C_{18}$-Alkyl, $C_2$-$C_{18}$-Alkenyl, Cyclohexyl, Methylcyclohexyl oder Benzyl und
$R_{10}'$ $C_1$-$C_{17}$-Alkyl, $C_2$-$C_{17}$-Alkenyl oder Phenyl bedeuten,
$R_5$ $C_1$-$C_{18}$-Alkyl,

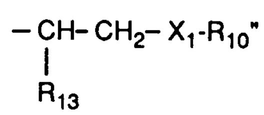 oder 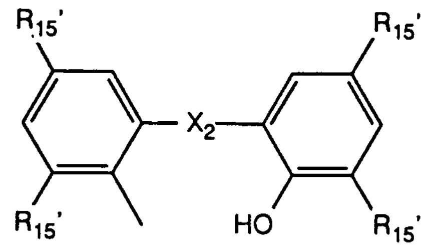

bedeutet, worin $R_{10}''$ Wasserstoff, $C_1$-$C_{18}$-Alkyl oder $C_2$-$C_{18}$-Alkenyl und die $R_{15}'$ unabhängig voneinander Methyl oder tert.Butyl sind,
$R_6$ $C_1$-$C_{18}$-Alkyl, $C_2$-$C_{18}$-Alkenyl, Methylcyclohexyl, Benzyl, $-CH_2CH_2OH$,

$$-CH_2CH_2O-\underset{\underset{O}{\|}}{C}-C_1\text{-}C_{18}\text{-Alkyl} \text{ oder } -CH_2CH_2O-\underset{\underset{O}{\|}}{C}-C_2\text{-}C_{18}\text{-Alkenyl}$$

ist,
$R_8$ Wasserstoff, $C_1$-$C_{12}$-Alkyl, -CO-$C_1$-$C_{18}$-Alkyl oder -CO-$C_2$-$C_{18}$-Alkenyl darstellt,
$R_{17}$ $C_1$-$C_{11}$-Alkyl, $C_2$-$C_{11}$-Alkenyl, $-(CH_2)_m$-S-$C_1$-$C_{18}$-Alkyl oder

$$-(CH_2)_m-\overset{\overset{O}{\|}}{P}-(C_1\text{-}C_8\text{-Alkoxy})_2$$

ist;
oder A, bei n = 2, $-O-(CH_2)_g-O-$, $-NH-(CH_2)_g-NH-$,

$$-O-\underset{\underset{R_{13}}{|}}{CH}-CH_2-S-CH_2-\underset{\underset{R_{13}}{|}}{CH}-O-,$$

$$-O-\underset{\underset{R_{13}}{|}}{CH}-CH_2-NH-CH_2\underset{\underset{R_{13}}{|}}{CH}-O-,\ -O-(\underset{\underset{R_{13}}{|}}{CH}-CH_2O)_{a'}-CH_2\underset{\underset{R_{13}}{|}}{CH}-O-$$

mit a' = Null bis 5, oder $-OCH_2-C(CH_3)_2CH_2-O-$, $-NHCH_2CH_2-X-CH_2CH_2O-$, $-NH(CH_2CH_2NH)_i-CH_2CH_2NH-$, $-NH(CH_2CH_2CH_2NH)_i-CH_2CH_2CH_2-NH$, $-NH-C(CH_3)_2-CH_2O-$,

$$-NHCH_2CH_2O\overset{\overset{O}{\|}}{C}(CH_2)_a\overset{\overset{O}{\|}}{C}OCH_2CH_2NH-,\ -NH-NH-\overset{\overset{O}{\|}}{C}(CH_2)_a\overset{\overset{O}{\|}}{C}-NH-NH-,$$

$-NH-N=CH-X_5-CH=N-NH-$, $-(CH_2)_p-$,

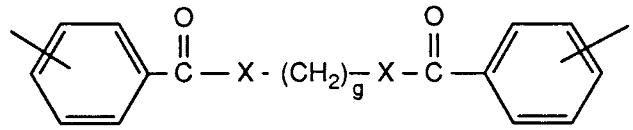

$$-(CH_2)_p-\overset{\overset{O}{\|}}{C}-X-(CH_2)_g-X-\overset{\overset{O}{\|}}{C}-(CH_2)_p-,\quad -(CH_2)_5-O-\overset{\overset{O}{\|}}{C}-(CH_2)_a-\overset{\overset{O}{\|}}{C}-O-(CH_2)_5-,$$

$-CH(R_{21})-$ mit $R_{21}$ = Benzyl, Phenyl oder

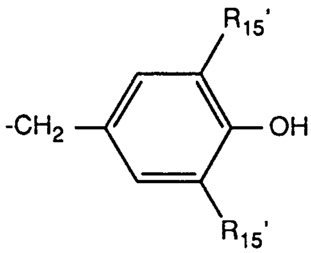

oder A

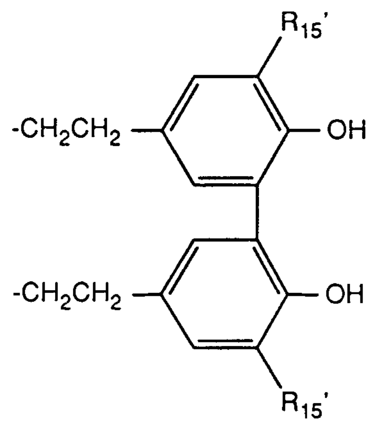

darstellt,
und a, b, g, i, m, p, X, $X_1$, $X_2$, $X_5$ und $R_{13}$ die in Anspruch 1 angegebene Bedeutung haben.

**13.** Verbindungen nach Anspruch 10, worin R Wasserstoff, Chlor, Methyl oder Methoxy ist und $R_1$, $R_2$ und $R_3$ Wasserstoff sind,
n 1 oder 2 ist,
A, bei n = 1, $-OR_5$, $-NHR_6$, $-NH-NH-R_8$, $-NH-N=CH-R_{17}$, $C_8$-$C_{17}$-Alkyl, $-(CH_2)_p-COOH$,

$$-(CH_2)_p-\overset{\overset{O}{\|}}{C}-O-C_{13}\text{-}C_{18}\text{-Alkyl},$$

$-(CH_2)_bOH$, $-(CH_2)_b-OCO-C_1-C_{18}$-Alkyl, $-CH_2-S-C_1-C_{18}$-Alkyl,

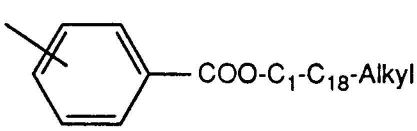

oder

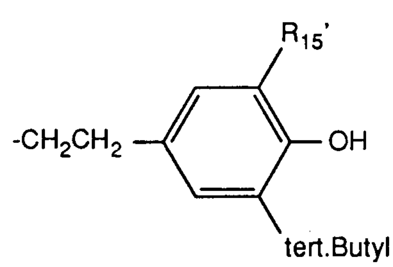

darstellt,
wobei $R_5$ $C_{13}$-$C_{18}$-Alkyl, Cyclohexyl, Methylcyclohexyl, Benzyl oder

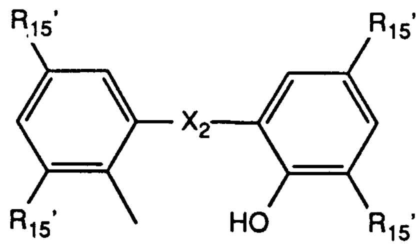

ist,
$R_6$ $C_2$-$C_{18}$-Alkyl, Benzyl, -$(CH_2)_f$-OH oder -$CH_2CH_2O$-CO-$C_1$-$C_{18}$-Alkyl bedeutet,
$R_8$ -CO-$C_1$-$C_{18}$-Alkyl oder -COCH = $CH_2$ ist,
die $R_{15}$' unabhängig voneinander Methyl oder tert.Butyl sind,
$R_{17}$ $C_1$-$C_{11}$-Alkyl, -$(CH_2)_m$-S-$C_1$-$C_{18}$-Alkyl oder

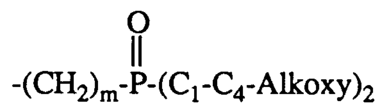

darstellt,
oder A, bei n = 2, -O-$(CH_2)_g$-O-, -NH-$(CH_2)_g$-NH-, -$OCH_2CH_2SCH_2CH_2O$-, -O$(CH_2CH_2O)_c$-$CH_2CH_2$-O-, -$OCH_2C(CH_3)_2CH_2O$-, $NHCH_2CH_2OCH_2CH_2O$-,

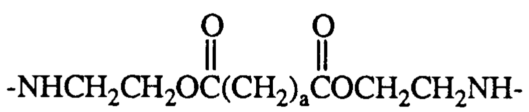, 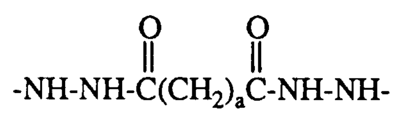,

-NH-N = CH-C$(CH_3)_2$-S-S-C$(CH_3)_2$-CH = N-NH- oder -$(CH_2)_p$- bedeutet,
wobei a, b, c, f, g, m, p und $X_2$ die in Anspruch 10 angegebene Bedeutung haben.

**14.** Verbindungen nach Anspruch 10, worin R Chlor, Methyl oder Methoxy und insbesondere Wasserstoff darstellt, $R_1$, $R_2$ und $R_3$ Wasserstoff sind,
n 1 oder 2 ist,
A, bei n = 1, -$OR_5$, -$NHR_6$, -NH-NH-$R_8$, -NH-N = CH-$R_{17}$, $C_8$-$C_{17}$-Alkyl, -$(CH_2)_{p'}$-CO-O-$C_8$-$C_{18}$-Alkyl mit p' = 2 bis 6, -$(CH_2)_b$-OH, -$(CH_2)_b$-OCO-$C_8$-$C_{18}$-Alkyl, -$CH_2$-S-$C_8$-$C_{18}$-Alkyl,

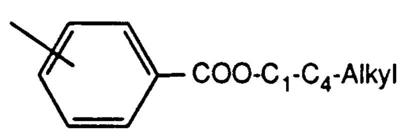 oder 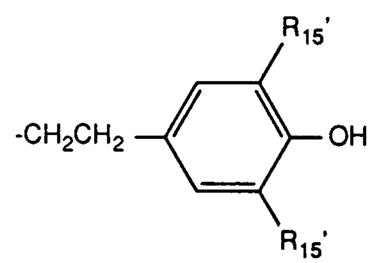

darstellt,

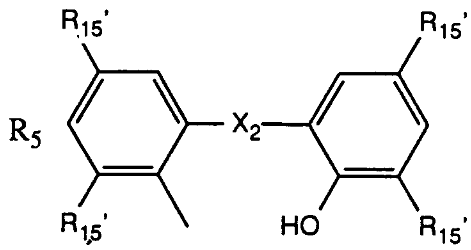

und insbesondere $C_{13}$-$C_{18}$-Alkyl ist,
$R_6$ $C_8$-$C_{18}$-Alkyl, $-(CH_2)_{f''}$-OH mit f" = 2 oder 3 oder $-CH_2CH_2OCO$-$C_8$-$C_{18}$-Alkyl bedeutet,
$R_8$ -CO-$C_8$-$C_{18}$-Alkyl oder $-COCH=CH_2$ ist,
$R_{17}$ $C_8$-$C_{11}$-Alkyl, $(CH_2)_m$-S-$C_8$-$C_{18}$-Alkyl oder

$$-(CH_2)_m-\overset{\overset{\displaystyle O}{\|}}{P}-(C_1\text{-}C_4\text{-Alkoxy})_2$$

darstellt,
oder A, bei n = 2, $-O(CH_2)_gO-$ $-NH(CH_2)_gNH-$, $-OCH_2CH_2SCH_2CH_2O-$, $-O(CH_2CH_2O)_cCH_2CH_2O-$, $-OCH_2C(CH_3)_2CH_2O-$, $NHCH_2CH_2OCH_2CH_2O-$, $-NHCH_2CH_2OCO-(CH_2)_{a''}-OCOCH_2CH_2NH-$ oder -NH-NHCO-$(CH_2)_{a''}$-CONH-NH- mit a" = 2 bis 8, $-NH-N=CH-C(CH_3)_2-S-S-C(CH_3)_2-CH=N-NH-$ oder $-(CH_2)_{p''}-$ mit p" = 5-10 bedeutet,
b 3 bis 5, besonders 5 ist, $X_2$ die direkte Bindung oder $>CH-CH_3$ ist, die $R_{15}$' unabhängig voneinander Methyl oder tert. Butyl sind und c, g und m die in Anspruch 10 angegebene Bedeutung haben.

**15.** Verbindungen nach Anspruch 10, worin R, $R_1$, $R_2$ und $R_3$ Wasserstoff bedeuten, n 1 oder 2 ist,
A, bei n = 1, $C_8$-$C_{17}$-Alkyl, $-OR_5$, $-NHR_6$ oder -NH-NH-CO-$C_8$-$C_{17}$-Alkyl ist,
$R_5$ $C_{13}$-$C_{18}$-Alkyl ist und $R_6$ $C_8$-$C_{18}$-Alkyl darstellt,
oder A, bei n = 2, $-O(CH_2)_g-O-$, $-NH(CH_2)_gNH-$, $-OCH_2C(CH_3)_2CH_2O-$, $-NH-NHCO-CH_2CH_2-OCNH-NH-$ oder $-(CH_2)_{p''}$ mit p" = 5 bis 10 bedeutet, wobei g die in Anspruch 10 angegebene Bedeutung hat.

**16.** Verwendung von Verbindungen der Formel I nach Anspruch 1 oder von Verbindungen der Formel Ib nach Anspruch 10 zum Stabilisieren von Schmierstoffen, Metallbearbeitungs flüssigkeiten, Hydraulik flüssigkeiten oder natürlichen, halbsynthetischen oder synthetischen Polymeren gegen oxidativen, thermischen und/oder aktinischen Abbau.

**17.** Verfahren zum Stabilisieren von Schmierstoffen, Metallbearbeitungs flüssigkeiten, Hydraulik flüssigkeiten oder natürlichen, halbsynthetischen oder synthetischen Polymeren, dadurch gekennzeichnet, dass man dem organischen Material als Stabilisatoren Verbindungen der Formel I nach Anspruch 1 oder Verbindungen der Formel Ib nach Anspruch 10 zusetzt bzw. auf dieses aufbringt.

## Claims

**1.** A composition comprising lubricants, metalworking fluids, hydraulic fluids or natural, semi-synthetic or synthetic polymers liable to thermal, oxidative and/or actinic degradation and at least one compound of the formula I

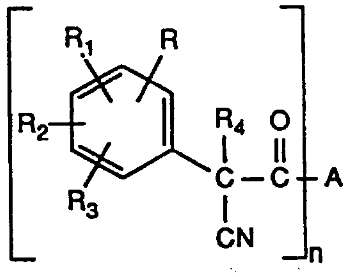

(I),

in which R is hydrogen, -OH, halogen, $C_1$-$C_{20}$alkyl, $C_2$-$C_{20}$alkenyl, $C_5$-$C_{12}$cycloalkyl, $C_1$-$C_4$alkyl-$C_5$-$C_{12}$cycloalkyl, phenyl, naphthyl, $C_1$-$C_{20}$alkoxy, phenyloxy, naphthyloxy, -S-$C_1$-$C_{20}$alkyl, -S-phenyl, -S-naphthyl, -O-CO-$C_1$-$C_{20}$alkyl, -O-CO-phenyl, -O-CO-naphthyl, -COOH, -COO-$C_1$-$C_{20}$alkyl, -COO-$C_2$-$C_{20}$alkenyl, -COO-phenyl, -COO-naphthyl, -CONR'R'', -CO-$C_1$-$C_{20}$alkyl, -CO-phenyl, -CO-naphthyl, -NHCO-$C_1$-$C_{20}$alkyl, -NHCO-$C_2$-$C_{20}$alkenyl, -NHCO-phenyl or -NHCO-naphthyl, $R_1$ can have the same meaning as R, $R_2$ is hydrogen, halogen or $C_1$-$C_4$alkyl and $R_3$ is hydrogen or halogen, or two radicals R, $R_1$, $R_2$ or $R_3$ bonded to one another in the ortho-position together form tetramethylene or -CH=CH-CH=CH-, where the remaining two radicals of R, $R_1$, $R_2$ and $R_3$ are hydrogen, or $R_1$ to $R_3$ are hydrogen and, if n = 1, R is a group

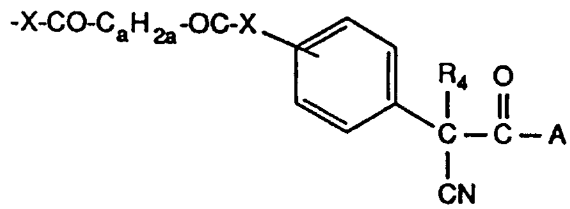

or

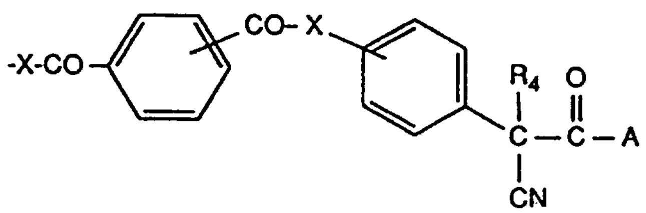

,

R' and R'' independently of one another are hydrogen, $C_1$-$C_{20}$alkyl, $C_2$-$C_{20}$alkenyl, phenyl or naphthyl or, together with the bonding N atom, form a 5- or 6-membered heterocyclic ring, a is zero to 10, n is 1 to 6, X is -O- or -NH-,
R is hydrogen or, if n = 1, is a group

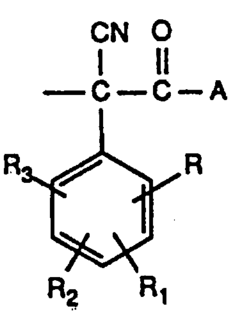

,

A, if n = 1, is -OH, -$OR_5$, -$NR_6R_7$, -NH-$NR_8R_9$, $C_1$-$C_{30}$alkyl, $C_2$-$C_{30}$alkenyl, $C_5$-$C_{12}$cycloalkyl, $C_1$-$C_4$alkyl-$C_5$-$C_{12}$cycloalkyl,

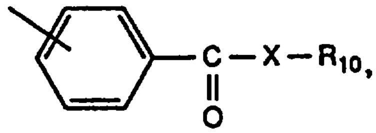

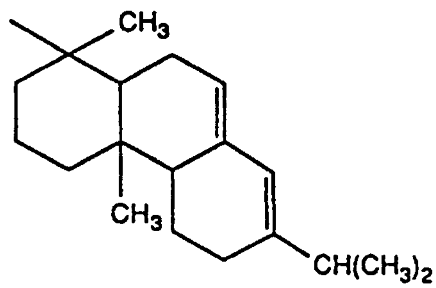

or the corresponding dihydro or tetrahydro derivative, $-C_aH_{2a}-CO-X-R_{10}$, $-C_bH_{2b}-OCO-R_{10}$, $-C_bH_{2b}-OH$,

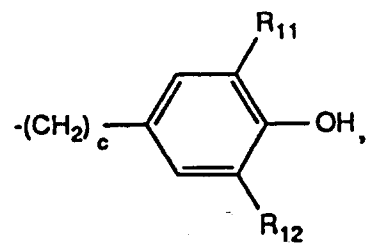

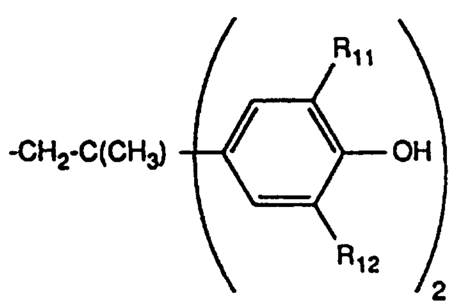

,

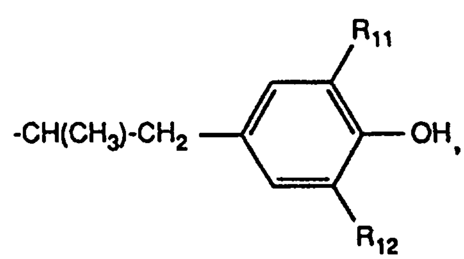

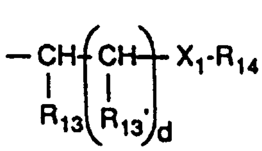

or

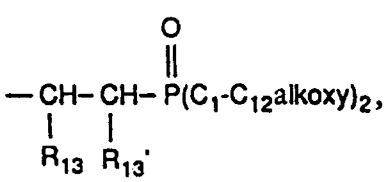

where $R_5$ is $C_1$-$C_{30}$alkyl, $C_2$-$C_{30}$alkenyl, $C_5$-$C_{12}$cycloalkyl, $C_1$-$C_4$alkyl-$C_5$-$C_{12}$cycloalkyl, phenyl-$C_1$-$C_4$alkyl, phenyl, naphthyl, biphenyl, the radical of a terpene alcohol or a radical of the formula

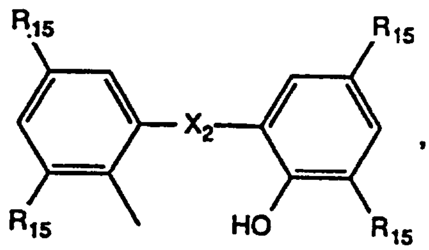, 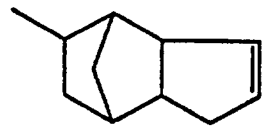, 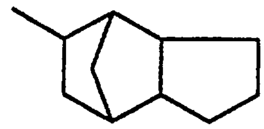,

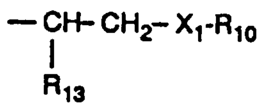 or 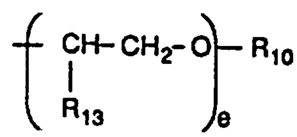,

$R_6$ and $R_7$ independently of one another are hydrogen, $C_1$-$C_{20}$alkyl, $C_2$-$C_{20}$alkenyl, $C_5$-$C_{12}$cycloalkyl, $C_1$-$C_4$alkyl-$C_5$-$C_{12}$cycloalkyl, phenyl-$C_1$-$C_4$alkyl, phenyl, naphthyl,

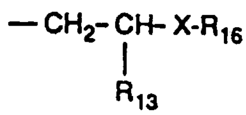

or -$CH_2C(CH_2$-O-$R_{16})_3$ or $R_6$, if $R_7$ = H, can also be -$(CH_2)_f$-X-$R_{16}$ or $R_6$ and $R_7$, together with the N bonding atom, form a 5- or 6-membered heterocyclic ring, $R_8$ and $R_9$ independently of one another are hydrogen, $C_1$-$C_{20}$alkyl, phenyl, -CO-$C_1$-$C_{20}$alkyl, -CO-$C_2$-$C_{18}$alkenyl, -CO-phenyl or -CO-naphthyl or together are =CH-$R_{17}$, $R_{10}$ is hydrogen, $C_1$-$C_{20}$alkyl, $C_2$-$C_{20}$alkenyl, $C_5$-$C_{12}$cycloalkyl, phenyl-$C_1$-$C_4$alkyl, phenyl, naphthyl, -CO-$C_1$-$C_{20}$alkyl, -CO-phenyl, -CO-naphthyl or

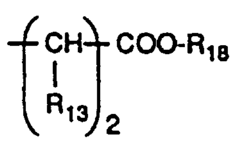

$R_{11}$ and $R_{12}$ independently of one another are hydrogen, $C_1$-$C_4$alkyl, cyclohexyl or phenyl, $R_{13}$ is hydrogen or methyl and $R_{13}'$ is hydrogen, methyl or phenyl, $R_{14}$ is $C_1$-$C_{20}$alkyl, $C_2$-$C_{20}$alkenyl, $C_5$-$C_{12}$cycloalkyl, phenyl-$C_1$-$C_4$alkyl, phenyl or naphthyl, the $R_{15}$ independently of one another are $C_1$-$C_4$alkyl, $R_{16}$ is hydrogen, -CO-$C_1$-$C_{20}$alkyl, -CO-$C_2$-$C_{20}$alkenyl, -CO-phenyl or -CO-naphthyl, $R_{17}$ is $C_1$-$C_{20}$alkyl, $C_2$-$C_{20}$alkenyl, $C_5$-$C_{12}$cycloalkyl, phenyl, naphthyl, 2-furyl,

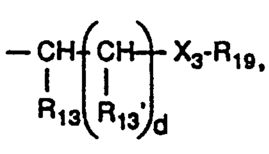 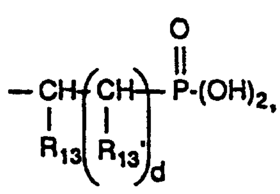 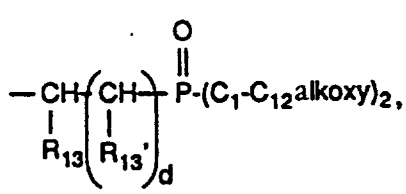

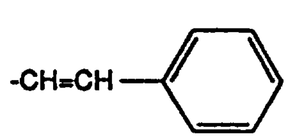, 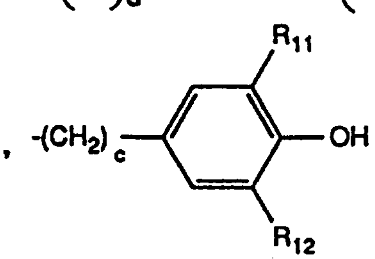 or 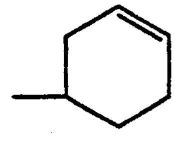,

$R_{18}$ is hydrogen, $C_1$-$C_{20}$alkyl or $C_2$-$C_{20}$alkenyl, $R_{19}$ is $C_1$-$C_{20}$alkyl, $C_5$-$C_{12}$cycloalkyl, phenyl or -$CH_2$-COO-$C_{12}$-$C_{20}$alkyl, X and a are as defined above, $X_1$ is -O-, -S-, -NH- or -$NR_{14}$-, $X_2$ is a direct bond, -$CH_2$-, $>$CH-$CH_3$ or -S- and $X_3$ is -O-, -S-, -NH- or -$NR_{19}$-, b is 3 to 5, c is 0 to 2, d is 0 or 1, e is 2 to 10 and f is 2 to 6;

or A, if n = 2, is -X'-$C_gH_{2g}$-X'-,

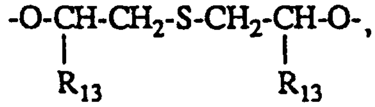,

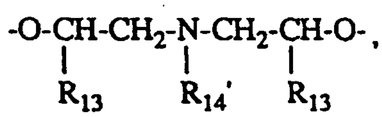, 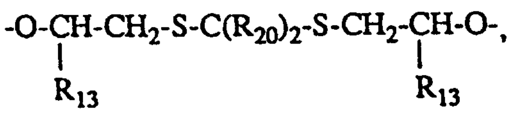,

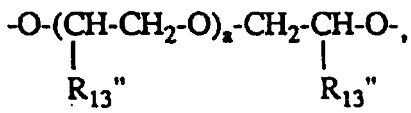,

-$OCH_2CH=CHCH_2O$-, -$OCH_2C\equiv CCH_2O$-, -O$(CH_2)_h$-NH-CO-$X_4$-OC-NH$(CH_2)_h$-O-, -NH$(CH_2)_2$-X$(CH_2)_2$-O-, -NH$(CH_2CH_2NH)_i$-$CH_2CH_2NH$-, -NH$(CH_2CH_2CH_2NH)_iCH_2CH_2CH_2NH$-,

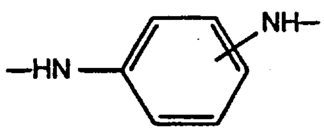, 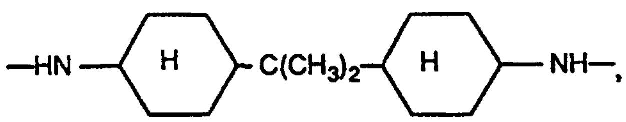,

-NH$(CH_2)_h$-O-CO-$X_4$-OC-O-$(CH_2)_h$-NH-, -NH-NH-, -NH-NH-CO-$X_4$-OC-NH-NH-, -NH-N=CH-$X_5$-CH=N-NH-, -$C_aH_{2a}$-, -$(CH_2)_m$-$X_1$-$(CH_2)_m$-, phenylene,

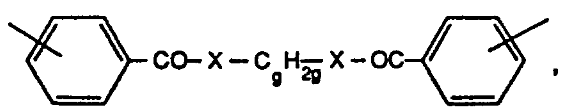,

-$C_pH_{2p}$-CO-X-$C_gH_{2g}$-X-OC-$C_pH_{2p}$-, -$(CH_2)_k$-O-CO-$X_4$-CO-O-$(CH_2)_k$-, -CH=CH-CO-X-$C_gH_{2g}$-X-OC-CH=CH-, -CH(OH)$CH_2$-, -CH(OH)CH(OH)-,

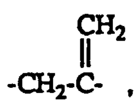,

-CH=CH-, -CH($R_{21}$)- or

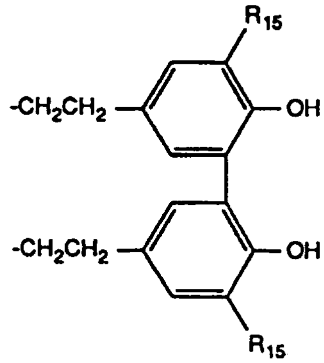

where X, $R_{13}$, $R_{15}$, a and f are as defined above, the X' independently of one another are -O- or -NH-, $R_{13}''$ is hydrogen or methyl and, if a = zero, can also be $C_2$-$C_{18}$alkyl or phenyl, $R_{14}'$ can have the same definition as $R_{14}$ or is hydrogen, the $R_{20}$ independently of one another are hydrogen, $C_1$-$C_{20}$alkyl, phenyl or together are a 5-12-membered cycloaliphatic ring, $R_{21}$ is $C_5$-$C_{12}$cycloalkyl, phenyl-$C_1$-$C_4$alkyl, phenyl or

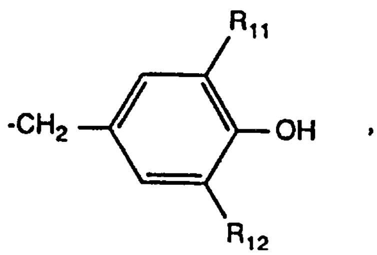

g is 2 to 12, h is 2 to 6, i is 1 to 5, k is 3 to 5, m is 1 or 2 and p is 1 to 10, $X_4$ is $-C_aH_{2a}-$, phenylene or -CH=CH- and $X_5$ is a direct bond, $-(CH_2)_3-$ or $-C(CH_3)_2-S-S-C(CH_3)_2-$;
or A, if n = 3, is

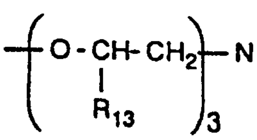, 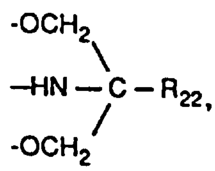, 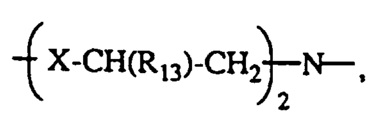,

$-(OCH_2)_3-C-R_{13}'$, $N(CH_2)_3-$ or

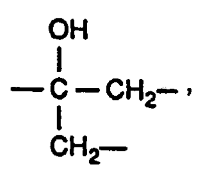

where $R_{13}$ and $R_{13}'$ are as defined above and $R_{22}$ is methyl or ethyl;
or A, if n = 4, is $C(CH_2O)_4-$,

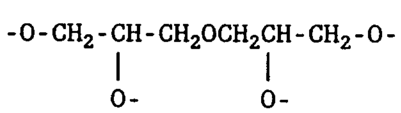

$-(OCH_2)_3$-C-NH- or $-(CH_2)_2NCH_2CH_2N(CH_2)_2$-;
or A, if n = 5, is

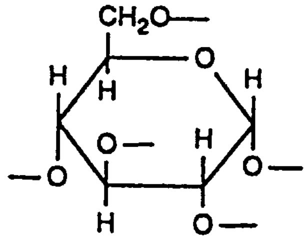

and,
if n = 6, is

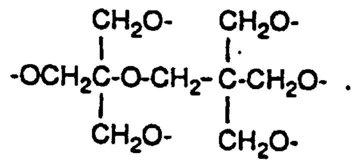

**2.** A composition according to claim 1, in which R and $R_1$ independently of one another are hydrogen, $C_1$-$C_4$alkyl, $C_1$-$C_4$alkoxy or halogen, especially chlorine, and $R_2$ and $R_3$ are hydrogen.

**3.** A composition according to claim 1, in which R is hydrogen, $C_1$-$C_4$alkyl, $C_1$-$C_4$alkoxy or chlorine, $R_1$, $R_2$, $R_3$ and $R_4$ are hydrogen, n is 1 or 2, A, if n = 1, is $-OR_5$, $-NHR_6$, $-NH-NH-R_8$, $-NH-N=CH-R_{17}$, $C_1$-$C_{17}$alkyl, $C_2$-$C_{17}$alkenyl,

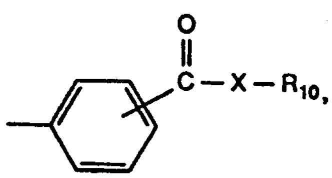

$-(CH_2)_pCO-X-R_{10}$, $-CH_2-X_1-C_1$-$C_{18}$alkyl,

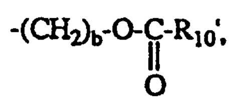

$-(CH_2)_b-OH$,

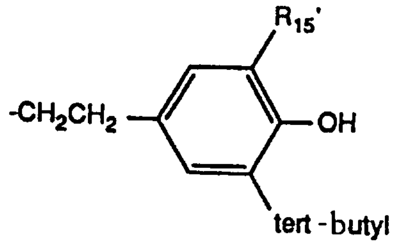

or

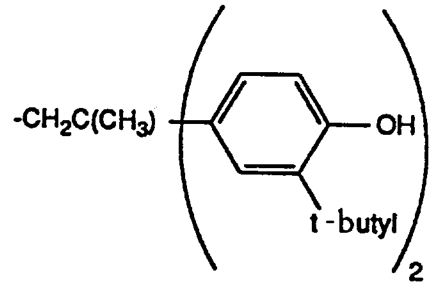

,

$R_{10}$ is hydrogen, $C_1$-$C_{18}$alkyl, $C_2$-$C_{18}$alkenyl, cyclohexyl, methylcyclohexyl or benzyl and $R_{10}'$ is $C_1$-$C_{17}$alkyl, $C_2$-$C_{17}$alkenyl or phenyl, $R_5$ is $C_1$-$C_{18}$alkyl, $C_2$-$C_{18}$alkenyl, cyclohexyl, methylcyclohexyl, benzyl,

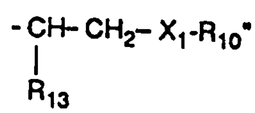

or

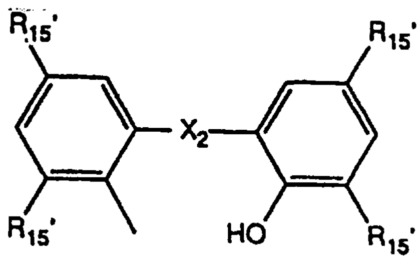

,

in which $R_{10}''$ is hydrogen, $C_1$-$C_{18}$alkyl or $C_2$-$C_{18}$alkenyl and the $R_{15}'$ independently of one another are methyl or tert-butyl, $R_6$ is $C_1$-$C_{18}$alkyl, $C_2$-$C_{18}$alkenyl, cyclohexyl, methylcyclohexyl, benzyl, $-CH_2CH_2OH$,

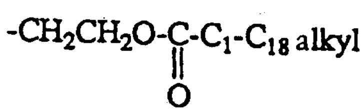

or

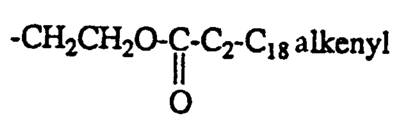

,

$R_8$ is hydrogen, $C_1$-$C_{12}$alkyl, -CO-$C_1$-$C_{18}$alkyl or -CO-$C_2$-$C_{18}$alkenyl, $R_{17}$ is $C_1$-$C_{11}$alkyl, $C_2$-$C_{11}$alkenyl, $-(CH_2)_m$-S-$C_1$-$C_{18}$alkyl or

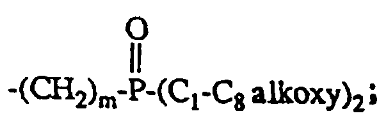

;

or A, if n = 2, is $-O-(CH_2)_g-O-$, $-NH-(CH_2)_g-NH-$,

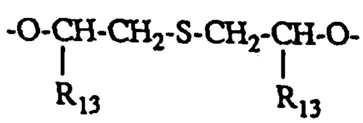

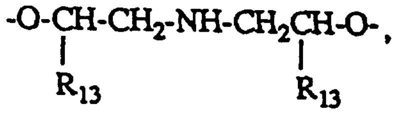

,

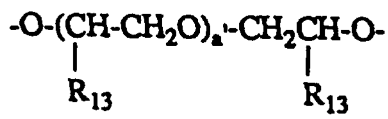

where a' is zero to 5 or $-OCH_2-C(CH_3)_2CH_2-O-$, $-NHCH_2CH_2-X-CH_2CH_2O-$, $-NH(CH_2CH_2NH)_i-$

$CH_2CH_2NH$-, -$NH(CH_2CH_2CH_2NH)_i$-$CH_2CH_2CH_2$-NH-, -NH-$C(CH_3)_2$-$CH_2O$-,

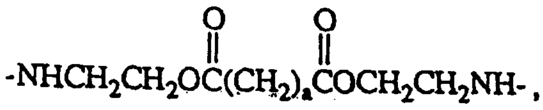, 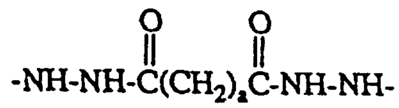,

-NH-N = CH-$X_5$-CH = N-NH-, -$(CH_2)_p$- phenylene,

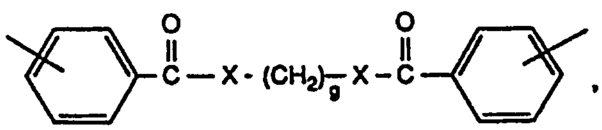, 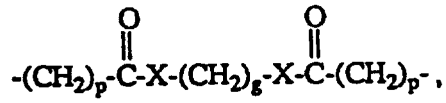,

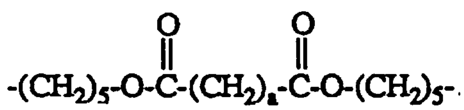.

-CH($R_{21}$)- where $R_{21}$ is benzyl, phenyl or

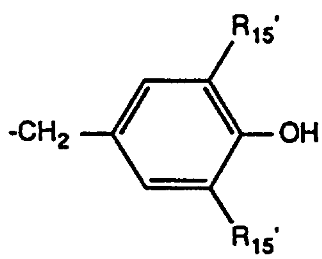 or A is 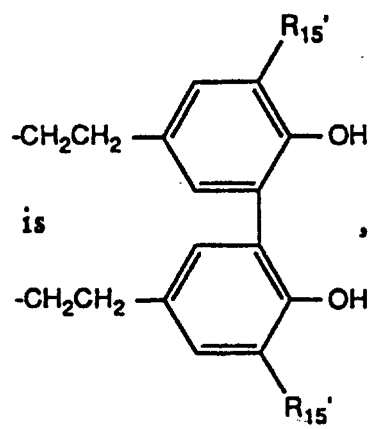,

and a, b, g, i, m, p, X, $X_1$, $X_2$, $X_5$ and $R_{13}$ are as defined in claim 1.

**4.** A composition according to claim 1, in which R is hydrogen, chlorine, methyl or methoxy and $R_1$, $R_2$, $R_3$ and $R_4$ are hydrogen, n is 1 or 2, A, if n = 1, is -$OR_5$, -$NHR_6$, -NH-NH-$R_8$, -NH-N = CH-$R_{17}$, $C_1$-$C_{17}$alkyl, -$(CH_2)_p$-COOH,

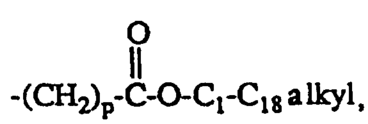,

-$(CH_2)_b$OH, -$(CH_2)_b$-OCO-$C_1$-$C_{18}$ alkyl, -$CH_2$-S-$C_1$-$C_{18}$ alkyl,

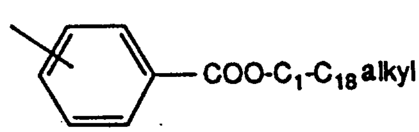 or

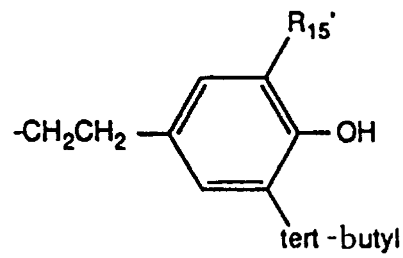

where $R_5$ is $C_1$-$C_{18}$alkyl, cyclohexyl, methylcyclohexyl, benzyl or

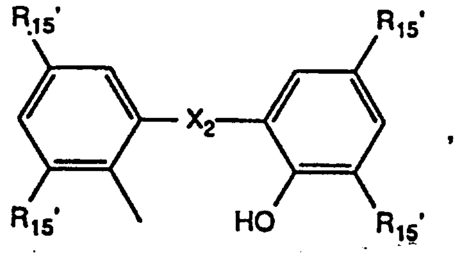

,

$R_6$ is $C_1$-$C_{18}$alkyl, cyclohexyl, benzyl, $-(CH_2)_f$-OH or $-CH_2CH_2O$-CO-$C_1$-$C_{18}$alkyl, $R_8$ is hydrogen, -CO-$C_1$-$C_{18}$alkyl or -COCH = $CH_2$ the $R_{15}$' independently of one another are methyl or tert-butyl, $R_{17}$ is $C_1$-$C_{11}$alkyl, $-(CH_2)_m$-S-$C_1$-$C_{18}$alkyl or

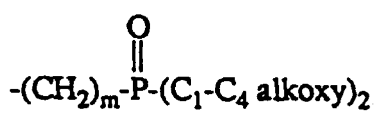

or A, if n = 2, is -O-$(CH_2)_g$-O-, -NH-$(CH_2)_g$-NH-, $-OCH_2CH_2SCH_2CH_2O$-, $-O(CH_2CH_2O)_c$-$CH_2CH_2$-O-, $OCH_2C(CH_3)_2CH_2O$-, $-NHCH_2CH_2OCH_2CH_2O$-,

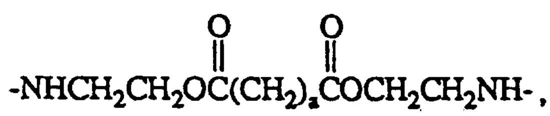

,

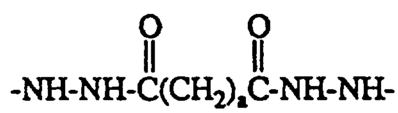

,

-NH-N = CH-$C(CH_3)_2$-S-S-$C(CH_3)_2$-CH = N-NH-, phenylene or $-(CH_2)_p$-, where a, b, c, f, g, m, p and $X_2$ are as defined in claim 1.

**5.** A composition according to claim 1, in which R is chlorine, methyl or methoxy and especially hydrogen, $R_1$, $R_2$, $R_3$ and $R_4$ are hydrogen, n is 1 or 2, A, if n = 1, is $-OR_5$, $-NHR_6$, -NH-NH-$R_8$, -NH-N = CH-$R_{17}$, $C_8$-$C_{17}$alkyl, $-(CH_2)_{p'}$-CO-O-$C_8$-$C_{18}$alkyl where p' = 2 to 6, - $(CH_2)_b$-OH, $-(CH_2)_b$-OCO-$C_8$-$C_{18}$alkyl, $-CH_2$-S-$C_8$-$C_{18}$alkyl,

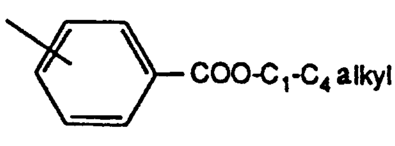

or

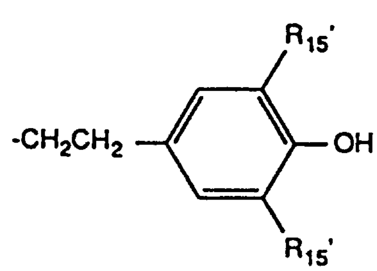

$R_5$ is

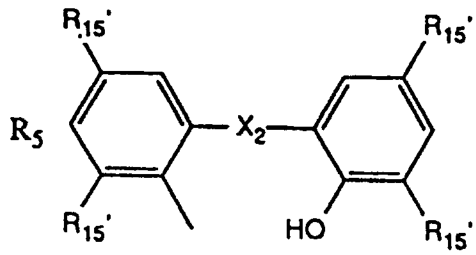

and in particular $C_8$-$C_{18}$alkyl, $R_6$ is $C_8$-$C_{18}$alkyl, $-(CH_2)_{f''}-OH$ where f" = 2 or 3 or $-CH_2CH_2OCO-C_8$-$C_{18}$alkyl, $R_8$ is $-CO-C_8$-$C_{18}$alkyl or $-COCH=CH_2$, $R_{17}$ is $C_8$-$C_{11}$alkyl, $(CH_2)_m-S-C_8$-$C_{18}$alkyl or

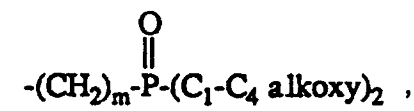

or A, if n = 2, is $-O(CH_2)_gO-$, $-NH(CH_2)_gNH-$, $-OCH_2CH_2SCH_2CH_2O-$, $-O(CH_2CH_2O)_cCH_2CH_2O-$, $-OCH_2C(CH_3)_2CH_2O-$, $-NHCH_2CH_2OCH_2CH_2O-$,$-NHCH_2CH_2OCO-(CH_2)_{a''}-OCOCH_2CH_2NH-$ or $-NH-NHCO-(CH_2)_{a''}-CONH-NH-$ where a" = 2 to 8, is $-NH-N=CH-C(CH_3)_2-S-S-C(CH_3)_2-CH=N-NH-$, $-(CH_2)_{p''}-$ where p" = 5-10 or phenylene, b is 3 to 5, especially 5, $X_2$ is a direct bond or $>CH-CH_3$ , the $R_{15}'$ independently of one another are methyl or tert-butyl and c, g and m are as defined in claim 1.

**6.** A composition according to claim 1, in which R, $R_1$, $R_2$, $R_3$ and $R_4$ are hydrogen, n is 1 or 2, A, if n = 1, is $C_8$-$C_{17}$alkyl, $-OR_5$, $-NHR_6$ or $-NH-NH-CO-C_8$-$C_{17}$alkyl, $R_5$ and $R_6$ independently of one another are $C_8$-$C_{18}$alkyl, or A, if n = 2, is $-O(CH_2)_g-O-$, $-NH(CH_2)_gNH-$, $-OCH_2C(CH_3)_2CH_2O-$, $-NH-NHCO-CH_2CH_2-OCNH-NH-$, $-(CH_2)_{p''}-$ where p" = 5 to 10 or phenylene, where g is as defined in claim 1.

**7.** A composition according to claim 1, comprising a compound of the formula Ia

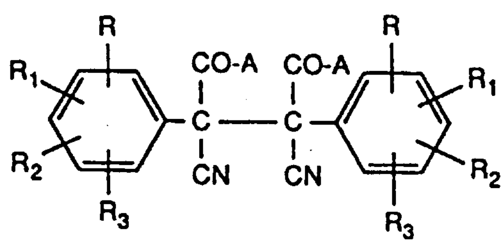

(Ia),

in which R is hydrogen, $C_1$-$C_4$alkyl, $C_1$-$C_4$alkoxy or chlorine, $R_1$, $R_2$ and $R_3$ are hydrogen and A is $-OC_1$-$C_{18}$alkyl, especially $-OC_1$-$C_4$alkyl.

**8.** A composition according to claim 1, in which the organic material is a lubricant, a metalworking fluid, a hydraulic fluid or a natural, semi-synthetic or synthetic polymer.

**9.** A composition according to claim 8, in which the organic material is a lubricant, an elastomer or a halogen-free thermoplastic, especially a halogen-free polyolefin.

**10.** A compound of the formula Ib

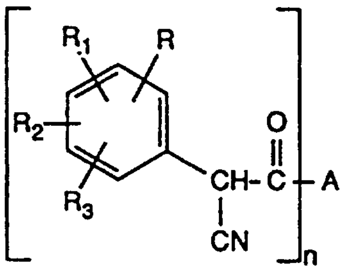

(Ib),

in which R is hydrogen, -OH, halogen, $C_1$-$C_{20}$alkyl, $C_2$-$C_{20}$alkenyl, $C_5$-$C_{12}$cycloalkyl, $C_1$-$C_4$alkyl-$C_5$-$C_{12}$cycloalkyl, phenyl, naphthyl, $C_1$-$C_{20}$alkoxy, phenyloxy, naphthyloxy, -S-$C_1$-$C_{20}$alkyl, -S-phenyl, -S-naphthyl, -O-CO-$C_1$-$C_{20}$alkyl, -O-CO-phenyl, -O-CO-naphthyl, -COOH, -COO-$C_1$-$C_{20}$alkyl, -COO-$C_2$-$C_{20}$alkenyl, -COO-phenyl, -COO-naphthyl, -CONR'R", -CO-$C_1$-$C_{20}$alkyl, -CO-phenyl, -CO-naphthyl, -NHCO-$C_1$-$C_{20}$alkyl, -NHCO-$C_2$-$C_{20}$alkenyl, -NHCO-phenyl or -NHCO-naphthyl, $R_1$ can have the same definition as R, $R_2$ is hydrogen, halogen or $C_1$-$C_4$alkyl and $R_3$ is hydrogen or halogen, or two radicals R, $R_1$, $R_2$ or $R_3$ bonded to one another in the ortho-position together form tetramethylene or -CH = CH-CH = CH-, where the remaining two of R, $R_1$, $R_2$ and $R_3$ are hydrogen, or $R_1$ to $R_3$ are hydrogen and R, if n = 1, is a group

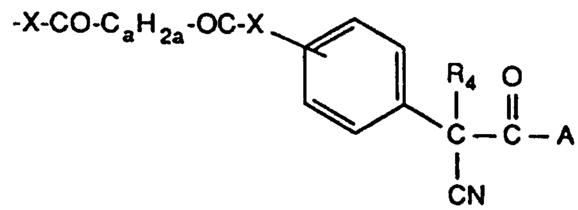

or

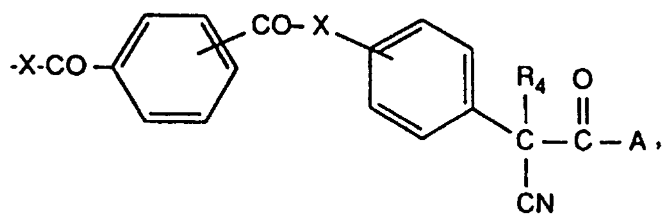

in which $R_4$ is in each case H;
R' and R" independently of one another are hydrogen, $C_1$-$C_{20}$alkyl, $C_2$-$C_{20}$alkenyl, phenyl or naphthyl or, together with the bonding N atom, form a 5- or 6-membered heterocyclic ring, a is zero to 10, n is 1 to 6, X is -O- or -NH-, A, if n = 1, is -$OR_5$, -$NR_6R_7$, -NH-$NR_8R_9$, $C_1$-$C_{30}$alkyl, $C_2$-$C_{30}$alkenyl, $C_5$-$C_{12}$cycloalkyl, $C_1$-$C_4$alkyl-$C_5$-$C_{12}$cycloalkyl,

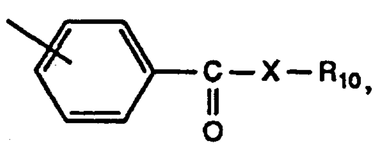

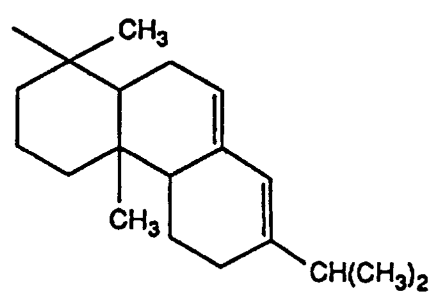

or the corresponding dihydro or tetrahydro derivative, -$C_aH_{2a}$-CO-X-$R_{10}$, -$C_bH_{2b}$-OCO-$R_{10}$, -$C_bH_{2b}$-OH,

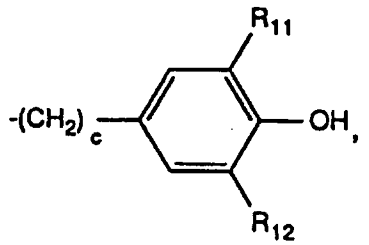

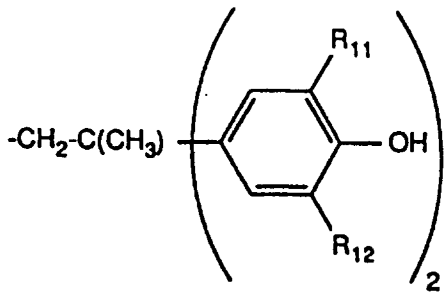,
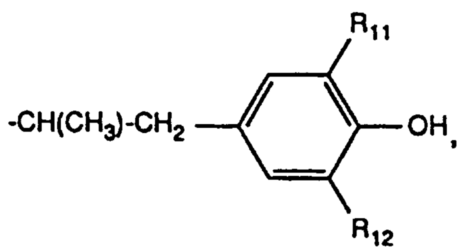

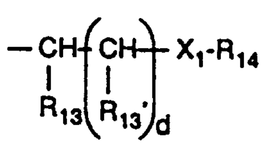 or 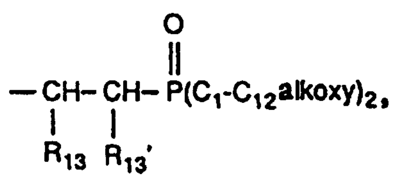

where $R_5$ is $C_1$-$C_{30}$alkyl, $C_2$-$C_{30}$alkenyl, -$C_5$-$C_{12}$cycloalkyl, $C_1$-$C_4$alkyl-$C_5$-$C_{12}$cycloalkyl, phenyl-$C_1$-$C_4$alkyl, phenyl, naphthyl, biphenyl, the radical of a terpene alcohol or a radical of the formula

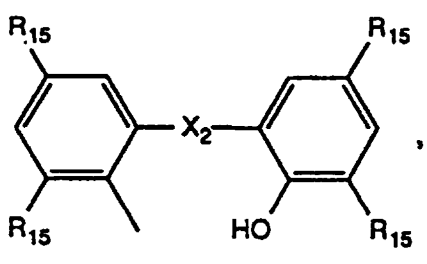,
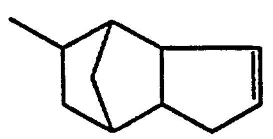,
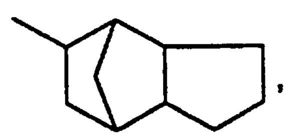,

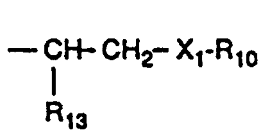 or 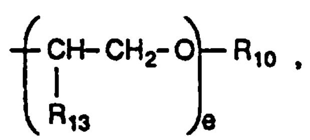

$R_6$ and $R_7$ independently of one another are hydrogen, $C_1$-$C_{20}$alkyl, $C_2$-$C_{20}$alkenyl, $C_5$-$C_{12}$cycloalkyl, $C_1$-$C_4$alkyl-$C_5$-$C_{12}$cycloalkyl, phenyl-$C_1$-$C_4$alkyl, phenyl, naphthyl,

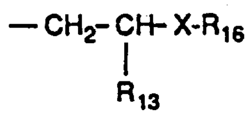

or -$CH_2C(CH_2$-O-$R_{16})_3$ or $R_6$, if $R_7$ = H, can also be -$(CH_2)_f$-X-$R_{16}$ or $R_6$ and $R_7$, together with the N-bonding atom, form a 5- or 6-membered heterocyclic ring, $R_8$ and $R_9$ independently of one another are

hydrogen, $C_1$-$C_{20}$alkyl, phenyl, -CO-$C_1$-$C_{20}$alkyl, -CO-$C_2$-$C_{18}$alkenyl, -CO-phenyl or -CO-naphthyl or together are =CH-$R_{17}$, $R_{10}$ is hydrogen, $C_1$-$C_{20}$alkyl, $C_2$-$C_{20}$alkenyl, $C_5$-$C_{12}$cycloalkyl, phenyl-$C_1$-$C_4$alkyl, phenyl, naphthyl, -CO-$C_1$-$C_{20}$alkyl, -CO-phenyl, -CO-naphthyl or

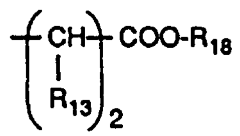,

$R_{11}$ and $R_{12}$ independently of one another are hydrogen, $C_1$-$C_4$alkyl, cyclohexyl or phenyl, $R_{13}$ is hydrogen or methyl and $R_{13}$' is hydrogen, methyl or phenyl, $R_{14}$ is $C_1$-$C_{20}$alkyl, $C_2$-$C_{20}$alkenyl, $C_5$-$C_{12}$cycloalkyl, phenyl-$C_1$-$C_4$alkyl, phenyl or naphthyl, the $R_{15}$ independently of one another are $C_1$-$C_4$alkyl, $R_{16}$ is hydrogen, -CO-$C_1$-$C_{20}$alkyl, -CO-$C_2$-$C_{20}$alkenyl, -CO-phenyl or -CO-naphthyl, $R_{17}$ is $C_1$-$C_{20}$alkyl, $C_2$-$C_{20}$alkenyl, $C_5$-$C_{12}$cycloalkyl, phenyl, naphthyl, 2-furyl,

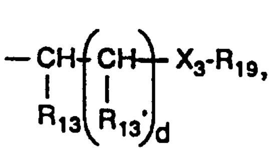, 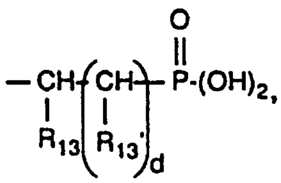, 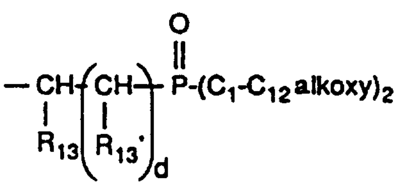

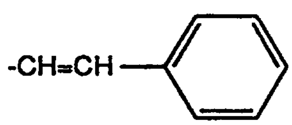, 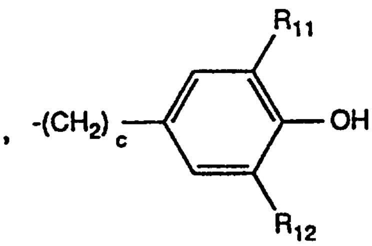 or 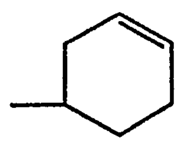,

$R_{18}$ is hydrogen, $C_1$-$C_{20}$alkyl or $C_2$-$C_{20}$alkenyl, $R_{19}$ is $C_1$-$C_{20}$alkyl, $C_5$-$C_{12}$cycloalkyl, phenyl or -$CH_2$-COO-$C_{12}$-$C_{20}$alkyl, X and a are as defined above, $X_1$ is -O-, -S-, -NH- or -$NR_{14}$-, $X_2$ is a direct bond, -$CH_2$-, $>$CH-$CH_3$ or -S- and $X_3$ is -O-, -S-, -NH- or -$NR_{19}$-, b is 3 to 5, c is 0 to 2, d is 0 or 1, e is 2 to 10 and f is 2 to 6;
with the proviso that A is not -O$C_1$-$C_{12}$alkyl, -$NH_2$, -NH-methyl, -NH-cyclohexyl, -NH-phenyl,

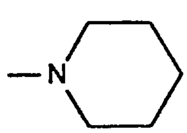,

-NH-$NH_2$, $C_1$-$C_7$alkyl, -$CH_2N(CH_3)_2$, -$CH_2$N(methyl)-(phenyl) or -$C_{a''}H_{2a''}$-CO-O-$R_{10'}$ where a'' = 2 or 4 and $R_{10'}$ = hydrogen, methyl or ethyl, if one of the radicals R, $R_1$, $R_2$ or $R_3$ is hydrogen, chlorine, methyl or methoxy and the other radicals R, $R_1$, $R_2$ or $R_3$ are hydrogen; and that compounds of the formula Ib are excluded in which R, $R_1$, $R_2$ and $R_3$ = H and A = $C_1$-$C_{24}$alkyl, -O-$C_1$-$C_{24}$alkyl, -$NH_2$, -NH-$C_1$-$C_{20}$alkyl, or -N($C_1$-$C_{20}$alkyl)$_2$, piperidino, morpholino, pyrrolidino, dimethylamino and diethylamino, and also compounds in which $R_{16}$ = hydrogen and f = 2, and that the compounds of the formulae

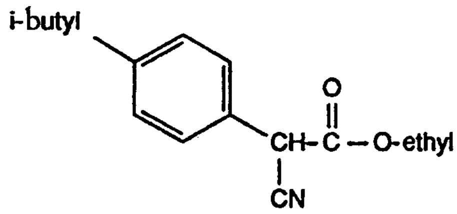
,
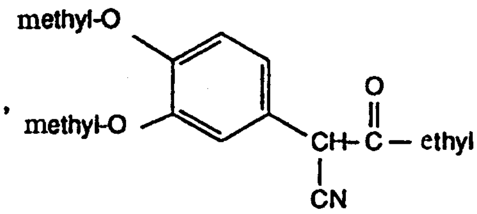
,

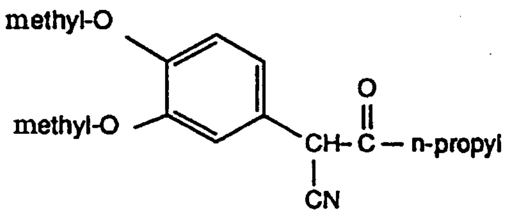
,
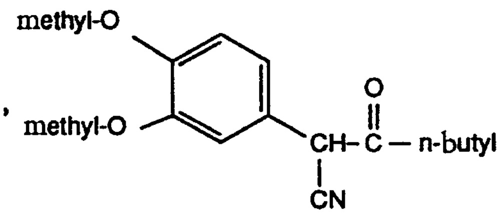
,

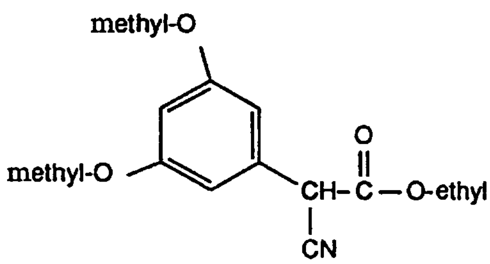

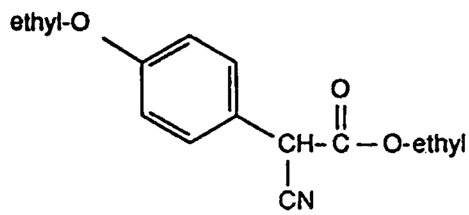
,

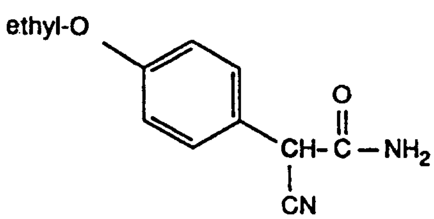

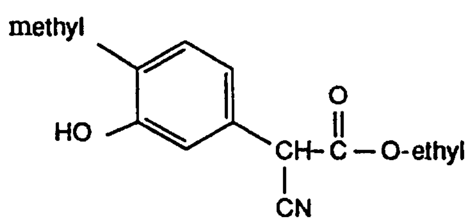
,

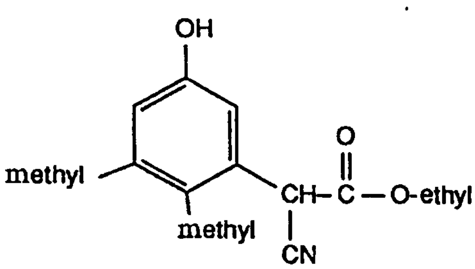

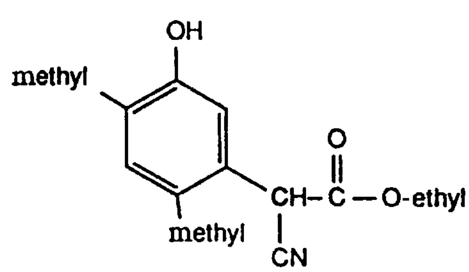
,

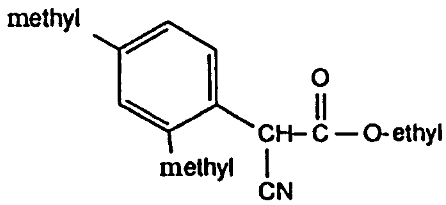 , 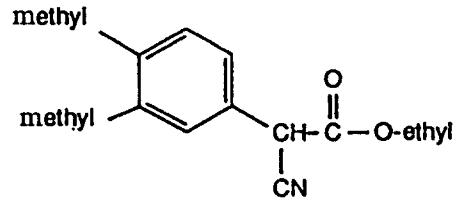 ,

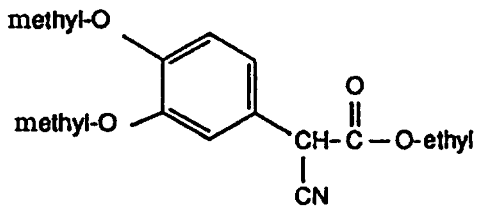 , 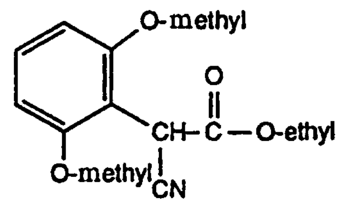 ,

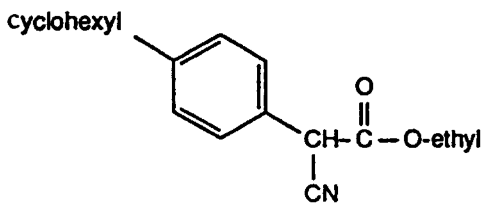 , 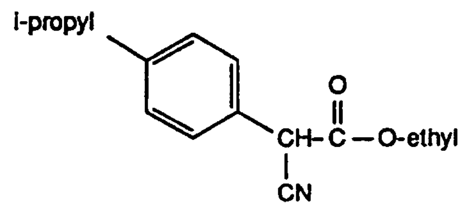 ,

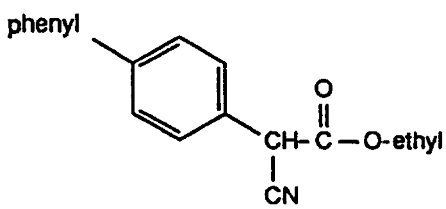 , 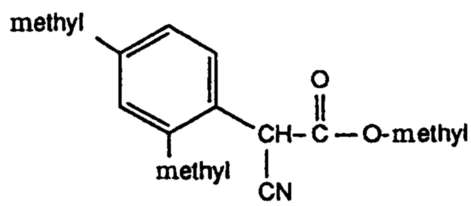 ,

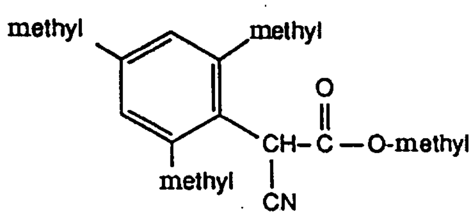 , 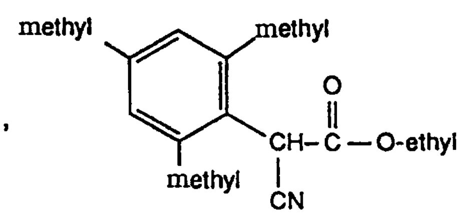 and

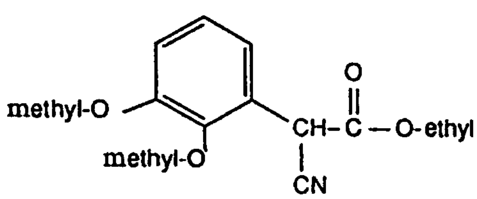 .

are likewise excluded;
or A, if n = 2, is -X'-$C_gH_{2g}$-X'-,

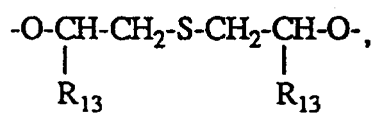

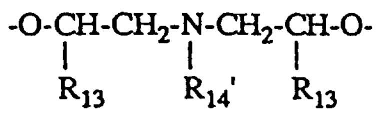, 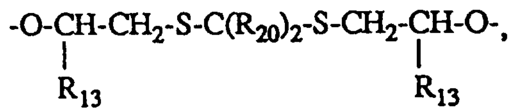

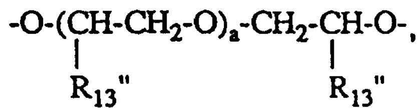

$-OCH_2CH=CHCH_2O-$, $-OCH_2C\equiv CCH_2O-$, $-OCH_2CH=CHCH_2O-$, $-OCH_2C\equiv CCH_2O$, $-O(CH_2)_h-NH-CO-X_4-OC-NH(CH_2)_h-O-$, $-NH(CH_2)_2-X(CH_2)_2-O-$, $-NH(CH_2CH_2NH)_i-CH_2CH_2NH-$, $-NH(CH_2CH_2CH_2NH)-{}_iCH_2CH_2CH_2NH-$,

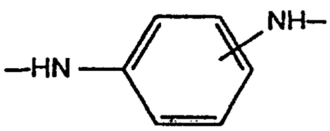, 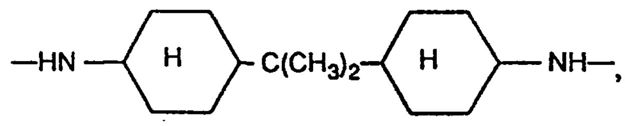

$-NH(CH_2)_h-O-CO-X_4-OC-O-(CH_2)_h-NH-$, $-NH-NH-$, $-NH-NH-CO-X_4-OC-NH-NH-$, $-NH-N=CH-X_5-CH=N-NH-$, $C_pH_{2p}-$, $-(CH_2)_m-X_1-(CH_2)_m-$,

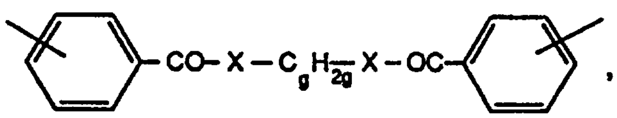,

$-C_pH_{2p}-CO-X-C_gH_{2g}-X-OC-C_pH_{2p}-$, $-(CH_2)_k-O-CO-X_4-CO-O-(CH_2)_k-$, $-CH=CH-CO-X-C_gH_{2g}-X-OC-CH=CH-$, $-CH(OH)CH_2-$, $-CH(OH)CH(OH)-$,

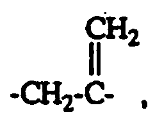,

$-CH=CH-$, $-CH(R_{21})-$ or

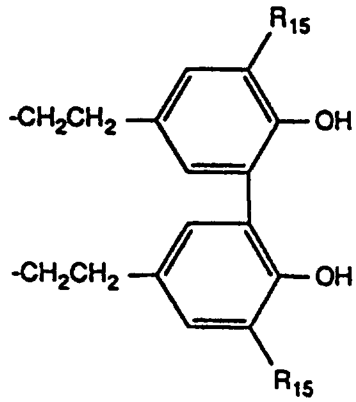

where X, $R_{13}$, $R_{15}$, a and f are as defined above, the X' independently of one another are -O- or -NH-, $R_{13}$'' is hydrogen or methyl and, if a = zero, can also be $C_2$-$C_{18}$alkyl or phenyl, $R_{14}$' can have the same definition as $R_{14}$ or is hydrogen, the $R_{20}$ independently of one another are hydrogen, $C_1$-$C_{20}$alkyl, phenyl or together are a 5-12-membered cycloaliphatic ring, $R_{21}$ is $C_5$-$C_{12}$cycloalkyl, phenyl-$C_1$-$C_4$alkyl, phenyl or

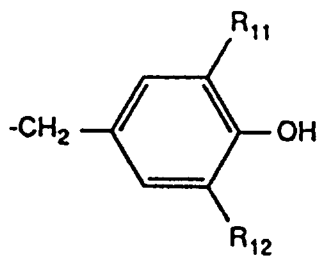 ,

g is 2 to 12, h is 2 to 6, i is 1 to 5, k is 3 to 5, m is 1 or 2 and p is 1 to 10, $X_4$, is $-C_aH_{2a}-$, phenylene or -CH=CH-and $X_5$ is a direct bond, $-(CH_2)_3-$ or $-C(CH_3)_2-S-S-C(CH_3)_2-$;
or A, if n = 3, is

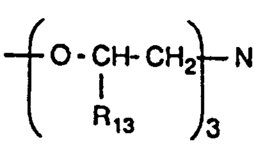 , 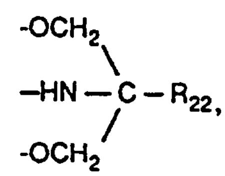 , 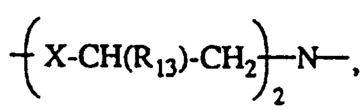 ,

$-(OCH_2)_3-C-R_{13}'$, $N(CH_2)_3-$ or

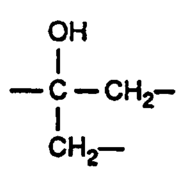 ,

where $R_{13}$ and $R_{13}$' are as defined above and $R_{22}$ is methyl or ethyl;
or A, if n = 4, is $C(CH_2O)_4-$,

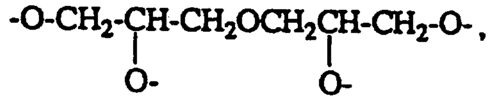

$-(OCH_2)_3$-C-NH- or $-(CH_2)_2NCH_2CH_2N(CH_2)_2-$;
or A, if n = 5, is

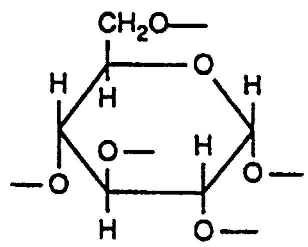

and,
if n = 6, is

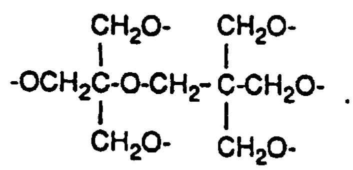

**11.** A compound according to claim 10, in which R and $R_1$ independently of one another are hydrogen, $C_1$-$C_4$alkyl, $C_1$-$C_4$alkoxy or halogen, especially chlorine, and $R_2$ and $R_3$ are hydrogen.

**12.** A compound according to claim 10, in which R is hydrogen, $C_1$-$C_4$alkyl, $C_1$-$C_4$alkoxy or chlorine, $R_1$, $R_2$ and $R_3$ are hydrogen, n is 1 or 2, A, if n = 1, is $-OR_5$, $-NHR_6$, $-NH-NH-R_8$, $-NH-N=CH-R_{17}$, $C_1$-$C_{17}$alkyl, $C_2$-$C_{17}$alkenyl,

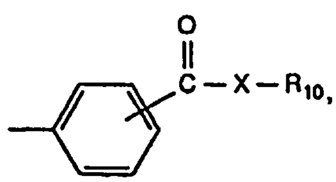

$-(CH_2)_pCO-X-R_{10}$, $-CH_2-X_1-C_1-C_{18}$ alkyl,

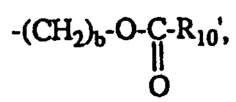

$-(CH_2)_b-OH$,

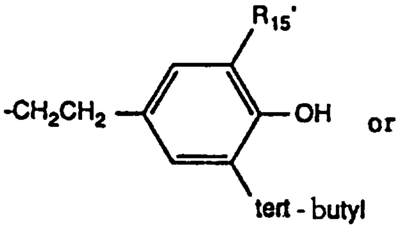 or 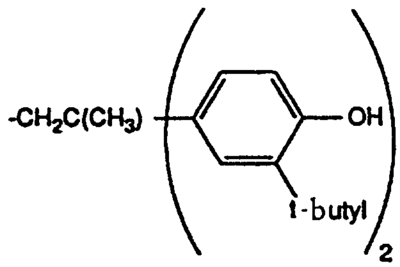,

$R_{10}$ is hydrogen, $C_1$-$C_{18}$alkyl, $C_2$-$C_{18}$alkenyl, cyclohexyl, methylcyclohexyl or benzyl and $R_{10}$' is $C_1$-$C_{17}$alkyl, $C_2$-$C_{17}$alkenyl or phenyl, $R_5$ is $C_1$-$C_{18}$alkyl, $C_2$-$C_{18}$alkenyl, cyclohexyl, methylcyclohexyl, benzyl,

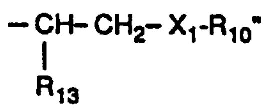 or 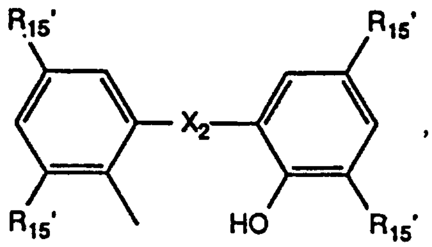,

in which $R_{10}$" is hydrogen, $C_1$-$C_{18}$alkyl or $C_2$-$C_{18}$alkenyl and the $R_{15}$' independently of one another are methyl or tert-butyl, $R_6$ is $C_1$-$C_{18}$alkyl, $C_2$-$C_{18}$alkenyl, methylcyclohexyl, benzyl, $-CH_2CH_2OH$,

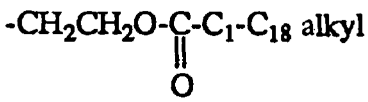 or 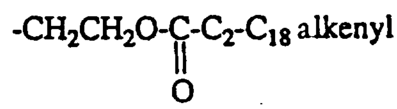,

$R_8$ is hydrogen, $C_1$-$C_{12}$alkyl, -CO-$C_1$-$C_{18}$alkyl or -CO-$C_2$-$C_{18}$alkenyl, $R_{17}$ is $C_1$-$C_{11}$alkyl, $C_2$-$C_{11}$alkenyl, $-(CH_2)_m$-S-$C_1$-$C_{18}$alkyl or

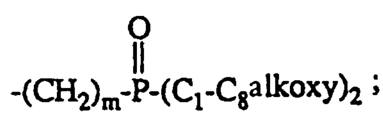;

or A, if n = 2, is $-O-(CH_2)_g-O-$, $-NH-(CH_2)_g-NH-$,

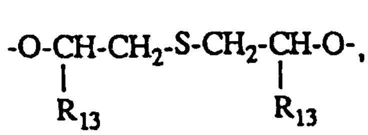, 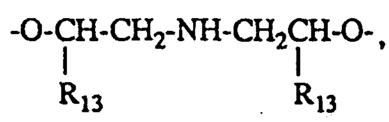, 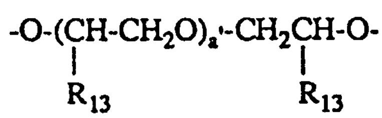

where a' = zero to 5, or $-OCH_2-C(CH_3)_2CH_2-O-$, $-NHCH_2CH_2-X-CH_2CH_2O-$, $-NH(CH_2CH_2NH)_i-CH_2CH_2NH-$, $-NH(CH_2CH_2CH_2NH)_i-CH_2CH_2CH_2-NH-$, $-NH-C(CH_3)_2-CH_2O-$,

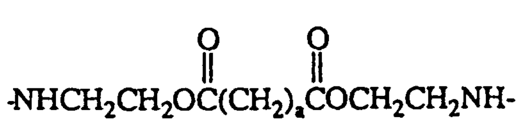, 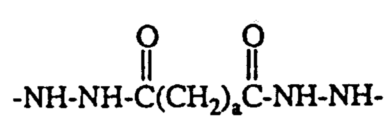,

$-NH-N=CH-X_5-CH=N-NH-$, $-(CH_2)_p-$,

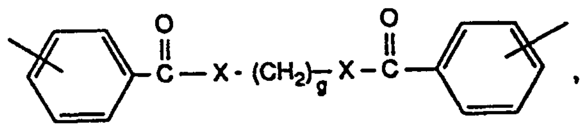,

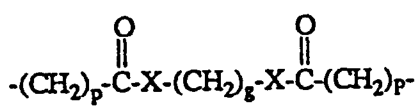, 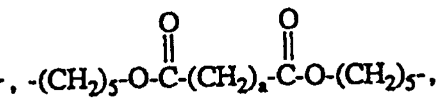,

$-CH(R_{21})-$where $R_{21}$ = benzyl, phenyl or

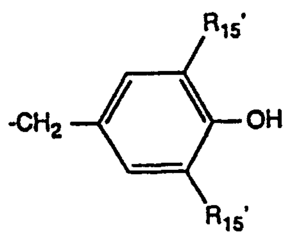 or A is 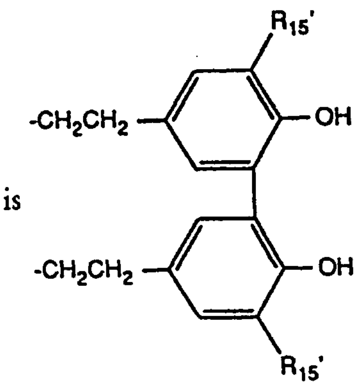,

and a, b, g, i, m, p, X, $X_1$, $X_2$, $X_5$ and $R_{13}$ are as defined in claim 1.

**13.** A compound according to claim 10, in which R is hydrogen, chlorine, methyl or methoxy and $R_1$, $R_2$ and $R_3$ are hydrogen, n is 1 or 2, A, if n = 1, is $-OR_5$, $-NHR_6$, $-NH-NH-R_8$, $-NH-N=CH-R_{17}$, $C_8$-$C_{17}$alkyl, $-(CH_2)_p-COOH$,

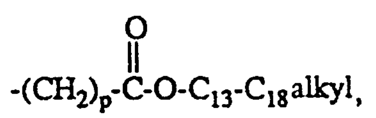,

$-(CH_2)_bOH$, $-(CH_2)_b-OCO-C_1-C_{18}$alkyl, $-CH_2-S-C_1-C_{18}$alkyl,

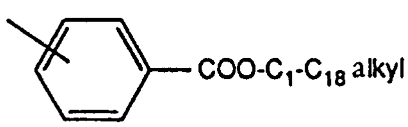 or

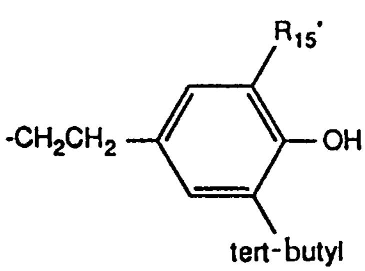

where $R_5$ is $C_{13}$-$C_{18}$alkyl, cyclohexyl, methylcyclohexyl, benzyl or

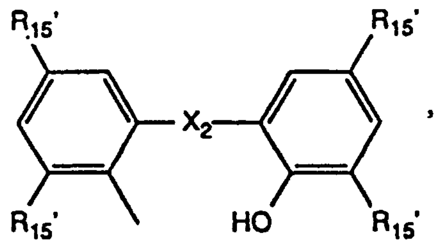

$R_6$ is $C_2$-$C_{18}$alkyl, benzyl, $-(CH_2)_f$-OH or $-CH_2CH_2O$-CO-$C_1$-$C_{18}$alkyl, $R_8$ is -CO-$C_1$-$C_{18}$alkyl or $-COCH=CH_2$, the $R_{15}'$ independently of one another are methyl or tert-butyl, $R_{17}$ is $C_1$-$C_{11}$alkyl, $-(CH_2)_m$-S-$C_1$-$C_{18}$alkyl or

$$-(CH_2)_m-\overset{O}{\overset{\|}{P}}-(C_1\text{-}C_4\text{alkoxy})_2$$

or A, if n = 2, is $-O-(CH_2)_g-O-$, $-NH-(CH_2)_g-NH-$, $-OCH_2CH_2SCH_2CH_2O-$, $-O(CH_2CH_2O)_c-CH_2CH_2-O-$, $-OCH_2C(CH_3)_2CH_2O-$, $-NHCH_2CH_2OCH_2CH_2O-$,

$$-NHCH_2CH_2O\overset{O}{\overset{\|}{C}}(CH_2)_a\overset{O}{\overset{\|}{C}}OCH_2CH_2NH-,\ -NH-NH-\overset{O}{\overset{\|}{C}}(CH_2)_a\overset{O}{\overset{\|}{C}}-NH-NH-,$$

$-NH-N=CH-C(CH_3)_2-S-S-C(CH_3)_2-CH=N-NH-$ or $-(CH_2)_p-$, where a, b, c, f, g, m, p and $X_2$ are as defined in claim 10.

**14.** A compound according to claim 10, in which R is chlorine, methyl or methoxy and especially hydrogen, $R_1$, $R_2$ and $R_3$ are hydrogen, n is 1 or 2, A, if n = 1, is $-OR_5$, $-NHR_6$, $-NH-NH-R_8$, $-NH-N=CH-R_{17}$, $C_8$-$C_{17}$alkyl, $-(CH_2)_{p'}$-CO-O-$C_8$-$C_{18}$alkyl where p' = 2 to 6, $-(CH_2)_b$-OH, $-(CH_2)_b$-OCO-$C_8$-$C_{18}$alkyl, $-CH_2$-S-$C_8$-$C_{18}$alkyl,

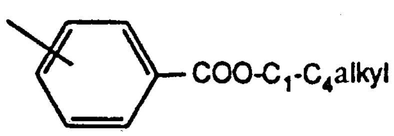 or 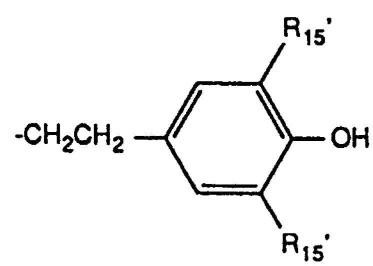 ,

$R_5$ is

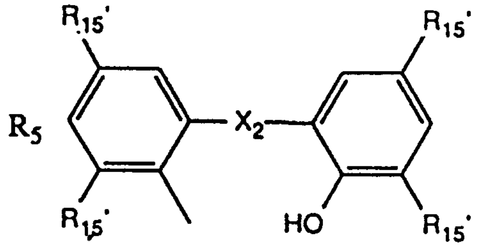

and in particular $C_{13}$-$C_{18}$alkyl, $R_6$ is $C_8$-$C_{18}$alkyl, - $(CH_2)_{f''}$-OH where f" = 2 or 3 or -$CH_2CH_2OCO$-$C_8$-$C_{18}$alkyl, $R_8$ is -CO-$C_8$-$C_{18}$alkyl or -COCH=$CH_2$, $R_{17}$ is $C_8$-$C_{11}$alkyl, $(CH_2)_m$-S-$C_8$-$C_{18}$alkyl or

$$-(CH_2)_m-\overset{\overset{\displaystyle O}{\|}}{P}-(C_1\text{-}C_4\,\text{alkoxy})_2 \quad ,$$

or A, if n = 2, is -$O(CH_2)_gO$-, -$NH(CH_2)_gNH$-, -$OCH_2CH_2SCH_2CH_2O$-, -$O(CH_2CH_2O)_cCH_2CH_2O$-, -$OCH_2C(CH_3)_2CH_2O$-, -$NHCH_2CH_2OCH_2CH_2O$-, -$NHCH_2CH_2OCO$-$(CH_2)_{a''}$-$OCOCH_2CH_2NH$- or -NH-NHCO-$(CH_2)_{a''}$-CONH-NH- where a" = 2 to 8, -NH-N=CH-C$(CH_3)_2$-S-S-C$(CH_3)_2$-CH=N-NH- or -$(CH_2)_{p''}$- where p" = 5-10, b is 3 to 5, $X_2$ is a direct bond or >CH-$CH_3$, the $R_{15}$' independently of one another are methyl or tert-butyl and c, g and m are as defined in claim 10.

**15.** A compound according to claim 10, in which R, $R_1$, $R_2$ and $R_3$ are hydrogen, n is 1 or 2, A, if n = 1, is $C_8$-$C_{17}$alkyl, -$OR_5$, -$NHR_6$ or -NH-NH-CO-$C_8$-$C_{17}$alkyl, $R_5$ is $C_{13}$-$C_{18}$alkyl and $R_6$ is $C_8$-$C_{18}$alkyl, or A, if n = 2, is -$O(CH_2)_g$-O-, -$NH(CH_2)_gNH$-, -$OCH_2C(CH_3)_2CH_2O$-, -NH-NHCO-$CH_2CH_2$-OCNH-NH- or -$(CH_2)_{p''}$- where p" = 5 to 10, where g is as defined in claim 10.

**16.** Use of compounds of the formula I according to claim 1 or of compounds of the formula Ib according to claim 10 for stabilising lubricants, metalworking fluids, hydraulic fluids or natural, semi-synthetic or synthetic polymers against thermal, oxidative and/or actinic degradation.

**17.** A method for stabilising lubricants, metalworking fluids, hydraulic fluids or natural, semi-synthetic or synthetic polymers, wherein compounds of the formula I according to claim 1 or compounds of the formula Ib according to claim 10 are added to the organic material or applied thereto as stabilisers.

## Revendications

**1.** Composition contenant des lubrifiants, des fuides de traitement de métaux, des fluides hydrauliques ou des polymères naturels, semi-synthétiques ou synthétiques, susceptibles de dégradation thermique, oxydative et/ou acténique et au moins un composé de formule I

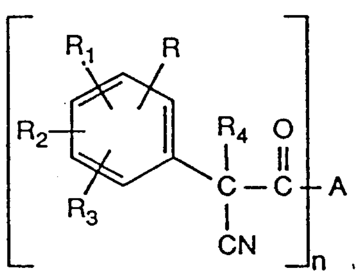

(I),

dans laquelle R représente hydrogène, -OH, halogène, alkyle en $C_1$-$C_{20}$, alcényle en $C_2$-$C_{20}$, cycloakyle en $C_5$-$C_{12}$, (alkyl en $C_1$-$C_4$)-(cycloalkyle en $C_5$-$C_{12}$), phényle, naphtyle, alcoxy en $C_1$-$C_{20}$, phényloxy, naphtyloxy, -S-(alkyle en $C_1$-$C_{20}$), -S-phényle, -S-naphtyle, -O-CO-(alkyle en $C_1$-$C_{20}$),-O-CO-phényle, -O-CO-naphtyle, -COOH, -COO-(alkyle en $C_1$-$C_{20}$), -COO-(alcényle en $C_2$-$C_{20}$), -COO-phényle, -COO-

naphtyle, -CONR'R'', -CO-(alkyle en $C_1$-$C_{20}$), -CO-phényle, -CO-naphtyle, -NHCO-(alkyle en $C_1$-$C_{20}$)-,-NHCO-(alcényle en $C_2$-$C_{20}$), -NHCO-phényle ou -NHCO-naphtyle,
$R_1$ peut avoir la même signification que R,
$R_2$ est hydrogène, halogène ou alkyle en C1-C4, et
$R_3$ représente hydrogène ou halogène,
ou deux radicaux, R, $R_1$, $R_2$ ou $R_3$, liés en position ortho l'un par rapport à l'autre, forment ensemble un tétraméthylène ou -CH=CH-CH=CH-, les deux restants de R, $R_1$, $R_2$ et $R_3$ sont hydrogène,
ou $R_1$ à $R_3$ sont hydrogène et R pour n = 1 représente un groupement

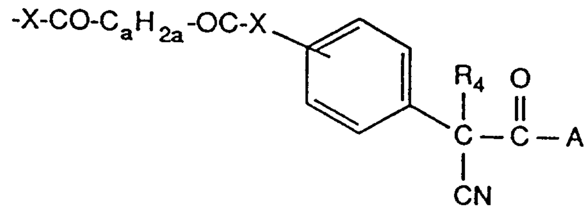

ou

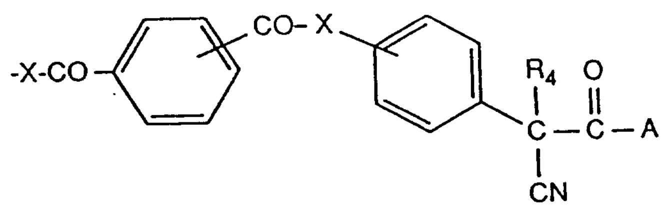

R' et R'', indépendamment l'un de l'autre, sont hydrogène, alkyle en $C_1$-$C_{20}$, alcényle en $C_2$-$C_{20}$, phényle ou naphtyle ou ensemble avec l'atome de liaison N forment un cycle hétérocyclique à 5 ou 6 chaînons,
a va de 0 à 10, n va de 1 à 6,
X représente -O- ou -NH-,
$R_4$ représente hydrogène ou pour n = 1 un groupement

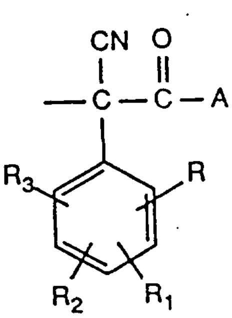

A pour n = 1, représente -OH, $-OR_5$, $-NR_6R_7$, $NH-NR_8R_9$, alkyle en $C_1$-$C_{30}$, alcényle en $C_2$-$C_{30}$, cycloalkyle en $C_5$-$C_{12}$, (alkyl en $C_1$-$C_4$)-(cycloalkyle en $C_5$-$C_{12}$)

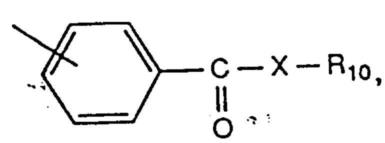

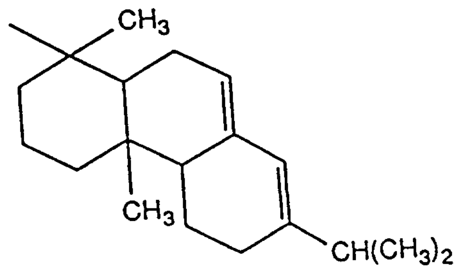

ou le dérivé dihydro ou tétrahydro correspondant,
$-C_aH_{2a}-CO-X-R_{10}$, $-C_bH_{2b}-OCO-R_{10}$, $-C_bH_{2b}-OH$,

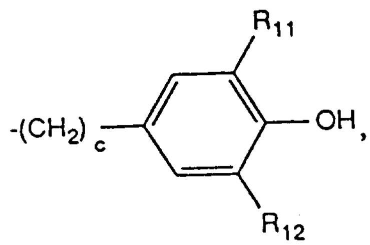

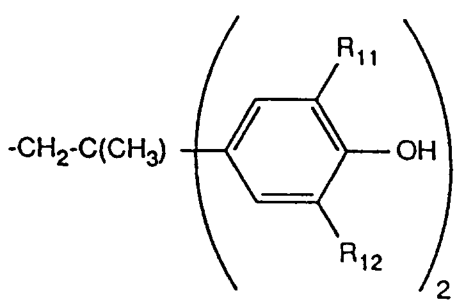
,
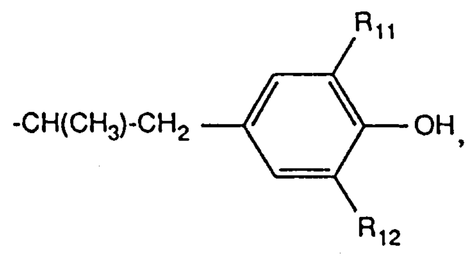

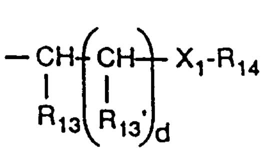
ou
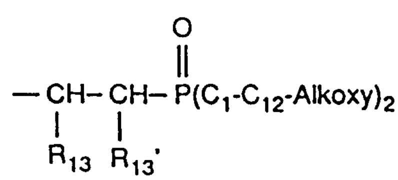

$R_5$ représente alkyle en $C_1$-$C_{30}$, alcényle en $C_2$-$C_{30}$, cycloalkyle en $C_5$-$C_{12}$, (alkyl en $C_1$-$C_4$)-(cycloalkyle en $C_5$-$C_{12}$), phénylalkyle en $C_1$-$C_4$, phényle, naphtyle, biphényle, le radical d'un alcool terpénique ou un radical de formules

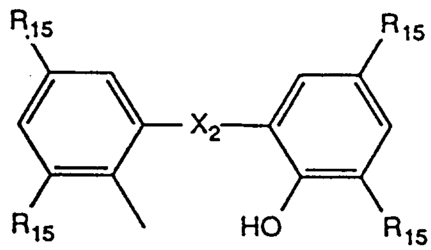 , 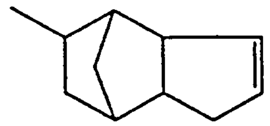 , 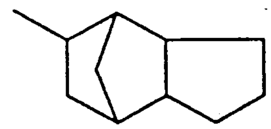 ,

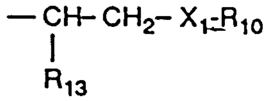 ou 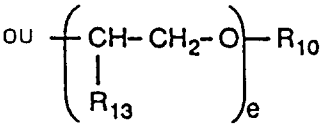

$R_6$ et $R_7$, indépendamment l'un de l'autre, représentent hydrogène, alkyle en $C_1$-$C_{20}$, alcényle en $C_2$-$C_{20}$, cycloalkyle en $C_5$-$C_{12}$, (alkyl en $C_1$-$C_4$)-(cycloalkyle en $C_5$-$C_{12}$), phénylalkyle en $C_1$-$C_4$, phényle, naphtyle,

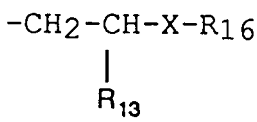

ou $-CH_2C(CH_2\text{-}O\text{-}R_{16})_3$ ou $R_6$ pour $R_7$ = H peut aussi être $-(CH_2)_f\text{-}X\text{-}R_{16}$ ou $R_6$ et $R_7$ ensemble avec l'atome de liaison N forment un cycle hétérocyclique à 5 ou 6 chaînons,

$R_8$ et $R_9$, indépendamment l'un de l'autre, représentent hydrogène, alkyle en $C_1$-$C_{20}$, phényle, -CO-(alkyle en $C_1$-$C_{20}$), -CO- (alcényle en $C_2$-$C_{18}$), -CO-phényle, ou -CO-naphtyle ou ensemble représentent $=CH\text{-}R_{17}$,

$R_{10}$ représente hydrogène, alkyle en $C_1$-$C_{20}$, alcényle en $C_2$-$C_{20}$, cycloalkyle en $C_5$-$C_{12}$, phénylalkyle en $C_1$-$C_4$, phényle, naphtyle, -CO-(alkyle en $C_1$-$C_{20}$), -CO-phényle, -CO-naphtyle ou

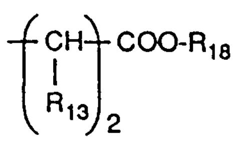

$R_{11}$ et $R_{12}$, indépendamment l'un de l'autre, représentent hydrogène, alkyle en $C_1$-$C_4$, cyclohexyle ou phényle.

$R_{13}$ représente hydrogène ou méthyle, $R_{13}'$ représente hydrogène, méthyle ou phényle,

$R_{14}$ représente alkyle en $C_1$-$C_{20}$, alcényle en $C_2$-$C_{20}$, cycloalkyle en $C_5$-$C_{12}$, phénylalkyle en $C_1$-$C_4$, phényle ou naphtyle,

les radicaux $R_{15}$, indépendamment l'un de l'autre, représentent alkyle en $C_1$-$C_4$,

$R_{16}$ représente hydrogène, -CO-(alkyle en $C_1$-$C_{20}$), -CO-(alcényle en $C_2$-$C_{20}$), -CO-phényle ou -CO-naphtyle,

$R_{17}$ représente alkyle en $C_1$-$C_{20}$, alcényle en $C_2$-$C_{20}$, cycloalkyle en $C_5$-$C_{12}$, phényle, naphtyle, 2-furyle,

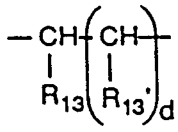, 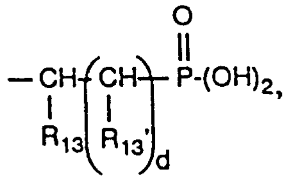, 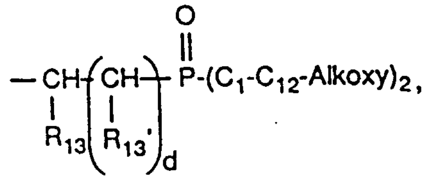,

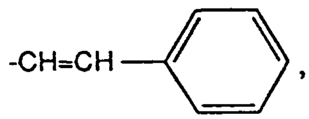, 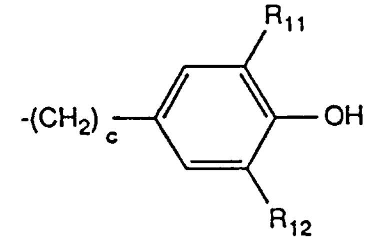 ou 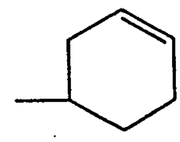

$R_{18}$ représente hydrogène, alkyle en $C_1$-$C_{20}$ ou alcényle en $C_2$-$C_{20}$,
$R_{19}$ représente alkyle en $C_1$-$C_{20}$, cycloalkyle en $C_5$-$C_{12}$, phényle ou -$CH_2$-COO-(alkyle en $C_{12}$-$C_{20}$),
X et a ont la signification donnée ci-dessus,
$X_1$ représente -O-, -S-, -NH- ou -$NR_{14}$-,
$X_2$ représente la liaison directe, -$CH_2$-, $>$CH-$CH_3$ ou -S- et
$X_3$ représente -O-, -S-, -NH-, ou -$NR_{19}$-
b va de 3 à 5,
c va de 0 à 2,
d vaut 0 ou 1,
e va de 2 à 10 et
f va de 2 à 6,
ou A pour n = 2 représente
-X'-$C_gH_{2g}$-X'-,

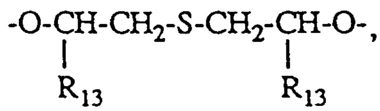,

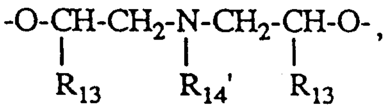, 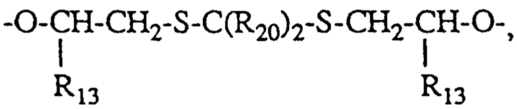,

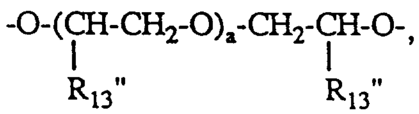,

-$OCH_2CH=CHCH_2O$-, -$OCH_2C\equiv CCH_2O$-, -$O(CH_2)_h$-NH-CO-$X_4$-OC-$NH(CH_2)_h$-O-, -$NH(CH_2)_2$-$X(CH_2)_2$-O-,-$NH(CH_2CH_2NH)_i$-$CH_2CH_2NH$-, -$NH(CH_2CH_2CH_2NH)_iCH_2CH_2CH_2NH$-,

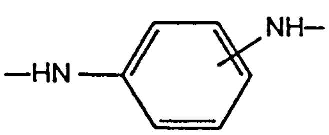, 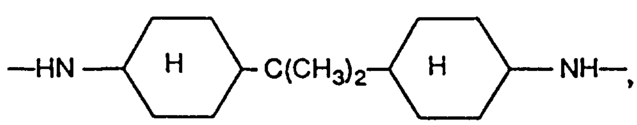,

-$NH(CH_2)_h$-O-CO-$X_4$-OC-O-$(CH_2)_h$-NH-, -NH-NH-, -NH-NH-CO-$X_4$-OC-NH-NH-, -NH-N=CH-$X_5$-CH=N-NH-, -$C_aH_{2a}$-, -$(CH_2)_m$-$X_1$-$(CH_2)_m$-, phénylène,

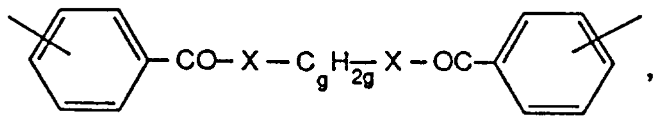

$-C_pH_{2p}-CO-X-C_gH_{2g}-X-OC-C_pH_{2p}-$, $-(CH_2)_k-O-CO-X_4-CO-O-(CH_2)_k-$, $-CH=CH-CO-X-C_gH_{2g}-X-OC-CH=CH-$, $-CH(OH)CH_2-$, $-CH(OH)CH(OH)-$,

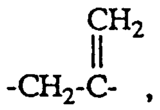

$-CH=CH-$, $-CH(R_{21})-$ ou

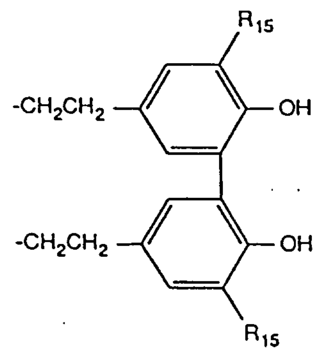

X, $R_{13}$, $R_{15}$, a et f ayant la signification donnée ci-dessus, les radicaux X', indépendamment l'un de l'autre, représentent -O- ou -NH-,
$R_{13}''$ est hydrogène ou méthyle et pour a = zéro peut être aussi alkyle en $C_2$-$C_{18}$ ou phényle,
$R_{14}'$ peut avoir la même signification que $R_{14}$ ou est hydrogène,
les radicaux $R_{20}$, indépendamment l'un de l'autre, représentent hydrogène, alkyle en $C_1$-$C_{20}$, phényle ou ensemble forment un cycle cycloaliphatique à 5 à 12 chaînons,
$R_{21}$ représente cycloalkyle en $C_5$-$C_{12}$, phényle(alkyle en $C_1$-$C_4$), phényle ou

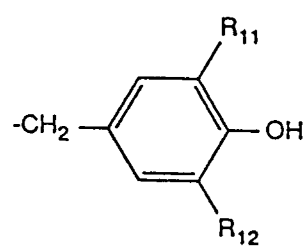

g va de 2 à 12, h va de 2 à 6, i va de 1 à 5, k va de 3 à 5, m vaut 1 ou 2 et p va de 1 à 10,
$X_4$ représente $-C_aH_{2a}-$, phénylène ou $-CH=CH-$ et
$X_5$ représente la liaison directe $-(CH_2)_3-$ ou $-C(CH_3)_2-S-S-C(CH_3)_2-$;
ou A pour n = 3 représente

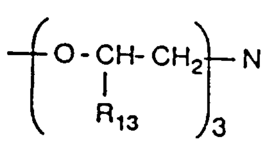, 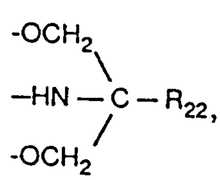, 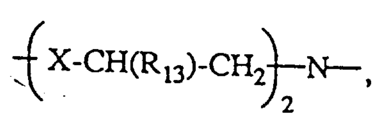,

$-(OCH_2)_3-C-R_{13}'$, $N(CH_2)_3-$ ou

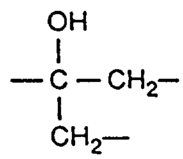

$R_{13}$ et $R_{13}'$ ayant la signification donnée ci-dessus et $R_{22}$ est méthyle ou éthyle;
ou A pour n = 4 est
$C(CH_2O)_4-$,

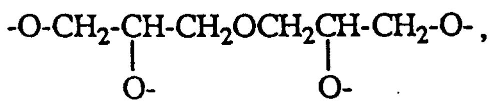

$\{OCH_2)_3-C-NH-$ ou $-(CH_2)_2NCH_2CH_2N(CH_2)_2-$ ist;
ou A pour n = 5 représente

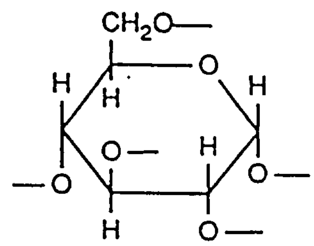

pour n = 6

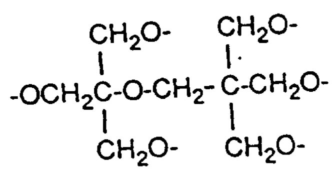

2. Composition selon la revendication 1, où R et $R_1$, indépendamment l'un de l'autre, représentent hydrogène, alkyle en $C_1$-$C_4$, alcoxy en $C_1$-$C_4$ ou halogène, plus particulièrement chlore, et $R_2$ et $R_3$ sont hydrogène.

3. Composition selon la revendication 1, dans lesquels R représente hydrogène, alkyle en $C_1$-$C_4$, alcoxy en $C_1$-$C_4$ ou chlore, $R_1$, $R_2$, $R_3$, et $R_4$ sont hydrogène, n vaut 1 ou 2, A pour n = 1 représente $-OR_5$, $-NHR_6$, $-NH-NH-R_8$, $-NH-N=CH-R_{17}$, alkyle en $C_1$-$C_{17}$, alcényle en $C_2$-$C_{17}$,

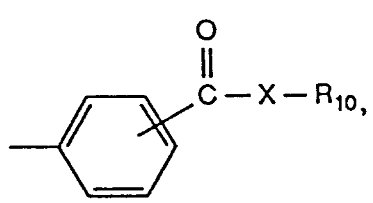

$-(CH_2)_pCO-X-R_{10}$, $-CH_2-X_1-C_1-C_{18}$-Alkyl,

$$-(CH_2)_b-O-\underset{\underset{O}{\|}}{C}-R_{10}',$$

$-(CH_2)_b-OH$,

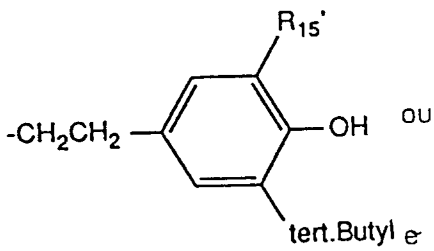 ou 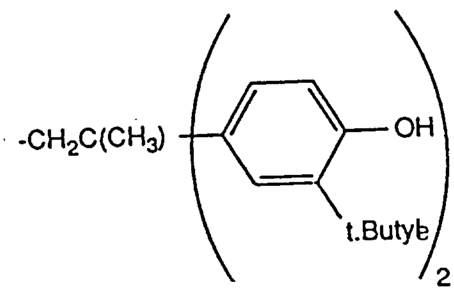 ,

$R_{10}$ représente hydrogène, alkyle en $C_1$-$C_{18}$, alcényle en $C_2$-$C_{18}$, cyclohexyle, méthylcylohexyle ou benzyle et
$R_{10}'$ représente alkyle en $C_1$-$C_{17}$, alcényle en $C_2$-$C_{17}$ ou phényle,
$R_5$ représente alkyle en $C_1$-$C_{18}$, alcényle en $C_2$-$C_{18}$, cyclohexyle, méthylcyclohexyle, benzyle,

$-\underset{\underset{R_{13}}{|}}{CH}-CH_2-X_1-R_{10}''$ ou 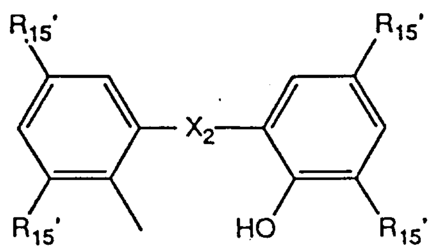

où $R_{10}''$ représente hydrogène, alkyle en $C_1$-$C_{18}$ ou alcényle en $C_2$-$C_{18}$ et les radicaux $R_{15}'$, indépendamment l'un l'autre, sont méthyle ou tert.butyle,
$R_6$ représente alkyle en $C_1$-$C_{18}$, alcényle en $C_2$-$C_{18}$, cyclohexyle, méthylcyclohexyle, benzyle, $-CH_2CH_2OH$,

$$-CH_2CH_2O-\underset{\underset{O}{\|}}{C}-(\text{alkyle en } C_1-C_{18}) \text{ ou}$$

$$-CH_2CH_2O-\underset{\underset{O}{\|}}{C}-(\text{alcényle en } C_2-C_{18}),$$

$R_8$ est hydrogène, alkyle en $C_1$-$C_{12}$, -CO-(alkyle en $C_1$-$C_{18}$) ou -CO-(alcényle en $C_2$-$C_{18}$)
$R_{17}$ représente alkyle en $C_1$-$C_{11}$, alcényle en $C_2$-$C_{11}$, $-(CH_2)m$-S-(alkyle en $C_1$-$C_{18}$) ou

$$-(CH_2)m-\overset{\overset{O}{\|}}{P}-(\text{alcoxy en } C_1-C_8)_2;$$

ou A pour n = 2 représente $-O-(CH_2)_g-O-$, $-NH-(CH_2)_g-NH-$,

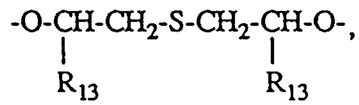

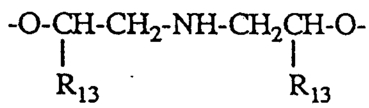, 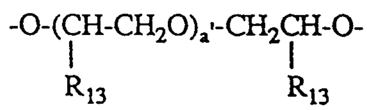

avec a' = 0 à 5 ou $-OCH_2-C(CH_3)_2CH_2-O-$, $-NHCH_2CH_2-X-CH_2CH_2O-$, $-NH(CH_2CH_2NH)_i-CH_2CH_2NH-$, $-NH(CH_2CH_2CH_2NH)_i-CH_2CH_2CH_2-NH-$, $-NH-C(CH_3)_2-CH_2O-$,

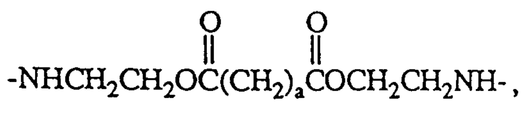, 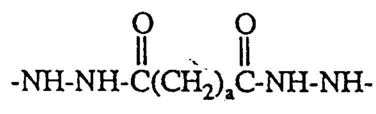,

$-NH-N=CH-X_5-CH=N-NH-$, $-(CH_2)_p-$, phénylène

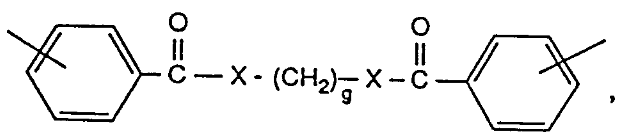, 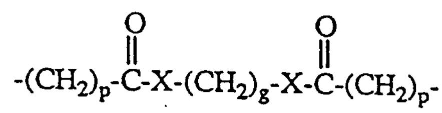,

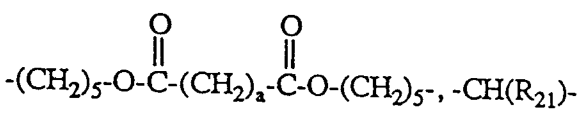, $-CH(R_{21})-$

avec $R_{21}$ = benzyle, phényle ou

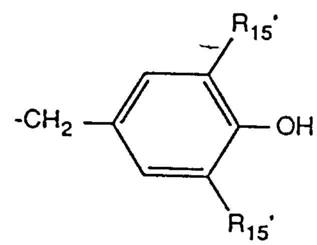 ou A 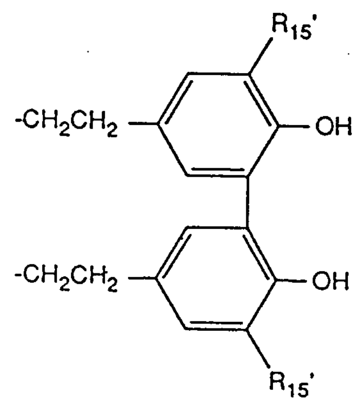

et a,b, g, i, m, p, X, $X_1$, $X_2$, $X_5$ et $R_{13}$ ont la signification donnée ci-dessus.

4. Composition selon la revendication 1, où R représente hydrogène, chlore, méthyle ou méthoxy et $R_1$, $R_2$, $R_3$ et $R_4$ sont hydrogène, n vaut 1 ou 2, A pour n = 1 représente $-OR_5$, $-NHR_6$, $-NH-NH-R_8$, $-NH-N=CH-R_{17}$, alkyle en $C_1$-$C_{17}$, $-(CH_2)p-COOH$,

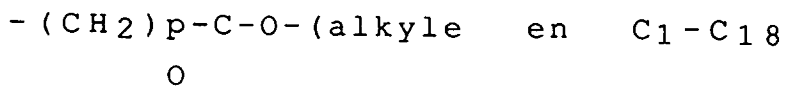

$-(CH_2)_bOH$, $-(CH_2)_b$-OCO- (alkyle en $C_1$-$C_{18}$) $-CH_2$-S- (alkyle en ($C_1$-$C_{18}$) $-CH_2$-S-$C_1$-$C_{18}$-Alkyl,

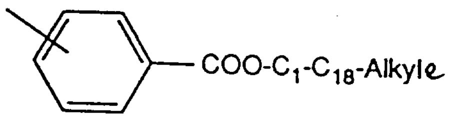
ou

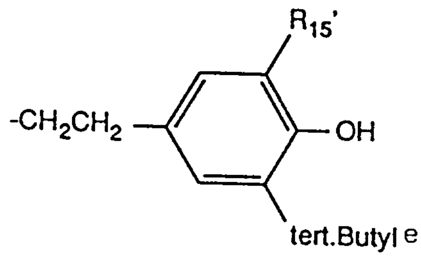

$R_5$ représentant alkyle en $C_1$-$C_{18}$, cyclohexyle, méthylcyclohexyle, benzyle ou

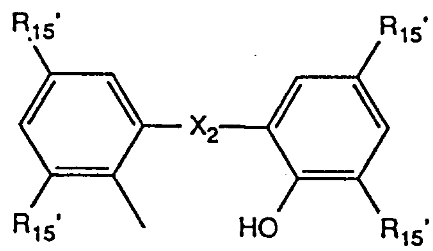

$R_6$ représente alkyle en $C_1$-$C_{18}$, cyclohexyle, benzyle, $-(CH_2)$f-OH ou $-CH_2CH_2O$-CO-(alkyle en $C_1$-$C_{18}$),
$R_8$ représente hydrogène, -CO-(alkyle en $C_1$-$C_{18}$) ou $-COCH=CH_2$,
les radicaux $R_{15}$', indépendamment l'un de l'autre, sont méthyle ou tert.butyle,
$R_{17}$ représente alkyle en $C_1$-$C_{11}$, $-(CH_2)$m-S-(alkyle en $C_1$-$C_{18}$), ou

$$-(CH_2)_m-\underset{\underset{O}{\|}}{P}-(\text{alcoxy en } C_1\text{-}C_4)_2$$

ou
ou A pour n = 2 représente
$-O-(CH_2)_g-O-$, $-NH-(CH_2)_g-NH-$, $-OCH_2CH_2SCH_2CH_2O-$, $-O(CH_2CH_2O)_c-CH_2CH_2-O-$, $-OCH_2C(CH_3)_2CH_2O-$, $NHCH_2CH_2OCH_2CH_2O-$,

$$-NHCH_2CH_2O\overset{O}{\overset{\|}{C}}(CH_2)_a\overset{O}{\overset{\|}{C}}OCH_2CH_2NH-,\ -NH-NH-\overset{O}{\overset{\|}{C}}(CH_2)_a\overset{O}{\overset{\|}{C}}-NH-NH-,$$

$-NH-N=CH-C(CH_3)_2-S-S-C(CH_3)_2-CH=N-NH-$, phénylène ou $-(CH_2)$p-
a, b, c, f, g, m, p et $X_2$ ayant la signification donnée dans la revendication 1.

5. Composition selon la revendication 1, où R représente chlore, méthyle ou méthoxy, et plus particulièrement hydrogène, $R_1$, $R_2$, $R_3$, et $R_4$ sont hydrogène,
n vaut 1 ou 2,

A pour n = 1 représente $-OR_5$, $-NHR_6$, $-NH-NH-R_8$, $NH-N-=CHR_{17}$, alkyle en $C_8$-$C_{17}$, $-(CH_2)_{p'}-CO-O-$(alkyle en $C_8$-$C_{18}$), avec p' = de 2 à 6, $-(CH_2)_b-OH$, $-(CH_2)_b-OCO-$(alkyle en $C_8$-$C_{18}$), $-CH_2-S-$(alkyle en $C_8$-$C_{18}$)

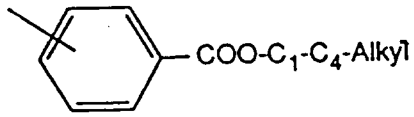

ou

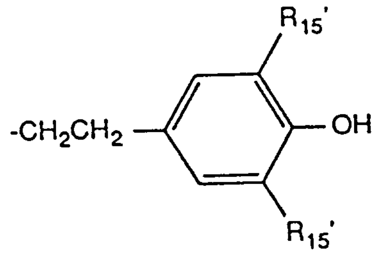

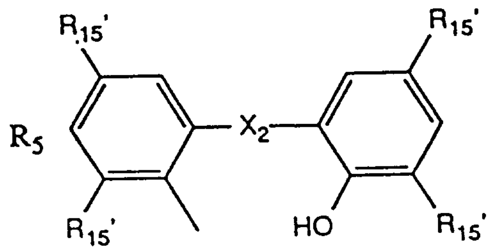

et représente plus particulièrement alkyle en $C_8$-$C_{18}$,
$R_6$ représente alkyle en $C_8$-$C_{18}$, $-(CH_2)_{f''}-OH$ avec f" = 2 ou 3 ou $-CH_2CH_2OCO-$(alkyle en $C_8$-$C_{18}$),
$R_8$ représente -CO-(alkyle en $C_8$-$C_{18}$) ou $-COCH=CH_2$,
$R_{17}$ représente alkyle en $C_8$-$C_{11}$, $(CH_2)m-S-$(alkyle en $C_8$-$C_{18}$) ou

$$-(CH_2)_m-\underset{\underset{O}{\|}}{P}-(\text{alcoxy en } C_1\text{-}C_4)_2$$

ou A pour n = 2 représente
$-O(CH_2)_gO-$, $-NH(CH_2)_gNH-$, $-OCH_2CH_2SCH_2CH_2O-$, $-O(CH_2CH_2O)_cCH_2CH_2O-$, $-OCH_2C(CH_3)_2CH_2O-$, $-NHCH_2CH_2OCH_2CH_2O-$,$-NHCH_2CH_2OCO-(CH_2)_{a''}-OCOCH_2CH_2NH-$ oder $-NH-NHCO-(CH_2)_{a''}-CONH-NH-$ avec a" = 2 à 8, $-NH-N=CH-C(CH_3)_2-S-S-C(CH_3)_2-CH=N-NH-$, $-(CH_2)_{p''}-$ avec p" = 5 à 10 ou phénylène,
b va de 3 à 5, est de préférence 5, $X_2$ est la liaison directe ou $CH-CH_3$, les radicaux $R_{15}'$, indépendamment l'un de l'autre, sont méthyle ou tert.butyle et c, g et m ont la signification donnée dans la revendication 1.

**6.** Composition selon la revendication 1, où R, $R_1$, $R_2$, $R_3$ et $R_4$ représentent hydrogène,
n vaut 1 ou 2,
A pour n = 1 représente alkyle en $C_8$-$C_{17}$, $-OR_5$, $-NHR_6$ ou -NH-NH-CO-(alkyle en $C_8$-$C_{17}$),
$R_5$ et $R_6$, indépendamment l'un de l'autre, représentent alkyle $C_8$-$C_{18}$,
ou A pour n = 2 représente
$-O(CH_2)_g-O-$, $NH(CH_2)_gNH-$, $-OCH_2C(CH_3)_2CH_2O-$, $-NH-NHCO-CH_2CH_2-OCNH-NH-$, $-(CH_2)_{p''}-$ avec p" = de 5 à 10 ou phénylène,
g ayant la signification donnée dans la revendication 1.

**7.** Composition selon la revendication 1, contenant un composé de formule Ia

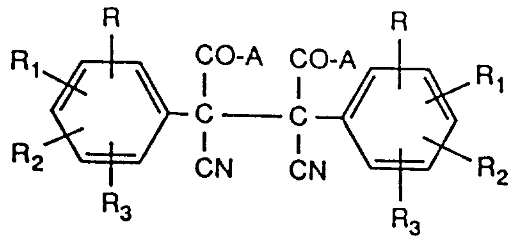

(Ia)

dans laquelle R est hydrogène, alkyle en $C_1$-$C_4$, alcoxy en $C_1$-$C_4$ ou chlore, $R_1$, $R_2$ et $R_3$ sont hydrogène et A représente -O-(alkyle en $C_1$-$C_{18}$), plus particulièrement -O-(alkyle en $C_1$-$C_4$).

**8.** Composition selon la revendication 1, où la matière organique est un lubrifiant, un fluide de traitement de métaux, un fluide hydraulique, un polymère naturel, semi-synthétique ou synthétique.

**9.** Composition selon la revendication 8, où la matière organique est un lubrifiant, un élastomère ou une matière plastique thermoplastique dépurvue d'halogène, plus particulièrement une polyoléfine non halogénée.

**10.** Composés de formule Ib

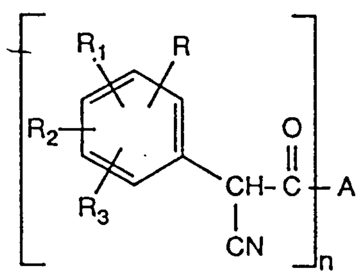

(1b)

dans laquelle R représente hydrogène, -OH, halogène, alkyle en $C_1$-$C_{20}$, alcényle en $C_2$-$C_{20}$, cycloalkyle en $C_5$-$C_{12}$, (alkyl en $C_1$-$C_4$)-(cycloalkyle en $C_5$-$C_{12}$), phényle, naphtyle, alcoxy en $C_1$-$C_{20}$, phényloxy, naphtyloxy, -S-(alkyle en $C_1$-$C_{20}$), -S-phényle, -S-naphtyle, -O-CO-(alkyle en $C_1$-$C_{20}$), -O-CO-phényle, -O-CO-naphtyle, -COOH, -COO-(alkyle en $C_1$-$C_{20}$), -COO-(alcényle en $C_2$-$C_{20}$), -COO-phényle, -COO-naphtyle, -CONR'R'', -CO-(alkyle en $C_1$-$C_{20}$), -CO-phényle, -CO-naphtyle, -NHCO-(alkyle en $C_1$-$C_{20}$), -NHCO-(alcényle en $C_2$-$C_{20}$), -NHCO-phényle ou -NHCO-naphtyle,
$R_1$ peut avoir la même signification que R
$R_2$ est hydrogène, halogène ou alkyle en $C_1$-$C_4$ et
$R_3$ représente hydrogène ou halogène,
ou deux radicaux R, $R_1$, $R_2$ ou $R_3$ liés en position ortho l'un par rapport à l'autre, ensemble, forment un tétraméthylène ou
-CH=CH-CH=CH-, les deux restants de R, $R_1$, $R_2$ et $R_3$ étant hydrogène,
ou R1 à R3 sont hydrogène et R pour n = 1 représente un groupement

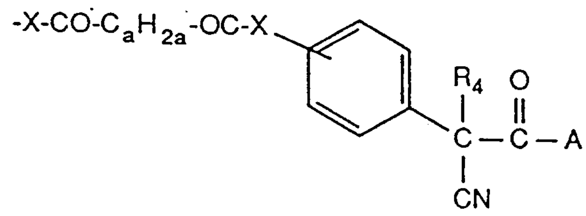

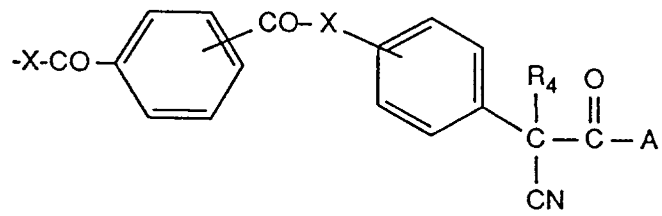

où $R_4$ est chaque fois hydrogène,
R' et R", indépendamment l'un de l'autre, représentent hydrogène, alkyle en $C_1$-$C_{20}$, alcényle en $C_2$-$C_{20}$, phényle ou naphtyle ou, ensemble avec l'atome liant N, forment un cycle hétérocyclique à 5 ou 6 chaînons,
a va de 0 à 10, n va de 1 à 6,
X représente -O- ou -NH-
A pour n = 1 représente $-OR_5$, $-NR_6R_7$, $-NH-NR_8R_9$, alkyle en $C_1$-$C_{30}$, alcényle en $C_2$-$C_{30}$, cycloalkyle en $C_5$-$C_{12}$, (alkyl en $C_1$-$C_4$)-(cycloalkyle en $C_5$-$C_{12}$)

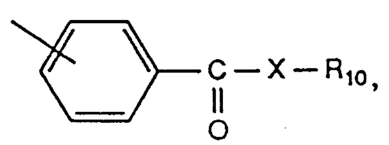

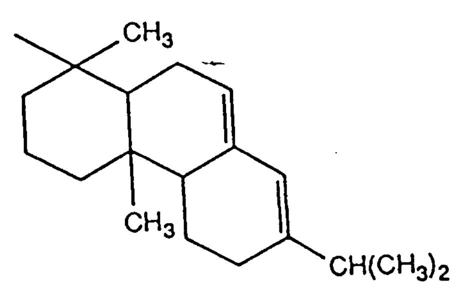

ou les dérivés dihydro ou tétrahydro correspondants
$-C_aH_{2a}-CO-X-R_{10}$, $-C_bH_{2b}OCO-R_{10}$, $-C_bH_{2b}-OH$,

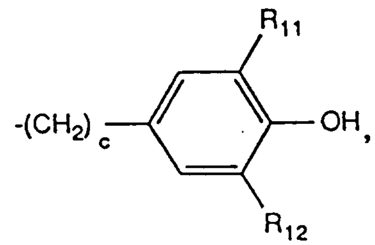

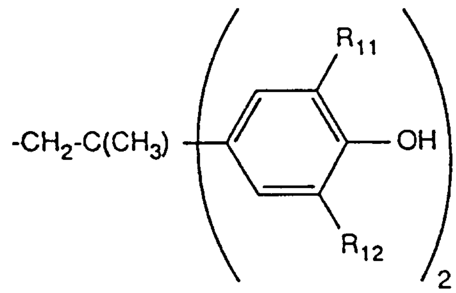, 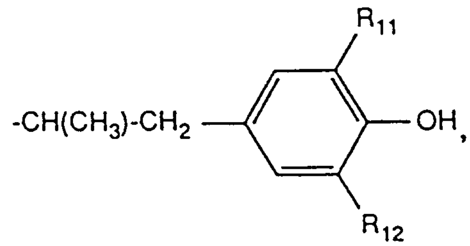

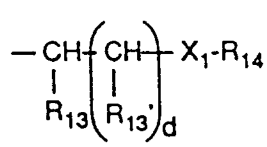 ou 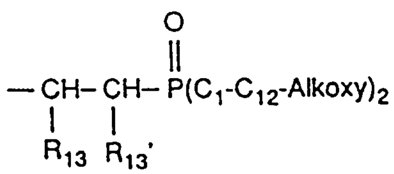

$R_5$ représentant alkyle en $C_1$-$C_{30}$, alcényle en $C_2$-$C_{30}$, cycloalkyle en $C_5$-$C_{12}$, (alkyl en $C_1$-$C_4$)-(cycloalkyle en $C_5$-$C_{12}$),phénylalkyle en $C_1$-$C_4$, phényle, naphtyle, biphényle, le radical d'un alcool terpénique ou un radical de formules

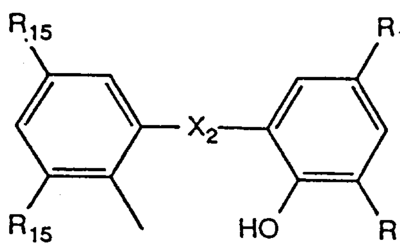, 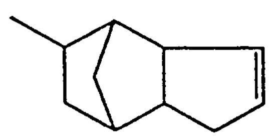, 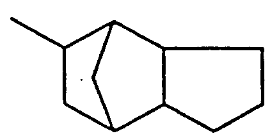,

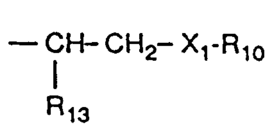 ou 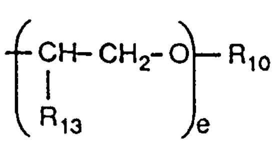

$R_6$ et $R_7$, indépendamment l'un de l'autre, représentent hydrogène, alkyle en $C_1$-$C_{20}$, alcényle en $C_2$-$C_{20}$, cycloalkyle en $C_5$-$C_{12}$, (alkyl en $C_1$-$C_4$)-(cycloalkyle en $C_5$-$C_{12}$), phénylalkyle en $C_1$-$C_4$, phényle, naphtyle,

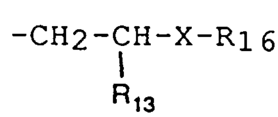

ou $-CH_2C(CH_2$-O-$R_{16})_3$ ou $R_6$ pour $R_7$ = H peut aussi être $-(CH_2)_f$-X-$R_{16}$ ou $R_6$ et $R_7$, ensemble avec l'atome de liaison N, forment un cycle hétérocyclique à 5 à 6 chaînons,

$R_8$ et $R_9$, indépendamment l'un de l'autre, représentent hydrogène, alkyle en $C_1$-$C_{20}$, phényle, -CO-

(alkyle en $C_1$-$C_{20}$), -CO-(alcényle en $C_2$-$C_{18}$), -CO-phényle ou -CO-naphtyle ou ensemble représentent $=CH$-$R_{17}$,

$R_{10}$ est hydrogène, alkyle en $C_1$-$C_{20}$, alcényle en $C_2$-$C_{20}$, cycloalkyle en $C_5$-$C_{12}$, phénylalkyle en $C_1$-$C_4$, phényle, naphthyle, -CO-(alkyle en $C_1$-$C_{20}$), -CO-phényle, -CO-naphthyle ou

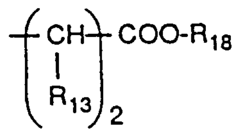

$R_{11}$ et $R_{12}$, indépendamment l'un de l'autre, sont hydrogène, alkyle en $C_1$-$C_4$, cyclohexyle ou phényle,

$R_{13}$ est hydrogène ou méthyle et $R_{13}$' est hydrogène, méthyle ou phényle,

$R_{14}$ représente alkyle en $C_1$-$C_{20}$, alcényle en $C_2$-$C_{20}$, cycloalkyle en $C_5$-$C_{12}$, phénylalkyle en $C_1$-$C_4$, phényle ou naphtyle,

les radicaux $R_{15}$ indépendamment l'un de l'autre représentent alkyle,

$R_{16}$ représente hydrogène, -CO-(alkyle en $C_1$-$C_{20}$), -CO-(alcényle en $C_2$-$C_{20}$), -CO-phényle ou -CO-naphtyle,

$R_{17}$ représente alkyle en $C_1$-$C_{20}$, alcényle en $C_2$-$C_{20}$, cycloalkyle en $C_5$-$C_{12}$, phényle, naphtyle, 2-furyle,

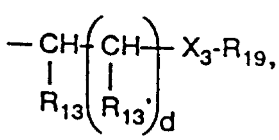, 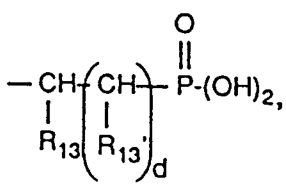, 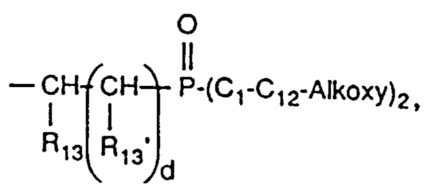,

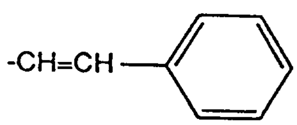, 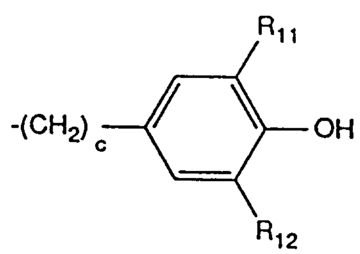 ou 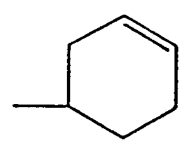

$R_{18}$ représente hydrogène, alkyle en $C_1$-$C_{20}$, alcényle en $C_2$-$C_{20}$,

$R_{19}$ représente alkyle en $C_1$-$C_{20}$, cycloalkyle en $C_5$-$C_{12}$, phényle ou -$CH_2$-COO-(alkyle en $C_{12}$-$C_{20}$),

X et a ont la signification donnée ci-dessus,

$X_1$ représente -O-, -S-, -NH- ou -$NR_{14}$-,

$X_2$ représente la liaison directe, -$CH_2$-, $>$CH-$CH_3$, ou -S- et

$X_3$ représente -O-, -S-, -NH- ou -$NR_{19}$,

b va de 3 à 5,

c va de 0 à 2,

d vaut 0 ou 1,

e va de 2 à 10 et

f va de 2 à 6 ;

à la condition que A ne soit pas égal à -O-(alkyle en $C_1$-$C_{12}$), -$NH_2$, -NH-méthyle, -NH-cyclohexyle, -NH-phényle,

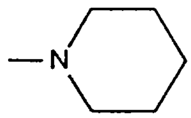,

-NH-NH$_2$, alkyle en $C_1$-$C_7$, -CH$_2$N(CH$_3$)$_2$, -CH$_2$N(méthyl)-(phényle) ou -$C_{a''}H_{2a''}$-CO-O-$R_{10'}$ avec a'' = 2 ou 4 et $R_{10'}$ = hydrogène, méthyle ou éthyle, lorsque l'un des radicaux R, $R_1$, $R_2$ ou $R_3$ sont hydrogène, chlore, méthyle ou méthoxy et les autres radicaux R, $R_1$, $R_2$ ou $R_3$ représentent hydrogène; et que sont exclus les composés de formule Ib, où R, $R_1$, $R_2$, $R_3$ = H, et A = alkyle en $C_1$-$C_{24}$, -O-(alkyle en $C_1$-$C_{24}$), -NH$_2$, -NH-alkyle en $C_1$-$C_{20}$ ou -N(alkyle en $C_1$-$C_{20}$)$_2$, pipéridino, morpholino, pyrrolidino, diméthylamino et diéthylamino, ainsi que les composés dans lesquels $R_{16}$ = hydrogène et f = 2, et que les composés de formules

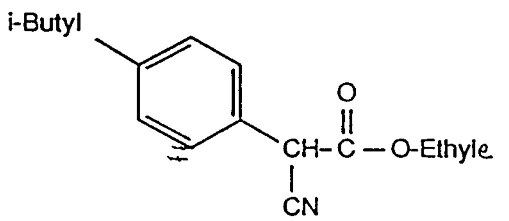, 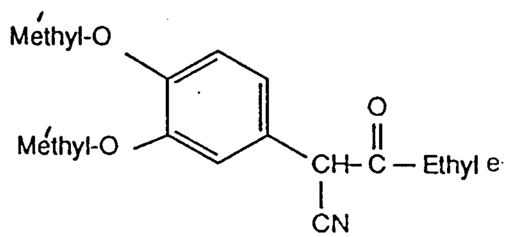,

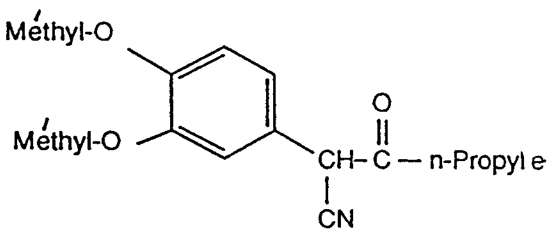, 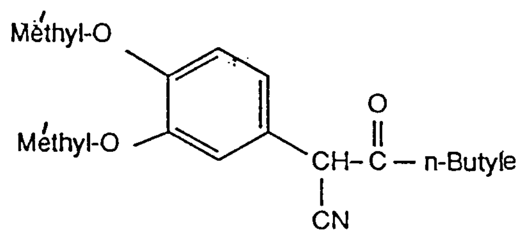,

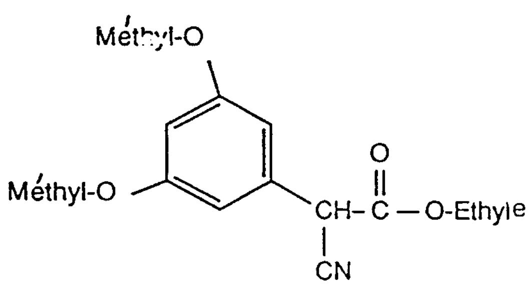 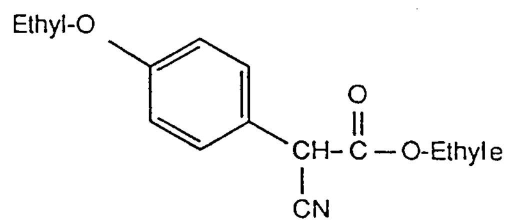,

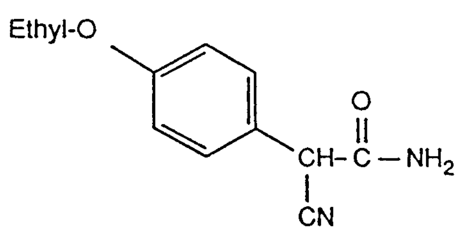 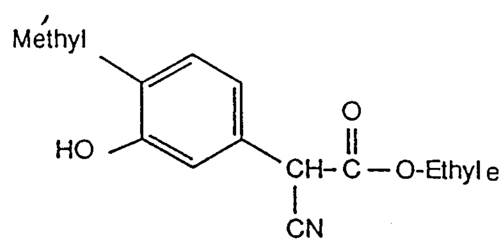,

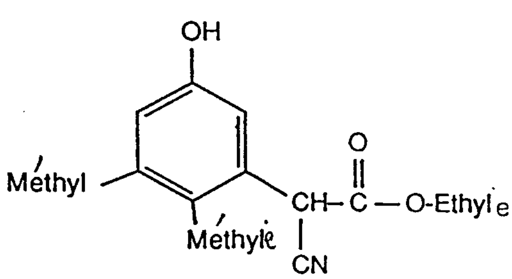 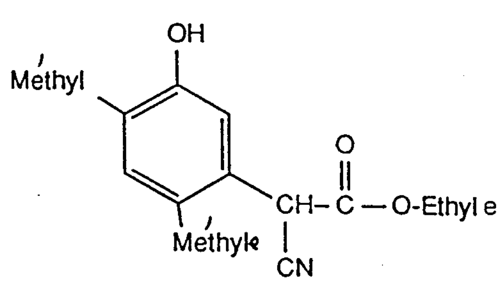,

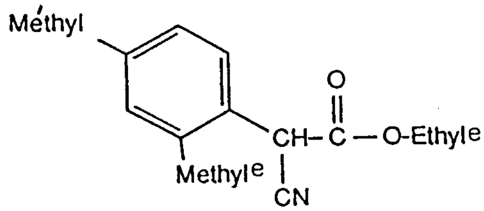
,
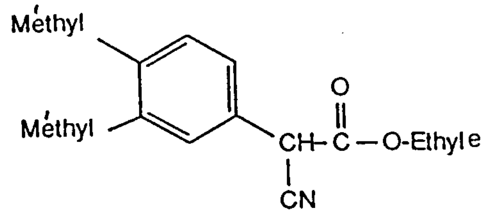
,

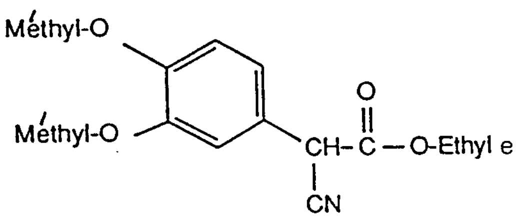
,
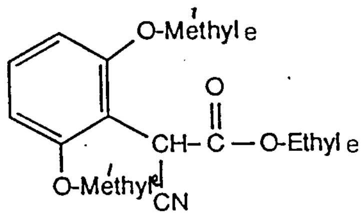
,

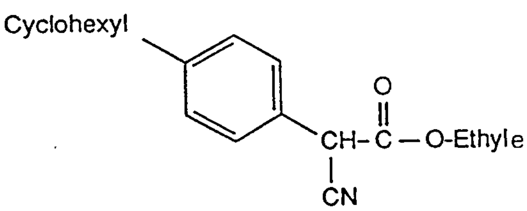

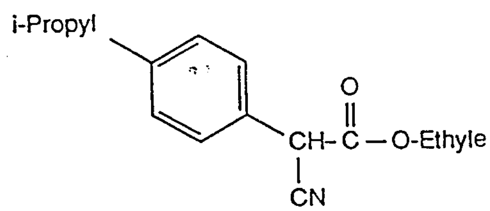
,

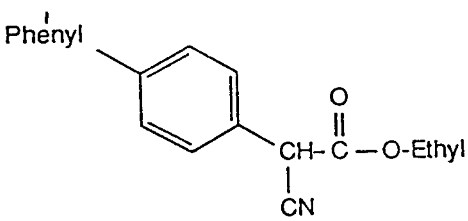

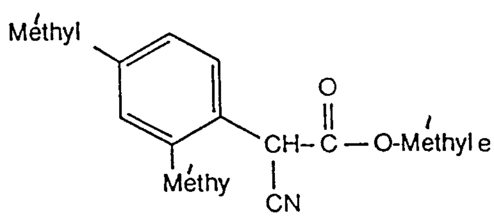
,

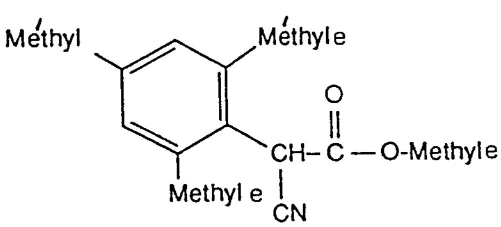
,
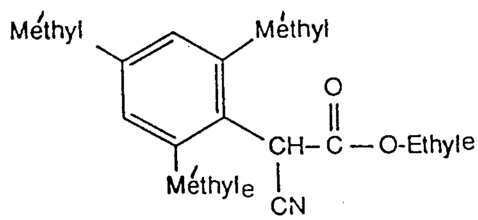
et

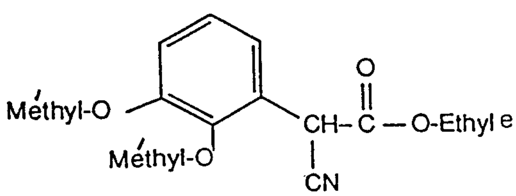

sont également exclus.
ou A pour n = 2 représente
$-X'-C_gH_{2g}-X'-$,

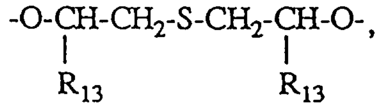

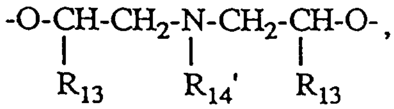, 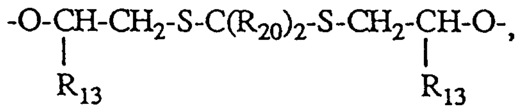

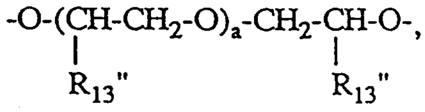

$-OCH_2CH=CHCH_2O-$, $-OCH_2C{\equiv}CCH_2O-$, $-O(CH_2)_h-NH-CO-X_4-OC-NH(CH_2)_h-O-$, $-NH(CH_2)_2-X(CH_2)_2-O-$, $-NH(CH_2CH_2NH)_i-CH_2CH_2NH-$, $-NH(CH_2CH_2CH_2NH)_iCH_2CH_2CH_2NH-$,

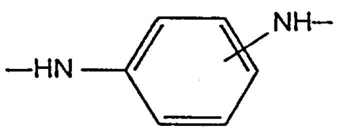, 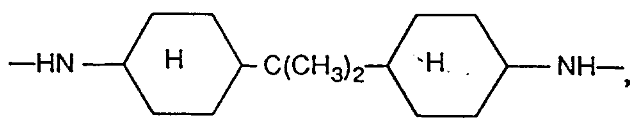

$-NH(CH_2)_h-O-CO-X_4-OC-O-(CH_2)_h-NH-$, $-NH-NH-$, $-NH-NH-CO-X_4-OC-NH-NH-$, $-NH-N=CH-X_5-CH=N-NH-$, $-C_pH_{2p}-$, $-(CH_2)_m-X_1-(CH_2)_m-$,

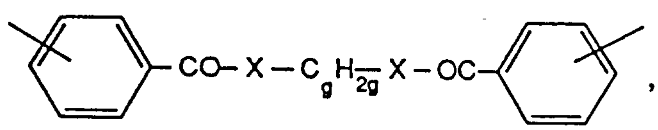,

$-C_pH_{2p}-CO-X-C_gH_{2g}-X-OC-C_pH_{2p}-$, $-(CH_2)_k-O-CO-X_4-CO-O-(CH_2)_k-$, $-CH=CH-CO-X-C_gH_{2g}-X-OC-CH=CH-$, $-CH(OH)CH_2-$, $-CH(OH)CH(OH)-$,

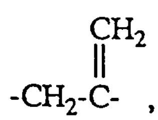,

$-CH=CH-$, $-CH(R_{21})-$ ou

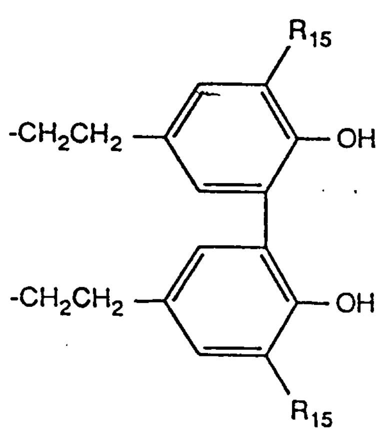

X, $R_{13}$, $R_{15}$, a et f ayant la signification donnée ci-dessus, les radicaux X', indépendamment l'un de

l'autre, représentent -O- ou -NH-,
$R_{13}$'' est hydrogène ou méthyle et pour a = 0 peut aussi être alkyle en $C_2$-$C_{18}$ ou phényle,
$R_{14}$' peut avoir la même signification que $R_{14}$ ou est hydrogène,
les radicaux $R_{20}$, indépendamennt l'un de l'autre, représentent hydrogène, alkyle en $C_1$-$C_{20}$, phényle ou ensemble forment un cycle cycloaliphatique de 5 à 12 chaînons,
$R_{21}$ représente cycloalkyle en $C_5$-$C_{12}$, phénylalkyle en $C_1$-$C_4$, phényle ou

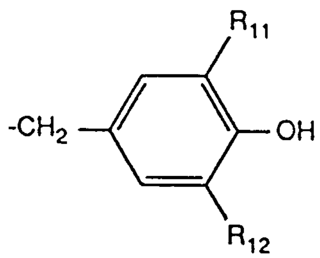

g va de 2 à 12, h va de 2 à 6, i va de 1 à 5, k va de 3 à 5,
m vaut 1 ou 2 et p va de 1 à 10,
$X_4$ représente $-C_aH_{2a}-$, phénylène ou -CH=CH-, et
$X_5$ représente la liaison directe, $-(CH_2)_3-$ ou $-C(CH_3)_2-S-S-C(CH_3)_2-$;
ou A pour n = 3 représente

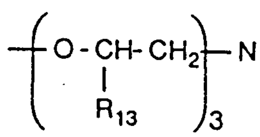, 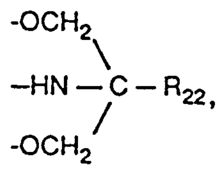, 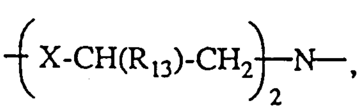,

$-(OCH_2)_3-C-R_{13}'$, $N(CH_2)_3-$ ou

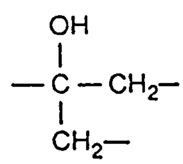

$R_{13}$ et $R_{13}$' ayant la signification donnée ci-dessus et $R_{22}$ est méthyle ou éthyle;
ou A pour n = 4 est
$C(CH_2O)_4-$,

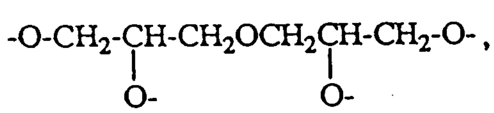

$\{OCH_2)_3-C-NH-$ ou $-(CH_2)_2NCH_2CH_2N(CH_2)_2-$ ist,
ou A pour n =5 représente

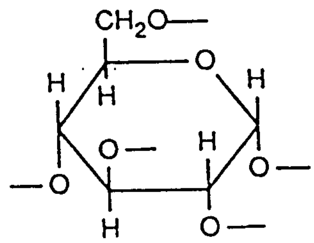

et pour n = 6 et

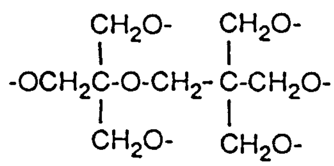

**11.** Composés selon la revendication 10, où R et $R_1$, indépendamment l'un de l'autre, représentent hydrogène, alkyle en $C_1$-$C_4$, alcoxy en $C_1$-$C_4$ ou halogène, plus particulièrement chlore, et $R_2$ et $R_3$ sont hydrogène.

**12.** Composés selon la revendication 10, où R est hydrogène, alkyle en $C_1$-$C_4$, alcoxy en $C_1$-$C_4$ ou chlore, $R_1$, $R_2$ et $R_3$ sont hydrogène, n vaut 1 ou 2,
A pour n = 1 représente -$OR_5$, -$NHR_6$, -NH-NH-$R_8$, -NH-N=CH-$R_{17}$, alkyle en $C_1$-$C_{17}$, alcényle en $C_2$-$C_{17}$,

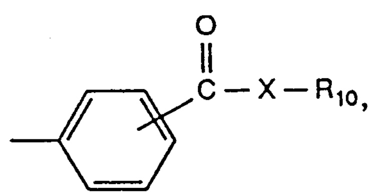

-$(CH_2)_p$CO-X-$R_{10}$, -$CH_2$-$X_1$-$C_1$-$C_{18}$-Alkyl,

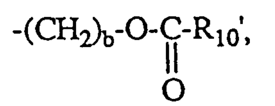

-$(CH_2)_b$-OH,

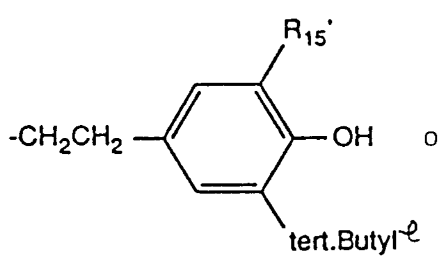 ou 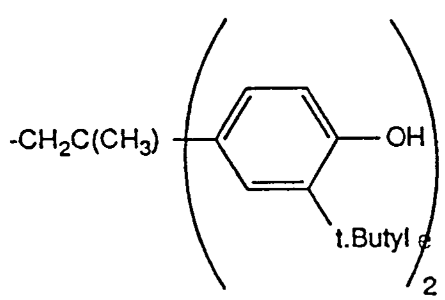 ,

$R_{10}$ représente hydrogène, alkyle en $C_1$-$C_{18}$, alcényle en $C_2$-$C_{18}$, cyclohexyle, méthylcyclohexyle ou benzyle et

$R_{10}'$ représente alkyle en $C_1$-$C_{17}$, alcényle en $C_2$-$C_{17}$ ou phényle,

$R_5$ représente alkyle en $C_1$-$C_{18}$, alcényle en $C_2$-$C_{18}$, cyclohexyle, méthylcyclohexyle, benzyle,

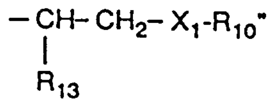 ou 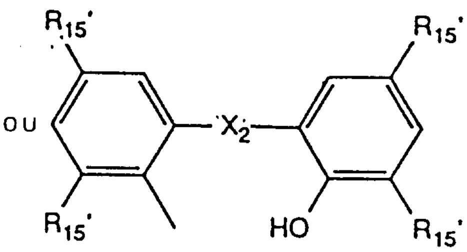

ou $R_{10}''$ est hydrogène, alkyle en $C_1$-$C_{18}$ ou alcényle en $C_2$-$C_{18}$ et les radicaux $R_{15}'$, indépendamment l'un de l'autre, sont méthyle ou tert.butyle,

$R_6$ représente alkyle en $C_1$-$C_{18}$ et alcényle en $C_2$-$C_{18}$, méthylcyclohexyle, benzyle $-CH_2CH_2OH$,

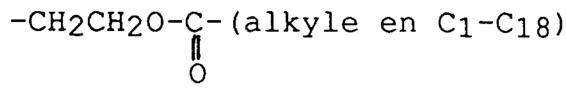 ou

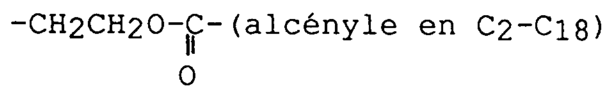

$R_8$ représente hydrogène, alkyle en $C_1$-$C_{12}$, -CO-(alkyle en $C_1$-$C_{18}$) ou -CO-(alcényle en $C_2$-$C_{18}$),

$R_{17}$ représente alkyle en $C_1$-$C_{11}$, alcényle en $C_2$-$C_{11}$, $-(CH_2)_m$-S-(alkyle en $C_1$-$C_{18}$) ou

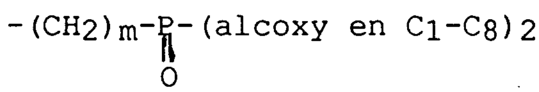

ou A pour n = 2 représente

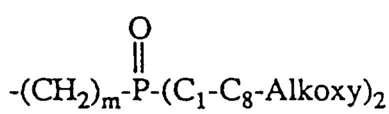

ist;

oder A, bei n = 2, $-O-(CH_2)_g-O-$, $-NH-(CH_2)_g-NH-$,

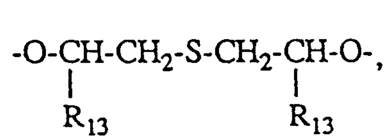,

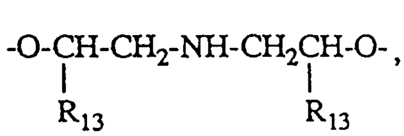, 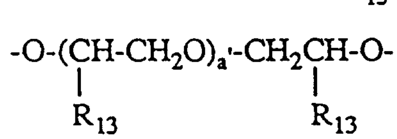

avec a' = 0 à 5, ou $-OCH_2-C(CH_3)_2CH_2-O-$, $-NHCH_2CH_2-X-CH_2CH_2O-$, $-NH(CH_2CH_2NH)_i-CH_2CH_2NH-$, $-NH(CH_2CH_2CH_2NH)_i-CH_2CH_2CH_2-NH-$, $-NH-C(CH_3)_2-CH_2O-$,

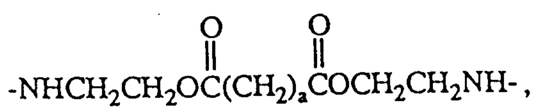, 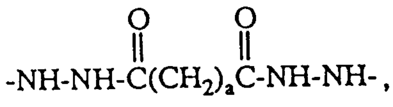,

-NH-N = CH-$X_5$-CH = N-NH-,

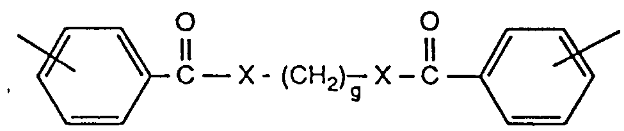,

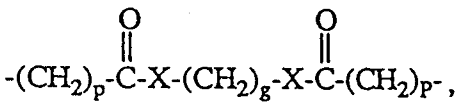, 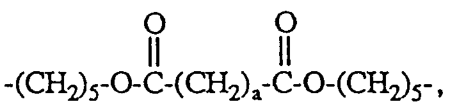,

$R_{21}$ = benzyle, phényle ou

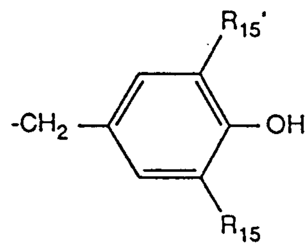 ou A 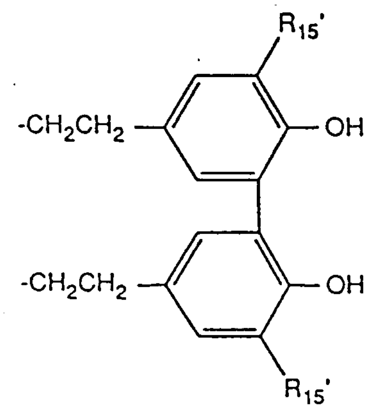

et a, b, g, i, m, p, X, $X_1$, $X_2$, $X_5$ et $R_{13}$ ont la signification donnée ci-dessus.

**13.** Composés selon la revendication 10, où R est hydrogène, chlore, méthyle ou méthoxy, $R_1$, $R_2$ et $R_3$ sont hydrogène,
n vaut 1 ou 2,
A pour n = 1 représente -$OR_5$, -$NHR_6$, -NH-NH-$R_8$,

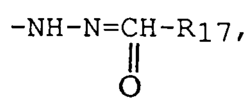,

alkyle en $C_8$-$C_{17}$, -(CH2)$_p$-COOH, -($CH_2$)p-C-O-(alkyle en $C_3$-$C_{18}$), -($CH_2$)$_b$OH, -($CH_2$)$_b$-OCO-(alkyle en $C_1$-$C_{18}$), -$CH_2$-S-(alkyle en $C_1$-$C_{18}$),

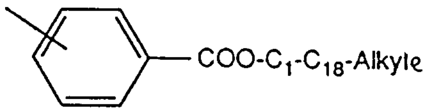 ou 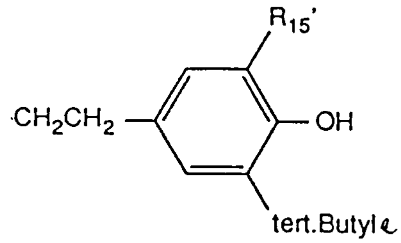

$R_5$ étant alkyle en $C_{13}$-$C_{18}$, cyclohexyle, méthylcyclohexyle, benzyle ou

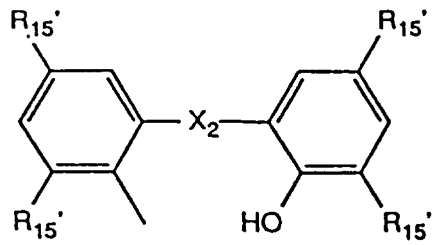

$R_6$ représente alkyle en $C_2$-$C_{18}$, benzyle, $-(CH_2)_f-OH$ ou $-CH_2CH_2O-CO-$(alkyle en $C_1$-$C_{18}$), $R_8$ représente -CO-(alkyle en $C_1$-$C_{18}$) ou $-COCH=CH_2$, les radicaux $R_{15}'$, indépendamment l'un de l'autre, sont méthyle ou tert.butyle, $R_{17}$ représente alkyle en $C_1$-$C_{11}$, $-(CH_2)_m-S-$(alkyle en $C_1$-$C_{18}$) ou

$$-(CH_2)_m-\underset{\underset{O}{\|}}{P}-(\text{alcoxy en } C_1-C_4)_2,$$

ou A pour n = 2 représente $-O-(CH_2)_g-O-$, $-NH-(CH_2)_g-NH-$, $-OCH_2CH_2SCH_2CH_2O-$, $-O(CH_2CH_2O)_c-CH_2CH_2-O-$, $-OCH_2C(CH_3)_2CH_2O-$, $NHCH_2CH_2OCH_2CH_2O-$,

$$-NHCH_2CH_2O\overset{\overset{O}{\|}}{C}(CH_2)_a\overset{\overset{O}{\|}}{C}OCH_2CH_2NH-,\quad -NH-NH-\overset{\overset{O}{\|}}{C}(CH_2)_a\overset{\overset{O}{\|}}{C}-NH-NH-,$$

$-NH-N=CH-C(CH_3)_2-S-S-C(CH_3)_2-CH=N-NH-$ ou $-(CH_2)_p-$
a, b, c, f, g, m, p, et $X_2$ ayant la signification donnée dans la revendication 10.

**14.** Composés selon la revendication 10, où R représente chlore, méthyle ou méthoxy et plus particulièrement hydrogène, $R_1$, $R_2$ et $R_3$ sont hydrogène,
n vaut 1 ou 2,
A pour n = 1 représente $-OR_5$, $-NHR_6$, $-NH-NH-R_8$, $-NH-N=CH-R_{17}$, alkyle en $C_8$-$C_{17}$, $-(CH_2)_{p'}-CO-O-$(alkyle en $C_8$-$C_{18}$) avec p' = de 2 à 6, $-(CH_2)_b-OH$, $-(CH_2)_b-OCO-$(alkyle en $C_8$-$C_{18}$), $-CH_2-S-$(alkyle en $C_8$-$C_{18}$),

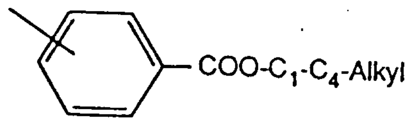

ou

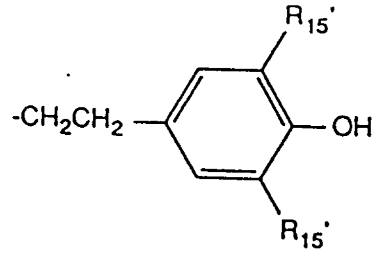

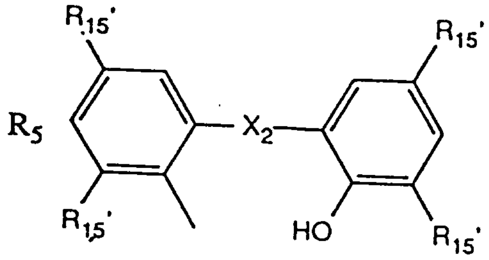

et plus particulièrement alkyle en $C_{13}$-$C_{18}$,
$R_6$ représente alkyle en $C_8$-$C_{18}$, -$(CH_2)_{f''}$-OH avec f" = 2 ou 3 ou -$CH_2CH_2OCO$-(alkyle en $C_8$-$C_{18}$),
$R_8$ est alkyle en $C_8$-$C_{18}$ ou -$COCH=CH_2$,
$R_{17}$ représente alkyle en $C_8$-$C_{11}$, -$(CH_2)_m$-S-(alkyle en $C_8$-$C_{18}$) ou

$$-(CH_2)_m-\underset{\underset{O}{\|}}{P}-(\text{alcoxy en } C_1\text{-}C_4)_2,$$

ou A pour n = 2 représente
-$O(CH_2)_gO$-, -$NH(CH_2)_gNH$-, -$OCH_2CH_2SCH_2CH_2O$-, -$O(CH_2CH_2O)_cCH_2CH_2O$-, -$OCH_2C(CH_3)_2CH_2O$-, $NHCH_2CH_2OCH_2CH_2O$-, -$NHCH_2CH_2OCO$-$(CH_2)_{a''}$-$OCOCH_2CH_2NH$- oder -NH-NHCO-$(CH_2)_{a''}$-CONH-NH- avec
a" = de 2 à 8, -NH-N=CH-$C(CH_3)_2$-S-S-$C(CH_3)_2$-CH=N-NH- ou -$(CH_2)_{p''}$- avec p" = de 5 à 10,
b va de 3 à 5, plus particulièrement est 5, $X_2$ est la liaison directe ou $>$CH-$CH_3$,
les radicaux $R_{15}'$, indépendamment l'un de l'autre, représnte méthyle ou tert.butyle et c, g et m ont la signification donnée dans la revendication 10.

**15.** Composés selon la revendication 10, dans lesquels R, $R_1$, $R_2$ et $R_3$ représentent hydrogène, n vaut 1 ou 2,
A pour n = 1 représente alkyle en $C_8$-$C_{17}$, -$OR_5$, -$NHR_6$ ou -NH-NH-CO-(alkyle en $C_8$-$C_{17}$),
$R_5$ représente alkyle en $C_{13}$-$C_{18}$ et $R_6$ représente alkyle en $C_8$-$C_{18}$,
ou A pour n = 2 représente
-$O(CH_2)_g$-O-, -$NH(CH_2)_gNH$-, -$OCH_2C(CH_3)_2CH_2O$-, -NH-NHCO-$CH_2CH_2$-OCNH-NH- oder -$(CH_2)_{p''}$-avec p" = 5 à 10, g ayant la signification donnée dans la revendication 10.

**16.** Utilisation des composés de formule I selon la revendication 1, ou des composés de formule Ib selon la revendication 10, pour la stabilisation des lubrifiants, des fluides de traitement de métaux, des fluides hydrauliques ou des polymères naturels, semi-synthétiques ou synthétiques contre la dégradation oxydative, thermique et/ou actinique.

**17.** Procédé pour la stabilisation des lubrifiants, des fluides de traitement de métaux, des fluides hydrauliques ou des polymères naturels, semisynthétiques ou synthétiques, caractérisé en ce qu'on ajoute à la matière organique en tant que stabilisant des composés de formule I selon la revendication 1, ou des composés de formule Ib selon la revendication 10, respectivement en ce qu'on les dépose sur celle-ci.